(19) Europäisches Patentamt / European Patent Office / Office européen des brevets

(11) EP 2 391 734 B1

(12) **EUROPEAN PATENT SPECIFICATION**

(45) Date of publication and mention of the grant of the patent:
**05.06.2013 Bulletin 2013/23**

(21) Application number: **10704252.5**

(22) Date of filing: **29.01.2010**

(51) Int Cl.:
***C12Q 1/68*** *(2006.01)*

(86) International application number:
**PCT/US2010/022518**

(87) International publication number:
**WO 2010/088470 (05.08.2010 Gazette 2010/31)**

(54) **MARKER TO PREDICT AND MONITOR RESPONSE TO AURORA KINASE B INHIBITOR THERAPY**

MARKER ZUR VORHERSAGE UND ÜBERWACHUNG DER REAKTION AUF EINE THERAPIE MIT AURORA-KINASE-B-INHIBITOR

MARQUEUR POUR LA PRÉDICTION ET LE SUIVI DE RÉACTION À UNE THÉRAPIE À BASE D'INHIBITEURS DES KINASES AURORA B

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.01.2009 US 148957 P**

(43) Date of publication of application:
**07.12.2011 Bulletin 2011/49**

(60) Divisional application:
**12164247.4 / 2 479 288**

(73) Proprietor: **Abbott Laboratories**
**Abbott Park, IL 60064 (US)**

(72) Inventors:
- **SEMIZAROV, Dimitri**
  **Chicago, IL 60657 (US)**
- **SHAH, Jameel**
  **Lake Villa, IL 60046 (US)**
- **GUO, Jun**
  **Libertyville, IL 60048 (US)**
- **ANDERSON, Mark**
  **Hoffman Estates, IL 60195 (US)**

(74) Representative: **Modiano, Micaela Nadia**
**Modiano & Partners**
**Thierschstrasse 11**
**80538 München (DE)**

(56) References cited:
**WO-A2-2006/123246**

- **GIRDLER F ET AL: "Molecular Basis of Drug Resistance in Aurora Kinases" CHEMISTRY AND BIOLOGY, CURRENT BIOLOGY, LONDON, GB LNKD- DOI:10.1016/J.CHEMBIOL.2008.04.013, vol. 15, no. 6, 23 June 2008 (2008-06-23), pages 552-562, XP022734025 ISSN: 1074-5521 [retrieved on 2008-06-20]**
- **KITADA ET AL: "The MDR1/ABCB1 regional amplification in large inverted repeats with asymmetric sequences and microhomologies at the junction sites" CANCER GENETICS AND CYTOGENETICS, ELSEVIER SCIENCE PUBLISHING, NEW YORK, NY, US, vol. 178, no. 2, 22 October 2007 (2007-10-22), pages 120-127, XP022308146 ISSN: 0165-4608**
- **MAHON FRANCOIS-XAVIER ET AL: "MDR1 gene overexpression confers resistance to imatinib mesylate in leukemia cell line models" BLOOD, AMERICAN SOCIETY OF HEMATOLOGY, US LNKD- DOI:10.1182/BLOOD.V101.6.2368, vol. 101, no. 6, 15 March 2003 (2003-03-15), pages 2368-2373, XP002404659 ISSN: 0006-4971**
- **WILKINSON ROBERT W ET AL: "AZD1152, a selective inhibitor of Aurora B kinase, inhibits human tumor xenograft growth by inducing apoptosis." CLINICAL CANCER RESEARCH : AN OFFICIAL JOURNAL OF THE AMERICAN ASSOCIATION FOR CANCER RESEARCH 15 JUN 2007 LNKD- PUBMED:17575233, vol. 13, no. 12, 15 June 2007 (2007-06-15) , pages 3682-3688, XP002578490 ISSN: 1078-0432**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 391 734 B1

SEQUENCE LISTING

<110> SEMIZAROV, DIMITRI SHAH, JAMEEL GUO, JUN ANDERSON, MARK

<120> MARKERS TO PREDICT AND MONITOR RESPONSE TO AURORA KINASE B INHIBITOR THERAPY

<130> D-24625

<140>
<141>

<160> 321

<170> PatentIn version 3.5

<210> 1
<211> 4872
<212> DNA
<213> Homo sapiens

<400> 1

```
tattcagata ttctccagat tcctaaagat tagagatcat ttctcattct cctaggagta     60
ctcacttcag gaagcaacca gataaaagag aggtgcaacg gaagccagaa cattcctcct    120
ggaaattcaa cctgtttcgc agtttctcga ggaatcagca ttcagtcaat ccgggccggg    180
agcagtcatc tgtggtgagg ctgattggct gggcaggaac agcgccgggg cgtgggctga    240
gcacagccgc ttcgctctct ttgccacagg aagcctgagc tcattcgagt agcggctctt    300
ccaagctcaa agaagcagag gccgctgttc gtttccttta ggtctttcca ctaaagtcgg    360
agtatcttct tccaaaattt cacgtcttgg tggccgttcc aaggagcgcg aggtcggaat    420
ggatcttgaa ggggaccgca atggaggagc aaagaagaag aactttttta aactgaacaa    480
taaaagtgaa aaagataaga aggaaaagaa accaactgtc agtgtatttt caatgtttcg    540
ctattcaaat tggcttgaca agttgtatat ggtggtggga actttggctg ccatcatcca    600
tggggctgga cttcctctca tgatgctggt gtttggagaa atgacagata tctttgcaaa    660
tgcaggaaat ttagaagatc tgatgtcaaa catcactaat agaagtgata tcaatgatac    720
agggttcttc atgaatctgg aggaagacat gaccaggtat gcctattatt acagtggaat    780
tggtgctggg gtgctggttg ctgcttacat tcaggtttca ttttggtgcc tggcagctgg    840
aagacaaata cacaaaatta gaaaacagtt ttttcatgct ataatgcgac aggagatagg    900
ctggtttgat gtgcacgatg ttggggagct taacacccga cttacagatg atgtctccaa    960
gattaatgaa ggaattggtg acaaaattgg aatgttcttt cagtcaatgg caacattttt   1020
cactgggttt atagtaggat ttacacgtgg ttggaagcta acccttgtga ttttggccat   1080
cagtcctgtt cttggactgt cagctgctgt ctgggcaaag atactatctt catttactga   1140
taaagaactc ttagcgtatg caaaagctgg agcagtagct gaagaggtct tggcagcaat   1200
```

```
tagaactgtg attgcatttg gaggacaaaa gaaagaactt gaaaggtaca acaaaaattt 1260
agaagaagct aaaagaattg ggataaagaa agctattaca gccaatattt ctataggtgc 1320
tgctttcctg ctgatctatg catcttatgc tctggccttc tggtatggga ccaccttggt 1380
cctctcaggg gaatattcta ttggacaagt actcactgta ttcttttctg tattaattgg 1440
ggcttttagt gttggacagg catctccaag cattgaagca tttgcaaatg caagaggagc 1500
agcttatgaa atcttcaaga taattgataa taagccaagt attgacagct attcgaagag 1560
tgggcacaaa ccagataata ttaagggaaa tttggaattc agaaatgttc acttcagtta 1620
cccatctcga aaagaagtta agatcttgaa gggtctgaac ctgaaggtgc agagtgggca 1680
gacggtggcc ctggttggaa acagtggctg tgggaagagc acaacagtcc agctgatgca 1740
gaggctctat gaccccacag aggggatggt cagtgttgat ggacaggata ttaggaccat 1800
aaatgtaagg tttctacggg aaatcattgg tgtggtgagt caggaacctg tattgtttgc 1860
caccacgata gctgaaaaca ttcgctatgg ccgtgaaaat gtcaccatgg atgagattga 1920
gaaagctgtc aaggaagcca atgcctatga ctttatcatg aaactgcctc ataaatttga 1980
caccctggtt ggagagagag gggcccagtt gagtggtggg cagaagcaga ggatcgccat 2040
tgcacgtgcc ctggttcgca accccaagat cctcctgctg gatgaggcca cgtcagcctt 2100
ggacacagaa agcgaagcag tggttcaggt ggctctggat aaggccagaa aaggtcggac 2160
caccattgtg atagctcatc gtttgtctac agttcgtaat gctgacgtca tcgctggttt 2220
cgatgatgga gtcattgtgg agaaaggaaa tcatgatgaa ctcatgaaag agaaaggcat 2280
ttacttcaaa cttgtcacaa tgcagacagc aggaaatgaa gttgaattag aaaatgcagc 2340
tgatgaatcc aaaagtgaaa ttgatgcctt ggaaatgtct tcaaatgatt caagatccag 2400
tctaataaga aaaagatcaa ctcgtaggag tgtccgtgga tcacaagccc aagacagaaa 2460
gcttagtacc aaagaggctc tggatgaaag tatacctcca gtttcctttt ggaggattat 2520
gaagctaaat ttaactgaat ggccttattt tgttgttggt gtattttgtg ccattataaa 2580
tggaggcctg caaccagcat ttgcaataat attttcaaag attatagggg tttttacaag 2640
aattgatgat cctgaaacaa aacgacagaa tagtaacttg ttttcactat tgtttctagc 2700
ccttggaatt atttctttta ttacattttt ccttcagggt ttcacatttg gcaaagctgg 2760
agagatcctc accaagcggc tccgatacat ggttttccga tccatgctca gacaggatgt 2820
gagttggttt gatgacccta aaaacaccac tggagcattg actaccaggc tcgccaatga 2880
tgctgctcaa gttaaagggg ctataggttc caggcttgct gtaattaccc agaatatagc 2940
aaatcttggg acaggaataa ttatatcctt catctatggt tggcaactaa cactgttact 3000
cttagcaatt gtacccatca ttgcaatagc aggagttgtt gaaatgaaaa tgttgtctgg 3060
acaagcactg aaagataaga aagaactaga aggttctggg aagatcgcta ctgaagcaat 3120
```

agaaaacttc cgaaccgttg tttctttgac tcaggagcag aagtttgaac atatgtatgc 3180
tcagagtttg caggtaccat acagaaactc tttgaggaaa gcacacatct ttggaattac 3240
attttccttc acccaggcaa tgatgtattt ttcctatgct ggatgtttcc ggtttggagc 3300
ctacttggtg gcacataaac tcatgagctt tgaggatgtt ctgttagtat tttcagctgt 3360
tgtctttggt gccatggccg tggggcaagt cagttcattt gctcctgact atgccaaagc 3420
caaaatatca gcagcccaca tcatcatgat cattgaaaaa acccctttga ttgacagcta 3480
cagcacggaa ggcctaatgc cgaacacatt ggaaggaaat gtcacatttg gtgaagttgt 3540
attcaactat cccacccgac cggacatccc agtgcttcag ggactgagcc tggaggtgaa 3600
gaagggccag acgctggctc tggtgggcag cagtggctgt gggaagagca cagtggtcca 3660
gctcctggag cggttctacg accccttggc agggaaagtg ctgcttgatg gcaaagaaat 3720
aaagcgactg aatgttcagt ggctccgagc acacctgggc atcgtgtccc aggagcccat 3780
cctgtttgac tgcagcattg ctgagaacat tgcctatgga gacaacagcc gggtggtgtc 3840
acaggaagag attgtgaggg cagcaaagga ggccaacata catgccttca tcgagtcact 3900
gcctaataaa tatagcacta aagtaggaga caaaggaact cagctctctg gtggccagaa 3960
acaacgcatt gccatagctc gtgcccttgt tagacagcct catattttgc ttttggatga 4020
agccacgtca gctctggata cagaaagtga aaaggttgtc caagaagccc tggacaaagc 4080
cagagaaggc cgcacctgca ttgtgattgc tcaccgcctg tccaccatcc agaatgcaga 4140
cttaatagtg gtgtttcaga atggcagagt caaggagcat ggcacgcatc agcagctgct 4200
ggcacagaaa ggcatctatt tttcaatggt cagtgtccag gctggaacaa agcgccagtg 4260
aactctgact gtatgagatg ttaaatactt tttaatattt gtttagatat gacatttatt 4320
caaagttaaa agcaaacact tacagaatta tgaagaggta tctgtttaac atttcctcag 4380
tcaagttcag agtcttcaga gacttcgtaa ttaaaggaac agagtgagag acatcatcaa 4440
gtggagagaa atcatagttt aaactgcatt ataaatttta taacagaatt aaagtagatt 4500
ttaaaagata aaatgtgtaa ttttgtttat attttcccat ttggactgta actgactgcc 4560
ttgctaaaag attatagaag tagcaaaaag tattgaaatg tttgcataaa gtgtctataa 4620
taaaactaaa ctttcatgtg actggagtca tcttgtccaa actgcctgtg aatatatctt 4680
ctctcaattg gaatattgta gataacttct gctttaaaaa agttttcttt aaatatacct 4740
actcattttt gtgggaatgg ttaagcagtt taaataattc ctgttgtata tgtctattca 4800
cattgggtct tacagaacca tctggcttca ttcttcttgg acttgatcct gctgattctt 4860
gcatttccac at 4872

<210> 2

<211> 3988
<212> DNA
<213> Homo sapiens

<400> 2

caaagtccag gcccctctgc tgcagcgccc gcgcgtccag aggccctgcc agacacgcgc 60
gaggttcgag gctgagatgg atcttgaggc ggcaaagaac ggaacagcct ggcgccccac 120
gagcgcggag ggcgactttg aactgggcat cagcagcaaa caaaaaagga aaaaaacgaa 180
gacagtgaaa atgattggag tattaacatt gtttcgatac tccgattggc aggataaatt 240
gtttatgtcg ctgggtacca tcatggccat agctcacgga tcaggtctcc ccctcatgat 300
gatagtattt ggagagatga ctgacaaatt tgttgatact gcaggaaact tctcctttcc 360
agtgaacttt tccttgtcgc tgctaaatcc aggcaaaatt ctggaagaag aaatgactag 420
atatgcatat tactactcag gattgggtgc tggagttctt gttgctgcct atatacaagt 480
ttcattttgg actttggcag ctggtcgaca gatcaggaaa attaggcaga agtttttca 540
tgctattcta cgacaggaaa taggatggtt tgacatcaac gacaccactg aactcaatac 600
gcggctaaca gatgacatct ccaaaatcag tgaaggaatt ggtgacaagg ttggaatgtt 660
ctttcaagca gtagccacgt ttttgcagg attcatagtg ggattcatca gaggatggaa 720
gctcaccctt gtgataatgg ccatcagccc tattctagga ctctctgcag ccgtttgggc 780
aaagatactc tcggcattta gtgacaaaga actagctgct tatgcaaaag caggcgccgt 840
ggcagaagag gctctggggg ccatcaggac tgtgatagct ttcgggggcc agaacaaaga 900
gctggaaagg tatcagaaac atttagaaaa tgccaaagag attggaatta aaaaagctat 960
ttcagcaaac atttccatgg gtattgcctt cctgttaata tatgcatcat atgcactggc 1020
cttctggtat ggatccactc tagtcatatc aaaagaatat actattggaa atgcaatgac 1080
agttttttt tcaatcctaa ttggagcttt cagtgttggc caggctgccc catgtattga 1140
tgcttttgcc aatgcaagag gagcagcata tgtgatcttt gatattattg ataataatcc 1200
taaaattgac agtttttcag agagaggaca caaaccagac agcatcaaag ggaatttgga 1260
gttcaatgat gttcactttt cttacccttc tcgagctaac gtcaagatct tgaagggcct 1320
caacctgaag gtgcagagtg ggcagacggt ggccctggtt ggaagtagtg gctgtgggaa 1380
gagcacaacg gtccagctga tacagaggct ctatgaccct gatgagggca caattaacat 1440
tgatgggcag gatattagga actttaatgt aaactatctg agggaaatca ttggtgtggt 1500
gagtcaggag ccggtgctgt tttccaccac aattgctgaa aatatttgtt atggccgtgg 1560
aaatgtaacc atggatgaga taaagaaagc tgtcaaagag gccaacgcct atgagtttat 1620
catgaaatta ccacagaaat ttgacaccct ggttggagag agaggggccc agctgagtgg 1680
tgggcagaag cagaggatcg ccattgcacg tgccctggtt cgcaaccccca agatccttct 1740
gctggatgag gccacgtcag cattggacac agaaagtgaa gctgaggtac aggcagctct 1800
ggataaggcc agagaaggcc ggaccaccat tgtgatagca caccgactgt ctacggtccg 1860
aaatgcagat gtcatcgctg ggtttgagga tggagtaatt gtggagcaag gaagccacag 1920

cgaactgatg aagaaggaag gggtgtactt caaacttgtc aacatgcaga catcaggaag 1980
ccagatccag tcagaagaat ttgaactaaa tgatgaaaag gctgccacta gaatggcccc 2040
aaatggctgg aaatctcgcc tatttaggca ttctactcag aaaaacctta aaaattcaca 2100
aatgtgtcag aagagccttg atgtggaaac cgatggactt gaagcaaatg tgccaccagt 2160
gtcctttctg aaggtcctga aactgaataa aacagaatgg ccctactttg tcgtgggaac 2220
agtatgtgcc attgccaatg gggggcttca gccggcattt tcagtcatat tctcagagat 2280
catagcgatt tttggaccag gcgatgatgc agtgaagcag cagaagtgca acatattctc 2340
tttgattttc ttatttctgg gaattatttc tttttttact ttcttccttc agggtttcac 2400
gtttgggaaa gctggcgaga tcctcaccag aagactgcgg tcaatggctt ttaaagcaat 2460
gctaagacag gacatgagct ggtttgatga ccataaaaac agtactggtg cactttctac 2520
aagacttgcc acagatgctg cccaagtcca aggagccaca ggaaccaggt tggctttaat 2580
tgcacagaat atagctaacc ttggaactgg tattatcata tcatttatct acggttggca 2640
gttaacccta ttgctattag cagttgttcc aattattgct gtgtcaggaa ttgttgaaat 2700
gaaattgttg gctggaaatg ccaaaagaga taaaaaagaa ctggaagctg ctggaaagat 2760
tgcaacagag gcaatagaaa atattaggac agttgtgtct ttgacccagg aaagaaaatt 2820
tgaatcaatg tatgttgaaa aattgtatgg accttacagg aattctgtgc agaaggcaca 2880
catctatgga attactttta gtatctcaca agcatttatg tatttttcct atgccggttg 2940
ttttcgattt ggtgcatatc tcattgtgaa tggacatatg cgcttcagag atgttattct 3000
ggtgttttct gcaattgtat ttggtgcagt ggctctagga catgccagtt catttgctcc 3060
agactatgct aaagctaagc tgtctgcagc ccacttattc atgctgtttg aaagacaacc 3120
tctgattgac agctacagtg aagaggggct gaagcctgat aaatttgaag gaaatataac 3180
atttaatgaa gtcgtgttca actatcccac ccgagcaaac gtgccagtgc ttcaggggct 3240
gagcctggag gtgaagaaag gccagacact agccctggtg ggcagcagtg gctgtgggaa 3300
gagcacggtg gtccagctcc tggagcggtt ctacgacccc ttggcgggga cagtgtttgt 3360
ggactttggt tttcagcttc tcgatggtca agaagcaaag aaactcaatg tccagtggct 3420
cagagctcaa ctcggaatcg tgtctcagga gcctatccta tttgactgca gcattgccga 3480
gaatattgcc tatggagaca acagccgggt tgtatcacag gatgaaattg tgagtgcagc 3540
caaagctgcc aacatacatc ctttcatcga gacgttaccc cacaaatatg aaacaagagt 3600
gggagataag gggactcagc tctcaggagg tcaaaaacag aggattgcta ttgcccgagc 3660
cctcatcaga caacctcaaa tcctcctgtt ggatgaagct acatcagctc tggatactga 3720
aagtgaaaag gttgtccaag aagccctgga caaagccaga gaaggccgca cctgcattgt 3780
gattgctcac cgcctgtcca ccatccagaa tgcagactta atagtggtgt ttcagaatgg 3840

gagagtcaag gagcatggca cgcatcagca gctgctggca cagaaaggca tctatttttc 3900

aatggtcagt gtccaggctg ggacacagaa cttatgaact tttgctacag tatattttaa 3960

aaataaattc aaattattct accatttt 3988

<210> 3
<211> 21
<212> RNA
<213> Artificial Sequence

<220>
<223> Description of Artificial Sequence: Synthetic oligonucleotide

<400> 3
aacguacgcg gaauacuucg a 21

<210> 4
<211> 23
<212> DNA
<213> Artificial Sequence

<220>
<223> Description of Artificial Sequence: Synthetic primer

<400> 4
ggagagtagc agtgccttgg acc 23

<210> 5
<211> 29
<212> DNA
<213> Artificial Sequence

<220>
<223> Description of Artificial Sequence: Synthetic primer

<400> 5
aggaggaggt agaaaacaga taagggaac 29

<210> 6
<211> 22
<212> DNA
<213> Artificial Sequence

<220>
<223> Description of Artificial Sequence: Synthetic primer

<400> 6
agtgccttgg accccagctc tc 22

<210> 7
<211> 29
<212> DNA
<213> Artificial Sequence

<220>
<223> Description of Artificial Sequence: Synthetic primer

<400> 7
gaaaacagat aagggaacag ttagggatc 29

<210> 8
<211> 51
<212> DNA
<213> Homo sapiens

<400> 8
ccatgttgaa aaacattgac ctgaartggt ggttctaaag cttcggtgaa t 51

<210> 9
<211> 51
<212> DNA
<213> Homo sapiens

<400> 9
tgccctacaa ttcattatag cacaaycttt aacacaccac ttaataactg t 51

<210> 10
<211> 51
<212> DNA
<213> Homo sapiens

<400> 10
aaccatcagg ctactgagat agtgayagca atttttttc atacttcttc t 51

<210> 11
<211> 51
<212> DNA
<213> Homo sapiens

<400> 11
ccaaaatatt agttatgctg taatayatcc attaagctat ttaagaaaac a 51

<210> 12
<211> 51
<212> DNA
<213> Homo sapiens

<400> 12
ctttccctac atagttatag tagctsaagt cccttagtct ctccacattc c 51

<210> 13
<211> 51
<212> DNA
<213> Homo sapiens

<400> 13
agtcctgtaa cttcttcctc tataartctc ataagtattt gctttctttt c 51

<210> 14
<211> 51
<212> DNA
<213> Homo sapiens

<400> 14
tacaataaac ataggatcta attagwcagc tctaaaacct tcttcagtaa g 51

<210> 15
<211> 51
<212> DNA
<213> Homo sapiens

<400> 15
tcttagaaac tagagcaaca aatacyatat tatataccat taaatacttt t 51

<210> 16
<211> 51
<212> DNA
<213> Homo sapiens

<400> 16
taaatattat gtgttgtgga ttaaayttgt gcgcttaaat aaatttcagt t 51

<210> 17
<211> 51
<212> DNA
<213> Homo sapiens

<400> 17
aaaattacaa tttatcttac tgctaycatt tgtgttttca atcttcatct t 51

<210> 18
<211> 51
<212> DNA
<213> Homo sapiens

<400> 18
taaggaaaaa tatagtacac tttacrgatt tgcaggtttt gctatttata a 51

<210> 19
<211> 51
<212> DNA
<213> Homo sapiens

<400> 19
caagaccctt tctttaatgg gtacawaatg tcaaaaatat ttttatataa t 51

<210> 20
<211> 51
<212> DNA
<213> Homo sapiens

<400> 20
gacaccttga tgcaggcttc tttaaygcag agtaggacac agatggctgg a 51

<210> 21
<211> 51
<212> DNA
<213> Homo sapiens

<400> 21
atttcagtat ctcctaaact cttgcyatgc aggaaaaatt attttatgtg a 51

<210> 22
<211> 51

<212> DNA
<213> Homo sapiens

<400> 22
taagagggaa gattaacatt cggacyaacc atgtttccac taaaccaatt a 51

<210> 23
<211> 25
<212> DNA
<213> Artificial Sequence

<220>
<223> Description of Artificial Sequence: Synthetic probe

<400> 23
agctttagaa ccaccacttc aggtc 25

<210> 24
<211> 25
<212> DNA
<213> Artificial Sequence

<220>
<223> Description of Artificial Sequence: Synthetic probe

<400> 24
agctttagaa ccaccatttc aggtc 25

<210> 25
<211> 25
<212> DNA
<213> Artificial Sequence

<220>
<223> Description of Artificial Sequence: Synthetic probe

<400> 25
gacctgaagt ggtggttcta aagct 25

<210> 26
<211> 25
<212> DNA
<213> Artificial Sequence

<220>
<223> Description of Artificial Sequence: Synthetic probe

<400> 26
tagaaccacc atttcaggtc aatgt 25

<210> 27
<211> 25
<212> DNA
<213> Artificial Sequence

<220>
<223> Description of Artificial Sequence: Synthetic probe

<400> 27
tagaaccacc acttcaggtc aatgt 25

<210> 28
<211> 25
<212> DNA
<213> Artificial Sequence

<220>
<223> Description of Artificial Sequence: Synthetic probe

<400> 28
gacctgaaat ggtggttcta aagct 25

<210> 29
<211> 25
<212> DNA
<213> Artificial Sequence

<220>
<223> Description of Artificial Sequence: Synthetic probe

<400> 29
ttgacctgaa gtggtggttc taaag 25

<210> 30
<211> 25
<212> DNA
<213> Artificial Sequence

<220>
<223> Description of Artificial Sequence: Synthetic probe

<400> 30
ttgacctgaa atggtggttc taaag 25

<210> 31
<211> 25
<212> DNA
<213> Artificial Sequence

<220>
<223> Description of Artificial Sequence: Synthetic probe

<400> 31
acattgacct gaaatggtgg ttcta 25

<210> 32
<211> 25
<212> DNA
<213> Artificial Sequence

<220>
<223> Description of Artificial Sequence: Synthetic probe

<400> 32
acattgacct gaagtggtgg ttcta 25

<210> 33
<211> 25
<212> DNA
<213> Artificial Sequence

<220>
<223> Description of Artificial Sequence: Synthetic probe

<400> 33
cattgacctg aagtggtggt tctaa 25

<210> 34
<211> 25
<212> DNA
<213> Artificial Sequence

<220>
<223> Description of Artificial Sequence: Synthetic probe

<400> 34
cattgacctg aaatggtggt tctaa 25

<210> 35
<211> 25
<212> DNA
<213> Artificial Sequence

<220>
<223> Description of Artificial Sequence: Synthetic probe

<400> 35
aaccaccact tcaggtcaat gtttt 25

<210> 36
<211> 25
<212> DNA
<213> Artificial Sequence

<220>
<223> Description of Artificial Sequence: Synthetic probe

<400> 36
aaccaccatt tcaggtcaat gtttt 25

<210> 37
<211> 25
<212> DNA
<213> Artificial Sequence

<220>
<223> Description of Artificial Sequence: Synthetic probe

<400> 37
aaaacattga cctgaaatgg tggtt 25

<210> 38
<211> 25
<212> DNA

<213> Artificial Sequence

<220>
<223> Description of Artificial Sequence: Synthetic probe

<400> 38
aaaacattga cctgaagtgg tggtt 25

<210> 39
<211> 25
<212> DNA
<213> Artificial Sequence

<220>
<223> Description of Artificial Sequence: Synthetic probe

<400> 39
aaacattgac ctgaaatggt ggttc 25

<210> 40
<211> 25
<212> DNA
<213> Artificial Sequence

<220>
<223> Description of Artificial Sequence: Synthetic probe

<400> 40
aaacattgac ctgaagtggt ggttc 25

<210> 41
<211> 25
<212> DNA
<213> Artificial Sequence

<220>
<223> Description of Artificial Sequence: Synthetic probe

<400> 41
tttagaacca ccacttcagg tcaat 25

<210> 42
<211> 25
<212> DNA
<213> Artificial Sequence

<220>
<223> Description of Artificial Sequence: Synthetic probe

<400> 42
tttagaacca ccatttcagg tcaat 25

<210> 43
<211> 25
<212> DNA
<213> Artificial Sequence

<220>

<223> Description of Artificial Sequence: Synthetic probe

<400> 43
tgttaaaggt tgtgctataa tgaat 25

<210> 44
<211> 25
<212> DNA
<213> Artificial Sequence

<220>
<223> Description of Artificial Sequence: Synthetic probe

<400> 44
tcattatagc acaaccttta acaca 25

<210> 45
<211> 25
<212> DNA
<213> Artificial Sequence

<220>
<223> Description of Artificial Sequence: Synthetic probe

<400> 45
gtgtgttaaa gattgtgcta taatg 25

<210> 46
<211> 25
<212> DNA
<213> Artificial Sequence

<220>
<223> Description of Artificial Sequence: Synthetic probe

<400> 46
atagcacaat ctttaacaca ccact 25

<210> 47
<211> 25
<212> DNA
<213> Artificial Sequence

<220>
<223> Description of Artificial Sequence: Synthetic probe

<400> 47
tcattatagc acaatcttta acaca 25

<210> 48
<211> 25
<212> DNA
<213> Artificial Sequence

<220>
<223> Description of Artificial Sequence: Synthetic probe

<400> 48

tagcacaacc tttaacacac cactt 25

<210> 49
<211> 25
<212> DNA
<213> Artificial Sequence

<220>
<223> Description of Artificial Sequence: Synthetic probe

<400> 49
gtgtgttaaa ggttgtgcta taatg 25

<210> 50
<211> 25
<212> DNA
<213> Artificial Sequence

<220>
<223> Description of Artificial Sequence: Synthetic probe

<400> 50
attatagcac aacctttaac acacc 25

<210> 51
<211> 25
<212> DNA
<213> Artificial Sequence

<220>
<223> Description of Artificial Sequence: Synthetic probe

<400> 51
attatagcac aatctttaac acacc 25

<210> 52
<211> 25
<212> DNA
<213> Artificial Sequence

<220>
<223> Description of Artificial Sequence: Synthetic probe

<400> 52
attcattata gcacaatctt taaca 25

<210> 53
<211> 25
<212> DNA
<213> Artificial Sequence

<220>
<223> Description of Artificial Sequence: Synthetic probe

<400> 53
atagcacaac ctttaacaca ccact 25

<210> 54

<211> 25
<212> DNA
<213> Artificial Sequence

<220>
<223> Description of Artificial Sequence: Synthetic probe

<400> 54
ttatagcaca acctttaaca cacca 25

<210> 55
<211> 25
<212> DNA
<213> Artificial Sequence

<220>
<223> Description of Artificial Sequence: Synthetic probe

<400> 55
ttatagcaca atctttaaca cacca 25

<210> 56
<211> 25
<212> DNA
<213> Artificial Sequence

<220>
<223> Description of Artificial Sequence: Synthetic probe

<400> 56
tgttaaagat tgtgctataa tgaat 25

<210> 57
<211> 25
<212> DNA
<213> Artificial Sequence

<220>
<223> Description of Artificial Sequence: Synthetic probe

<400> 57
tggtgtgtta aaggttgtgc tataa 25

<210> 58
<211> 25
<212> DNA
<213> Artificial Sequence

<220>
<223> Description of Artificial Sequence: Synthetic probe

<400> 58
tggtgtgtta aagattgtgc tataa 25

<210> 59
<211> 25
<212> DNA
<213> Artificial Sequence

<220>
<223> Description of Artificial Sequence: Synthetic probe

<400> 59
aagtggtgtg ttaaagattg tgcta 25

<210> 60
<211> 25
<212> DNA
<213> Artificial Sequence

<220>
<223> Description of Artificial Sequence: Synthetic probe

<400> 60
attcattata gcacaacctt taaca 25

<210> 61
<211> 25
<212> DNA
<213> Artificial Sequence

<220>
<223> Description of Artificial Sequence: Synthetic probe

<400> 61
aagtggtgtg ttaaaggttg tgcta 25

<210> 62
<211> 25
<212> DNA
<213> Artificial Sequence

<220>
<223> Description of Artificial Sequence: Synthetic probe

<400> 62
tagcacaatc tttaacacac cactt 25

<210> 63
<211> 25
<212> DNA
<213> Artificial Sequence

<220>
<223> Description of Artificial Sequence: Synthetic probe

<400> 63
actgagatag tgatagcaat ttttt 25

<210> 64
<211> 25
<212> DNA
<213> Artificial Sequence

<220>
<223> Description of Artificial Sequence: Synthetic probe

<400> 64
aaaaaattgc tatcactatc tcagt 25

<210> 65
<211> 25
<212> DNA
<213> Artificial Sequence

<220>
<223> Description of Artificial Sequence: Synthetic probe

<400> 65
aaaaaaaatt gctgtcacta tctca 25

<210> 66
<211> 25
<212> DNA
<213> Artificial Sequence

<220>
<223> Description of Artificial Sequence: Synthetic probe

<400> 66
aaaaaattgc tgtcactatc tcagt 25

<210> 67
<211> 25
<212> DNA
<213> Artificial Sequence

<220>
<223> Description of Artificial Sequence: Synthetic probe

<400> 67
gctactgaga tagtgatagc aattt 25

<210> 68
<211> 25
<212> DNA
<213> Artificial Sequence

<220>
<223> Description of Artificial Sequence: Synthetic probe

<400> 68
gctactgaga tagtgacagc aattt 25

<210> 69
<211> 25
<212> DNA
<213> Artificial Sequence

<220>
<223> Description of Artificial Sequence: Synthetic probe

<400> 69
atgaaaaaaa attgctatca ctatc 25

<210> 70
<211> 25
<212> DNA
<213> Artificial Sequence

<220>
<223> Description of Artificial Sequence: Synthetic probe

<400> 70
atgaaaaaaa attgctgtca ctatc 25

<210> 71
<211> 25
<212> DNA
<213> Artificial Sequence

<220>
<223> Description of Artificial Sequence: Synthetic probe

<400> 71
aaattgctgt cactatctca gtagc 25

<210> 72
<211> 25
<212> DNA
<213> Artificial Sequence

<220>
<223> Description of Artificial Sequence: Synthetic probe

<400> 72
aaattgctat cactatctca gtagc 25

<210> 73
<211> 25
<212> DNA
<213> Artificial Sequence

<220>
<223> Description of Artificial Sequence: Synthetic probe

<400> 73
aaaaaaaatt gctatcacta tctca 25

<210> 74
<211> 25
<212> DNA
<213> Artificial Sequence

<220>
<223> Description of Artificial Sequence: Synthetic probe

<400> 74
agatagtgac agcaattttt tttca 25

<210> 75
<211> 25
<212> DNA

<213> Artificial Sequence

<220>
<223> Description of Artificial Sequence: Synthetic probe

<400> 75
agatagtgat agcaattttt tttca 25

<210> 76
<211> 25
<212> DNA
<213> Artificial Sequence

<220>
<223> Description of Artificial Sequence: Synthetic probe

<400> 76
actgagatag tgacagcaat ttttt 25

<210> 77
<211> 25
<212> DNA
<213> Artificial Sequence

<220>
<223> Description of Artificial Sequence: Synthetic probe

<400> 77
tactgagata gtgatagcaa ttttt 25

<210> 78
<211> 25
<212> DNA
<213> Artificial Sequence

<220>
<223> Description of Artificial Sequence: Synthetic probe

<400> 78
tactgagata gtgacagcaa ttttt 25

<210> 79
<211> 25
<212> DNA
<213> Artificial Sequence

<220>
<223> Description of Artificial Sequence: Synthetic probe

<400> 79
ctgagatagt gatagcaatt ttttt 25

<210> 80
<211> 25
<212> DNA
<213> Artificial Sequence

<220>

<223> Description of Artificial Sequence: Synthetic probe

<400> 80
ctgagatagt gacagcaatt ttttt 25

<210> 81
<211> 25
<212> DNA
<213> Artificial Sequence

<220>
<223> Description of Artificial Sequence: Synthetic probe

<400> 81
aaaaattgct atcactatct cagta 25

<210> 82
<211> 25
<212> DNA
<213> Artificial Sequence

<220>
<223> Description of Artificial Sequence: Synthetic probe

<400> 82
aaaaattgct gtcactatct cagta 25

<210> 83
<211> 25
<212> DNA
<213> Artificial Sequence

<220>
<223> Description of Artificial Sequence: Synthetic probe

<400> 83
ttatgctgta atacatccat taagc 25

<210> 84
<211> 25
<212> DNA
<213> Artificial Sequence

<220>
<223> Description of Artificial Sequence: Synthetic probe

<400> 84
ttatgctgta atatatccat taagc 25

<210> 85
<211> 25
<212> DNA
<213> Artificial Sequence

<220>
<223> Description of Artificial Sequence: Synthetic probe

<400> 85

agttatgctg taatatatcc attaa 25

<210> 86
<211> 25
<212> DNA
<213> Artificial Sequence

<220>
<223> Description of Artificial Sequence: Synthetic probe

<400> 86
agttatgctg taatacatcc attaa 25

<210> 87
<211> 25
<212> DNA
<213> Artificial Sequence

<220>
<223> Description of Artificial Sequence: Synthetic probe

<400> 87
tgctgtaata tatccattaa gctat 25

<210> 88
<211> 25
<212> DNA
<213> Artificial Sequence

<220>
<223> Description of Artificial Sequence: Synthetic probe

<400> 88
tgctgtaata catccattaa gctat 25

<210> 89
<211> 25
<212> DNA
<213> Artificial Sequence

<220>
<223> Description of Artificial Sequence: Synthetic probe

<400> 89
atgctgtaat atatccatta agcta 25

<210> 90
<211> 25
<212> DNA
<213> Artificial Sequence

<220>
<223> Description of Artificial Sequence: Synthetic probe

<400> 90
atgctgtaat acatccatta agcta 25

<210> 91

<211> 25
<212> DNA
<213> Artificial Sequence

<220>
<223> Description of Artificial Sequence: Synthetic probe

<400> 91
agcttaatgg atgtattaca gcata 25

<210> 92
<211> 25
<212> DNA
<213> Artificial Sequence

<220>
<223> Description of Artificial Sequence: Synthetic probe

<400> 92
agcttaatgg atatattaca gcata 25

<210> 93
<211> 25
<212> DNA
<213> Artificial Sequence

<220>
<223> Description of Artificial Sequence: Synthetic probe

<400> 93
tagttatgct gtaatacatc catta 25

<210> 94
<211> 25
<212> DNA
<213> Artificial Sequence

<220>
<223> Description of Artificial Sequence: Synthetic probe

<400> 94
tagttatgct gtaatatatc catta 25

<210> 95
<211> 25
<212> DNA
<213> Artificial Sequence

<220>
<223> Description of Artificial Sequence: Synthetic probe

<400> 95
ctgtaatata tccattaagc tattt 25

<210> 96
<211> 25
<212> DNA
<213> Artificial Sequence

<220>
<223> Description of Artificial Sequence: Synthetic probe

<400> 96
ttaatggatg tattacagca taact 25

<210> 97
<211> 25
<212> DNA
<213> Artificial Sequence

<220>
<223> Description of Artificial Sequence: Synthetic probe

<400> 97
ctgtaataca tccattaagc tattt 25

<210> 98
<211> 25
<212> DNA
<213> Artificial Sequence

<220>
<223> Description of Artificial Sequence: Synthetic probe

<400> 98
ttaatggata tattacagca taact 25

<210> 99
<211> 25
<212> DNA
<213> Artificial Sequence

<220>
<223> Description of Artificial Sequence: Synthetic probe

<400> 99
tatgctgtaa tatatccatt aagct 25

<210> 100
<211> 25
<212> DNA
<213> Artificial Sequence

<220>
<223> Description of Artificial Sequence: Synthetic probe

<400> 100
tatgctgtaa tacatccatt aagct 25

<210> 101
<211> 25
<212> DNA
<213> Artificial Sequence

<220>
<223> Description of Artificial Sequence: Synthetic probe

<400> 101
atagcttaat ggatatatta cagca 25

<210> 102
<211> 25
<212> DNA
<213> Artificial Sequence

<220>
<223> Description of Artificial Sequence: Synthetic probe

<400> 102
tatagtagct caagtccctt agtct 25

<210> 103
<211> 25
<212> DNA
<213> Artificial Sequence

<220>
<223> Description of Artificial Sequence: Synthetic probe

<400> 103
tatagtagct gaagtccctt agtct 25

<210> 104
<211> 25
<212> DNA
<213> Artificial Sequence

<220>
<223> Description of Artificial Sequence: Synthetic probe

<400> 104
tagtagctca agtcccttag tctct 25

<210> 105
<211> 25
<212> DNA
<213> Artificial Sequence

<220>
<223> Description of Artificial Sequence: Synthetic probe

<400> 105
tagtagctga agtcccttag tctct 25

<210> 106
<211> 25
<212> DNA
<213> Artificial Sequence

<220>
<223> Description of Artificial Sequence: Synthetic probe

<400> 106
catagttata gtagctgaag tccct 25

<210> 107
<211> 25
<212> DNA
<213> Artificial Sequence

<220>
<223> Description of Artificial Sequence: Synthetic probe

<400> 107
catagttata gtagctcaag tccct 25

<210> 108
<211> 25
<212> DNA
<213> Artificial Sequence

<220>
<223> Description of Artificial Sequence: Synthetic probe

<400> 108
actaagggac ttcagctact ataac 25

<210> 109
<211> 25
<212> DNA
<213> Artificial Sequence

<220>
<223> Description of Artificial Sequence: Synthetic probe

<400> 109
actaagggac ttgagctact ataac 25

<210> 110
<211> 25
<212> DNA
<213> Artificial Sequence

<220>
<223> Description of Artificial Sequence: Synthetic probe

<400> 110
gttatagtag ctcaagtccc ttagt 25

<210> 111
<211> 25
<212> DNA
<213> Artificial Sequence

<220>
<223> Description of Artificial Sequence: Synthetic probe

<400> 111
gttatagtag ctgaagtccc ttagt 25

<210> 112
<211> 25
<212> DNA

<213> Artificial Sequence

<220>
<223> Description of Artificial Sequence: Synthetic probe

<400> 112
ttatagtagc tcaagtccct tagtc 25

<210> 113
<211> 25
<212> DNA
<213> Artificial Sequence

<220>
<223> Description of Artificial Sequence: Synthetic probe

<400> 113
ttatagtagc tgaagtccct tagtc 25

<210> 114
<211> 25
<212> DNA
<213> Artificial Sequence

<220>
<223> Description of Artificial Sequence: Synthetic probe

<400> 114
agactaaggg acttcagcta ctata 25

<210> 115
<211> 25
<212> DNA
<213> Artificial Sequence

<220>
<223> Description of Artificial Sequence: Synthetic probe

<400> 115
agactaaggg acttgagcta ctata 25

<210> 116
<211> 25
<212> DNA
<213> Artificial Sequence

<220>
<223> Description of Artificial Sequence: Synthetic probe

<400> 116
gactaaggga cttcagctac tataa 25

<210> 117
<211> 25
<212> DNA
<213> Artificial Sequence

<220>

<223> Description of Artificial Sequence: Synthetic probe

<400> 117
gactaaggga cttgagctac tataa 25

<210> 118
<211> 25
<212> DNA
<213> Artificial Sequence

<220>
<223> Description of Artificial Sequence: Synthetic probe

<400> 118
agttatagta gctgaagtcc cttag 25

<210> 119
<211> 25
<212> DNA
<213> Artificial Sequence

<220>
<223> Description of Artificial Sequence: Synthetic probe

<400> 119
agttatagta gctcaagtcc cttag 25

<210> 120
<211> 25
<212> DNA
<213> Artificial Sequence

<220>
<223> Description of Artificial Sequence: Synthetic probe

<400> 120
aagggacttc agctactata actat 25

<210> 121
<211> 25
<212> DNA
<213> Artificial Sequence

<220>
<223> Description of Artificial Sequence: Synthetic probe

<400> 121
aagggacttg agctactata actat 25

<210> 122
<211> 25
<212> DNA
<213> Artificial Sequence

<220>
<223> Description of Artificial Sequence: Synthetic probe

<400> 122

aatacttatg agatttatag aggaa 25

<210> 123
<211> 25
<212> DNA
<213> Artificial Sequence

<220>
<223> Description of Artificial Sequence: Synthetic probe

<400> 123
cttatgagat ttatagagga agaag 25

<210> 124
<211> 25
<212> DNA
<213> Artificial Sequence

<220>
<223> Description of Artificial Sequence: Synthetic probe

<400> 124
cttatgagac ttatagagga agaag 25

<210> 125
<211> 25
<212> DNA
<213> Artificial Sequence

<220>
<223> Description of Artificial Sequence: Synthetic probe

<400> 125
atacttatga gacttataga ggaag 25

<210> 126
<211> 25
<212> DNA
<213> Artificial Sequence

<220>
<223> Description of Artificial Sequence: Synthetic probe

<400> 126
atacttatga gatttataga ggaag 25

<210> 127
<211> 25
<212> DNA
<213> Artificial Sequence

<220>
<223> Description of Artificial Sequence: Synthetic probe

<400> 127
acttcttcct ctataagtct cataa 25

<210> 128

<211> 25
<212> DNA
<213> Artificial Sequence

<220>
<223> Description of Artificial Sequence: Synthetic probe

<400> 128
acttcttcct ctataaatct cataa 25

<210> 129
<211> 25
<212> DNA
<213> Artificial Sequence

<220>
<223> Description of Artificial Sequence: Synthetic probe

<400> 129
aatacttatg agacttatag aggaa 25

<210> 130
<211> 25
<212> DNA
<213> Artificial Sequence

<220>
<223> Description of Artificial Sequence: Synthetic probe

<400> 130
tacttatgag atttatagag gaaga 25

<210> 131
<211> 25
<212> DNA
<213> Artificial Sequence

<220>
<223> Description of Artificial Sequence: Synthetic probe

<400> 131
tacttatgag acttatagag gaaga 25

<210> 132
<211> 25
<212> DNA
<213> Artificial Sequence

<220>
<223> Description of Artificial Sequence: Synthetic probe

<400> 132
ttcctctata aatctcataa gtatt 25

<210> 133
<211> 25
<212> DNA
<213> Artificial Sequence

<220>
<223> Description of Artificial Sequence: Synthetic probe

<400> 133
tcttcctcta taagtctcat aagta 25

<210> 134
<211> 25
<212> DNA
<213> Artificial Sequence

<220>
<223> Description of Artificial Sequence: Synthetic probe

<400> 134
tcttcctcta taaatctcat aagta 25

<210> 135
<211> 25
<212> DNA
<213> Artificial Sequence

<220>
<223> Description of Artificial Sequence: Synthetic probe

<400> 135
ctctataagt ctcataagta tttgc 25

<210> 136
<211> 25
<212> DNA
<213> Artificial Sequence

<220>
<223> Description of Artificial Sequence: Synthetic probe

<400> 136
ctctataaat ctcataagta tttgc 25

<210> 137
<211> 25
<212> DNA
<213> Artificial Sequence

<220>
<223> Description of Artificial Sequence: Synthetic probe

<400> 137
ttatgagatt tatagaggaa gaagt 25

<210> 138
<211> 25
<212> DNA
<213> Artificial Sequence

<220>
<223> Description of Artificial Sequence: Synthetic probe

<400> 138
ttatgagact tatagaggaa gaagt 25

<210> 139
<211> 25
<212> DNA
<213> Artificial Sequence

<220>
<223> Description of Artificial Sequence: Synthetic probe

<400> 139
aaatacttat gagatttata gagga 25

<210> 140
<211> 25
<212> DNA
<213> Artificial Sequence

<220>
<223> Description of Artificial Sequence: Synthetic probe

<400> 140
aaatacttat gagacttata gagga 25

<210> 141
<211> 25
<212> DNA
<213> Artificial Sequence

<220>
<223> Description of Artificial Sequence: Synthetic probe

<400> 141
ttcctctata agtctcataa gtatt 25

<210> 142
<211> 25
<212> DNA
<213> Artificial Sequence

<220>
<223> Description of Artificial Sequence: Synthetic probe

<400> 142
ttagagctga ctaattagat cctat 25

<210> 143
<211> 25
<212> DNA
<213> Artificial Sequence

<220>
<223> Description of Artificial Sequence: Synthetic probe

<400> 143
ttagagctgt ctaattagat cctat 25

<210> 144
<211> 25
<212> DNA
<213> Artificial Sequence

<220>
<223> Description of Artificial Sequence: Synthetic probe

<400> 144
atctaattag acagctctaa aacct 25

<210> 145
<211> 25
<212> DNA
<213> Artificial Sequence

<220>
<223> Description of Artificial Sequence: Synthetic probe

<400> 145
atctaattag tcagctctaa aacct 25

<210> 146
<211> 25
<212> DNA
<213> Artificial Sequence

<220>
<223> Description of Artificial Sequence: Synthetic probe

<400> 146
aggatctaat tagtcagctc taaaa 25

<210> 147
<211> 25
<212> DNA
<213> Artificial Sequence

<220>
<223> Description of Artificial Sequence: Synthetic probe

<400> 147
ataggatcta attagtcagc tctaa 25

<210> 148
<211> 25
<212> DNA
<213> Artificial Sequence

<220>
<223> Description of Artificial Sequence: Synthetic probe

<400> 148
ataggatcta attagacagc tctaa 25

<210> 149
<211> 25
<212> DNA

<213> Artificial Sequence

<220>
<223> Description of Artificial Sequence: Synthetic probe

<400> 149
ggttttagag ctgactaatt agatc 25

<210> 150
<211> 25
<212> DNA
<213> Artificial Sequence

<220>
<223> Description of Artificial Sequence: Synthetic probe

<400> 150
ggttttagag ctgtctaatt agatc 25

<210> 151
<211> 25
<212> DNA
<213> Artificial Sequence

<220>
<223> Description of Artificial Sequence: Synthetic probe

<400> 151
tagagctgac taattagatc ctatg 25

<210> 152
<211> 25
<212> DNA
<213> Artificial Sequence

<220>
<223> Description of Artificial Sequence: Synthetic probe

<400> 152
tagagctgtc taattagatc ctatg 25

<210> 153
<211> 25
<212> DNA
<213> Artificial Sequence

<220>
<223> Description of Artificial Sequence: Synthetic probe

<400> 153
ggatctaatt agacagctct aaaac 25

<210> 154
<211> 25
<212> DNA
<213> Artificial Sequence

<220>

<223> Description of Artificial Sequence: Synthetic probe

<400> 154
ggatctaatt agtcagctct aaaac 25

<210> 155
<211> 25
<212> DNA
<213> Artificial Sequence

<220>
<223> Description of Artificial Sequence: Synthetic probe

<400> 155
aggatctaat tagacagctc taaaa 25

<210> 156
<211> 25
<212> DNA
<213> Artificial Sequence

<220>
<223> Description of Artificial Sequence: Synthetic probe

<400> 156
ttttagagct gtctaattag atcct 25

<210> 157
<211> 25
<212> DNA
<213> Artificial Sequence

<220>
<223> Description of Artificial Sequence: Synthetic probe

<400> 157
gatctaatta gacagctcta aaacc 25

<210> 158
<211> 25
<212> DNA
<213> Artificial Sequence

<220>
<223> Description of Artificial Sequence: Synthetic probe

<400> 158
gatctaatta gtcagctcta aaacc 25

<210> 159
<211> 25
<212> DNA
<213> Artificial Sequence

<220>
<223> Description of Artificial Sequence: Synthetic probe

<400> 159

ttttagagct gactaattag atcct 25

<210> 160
<211> 25
<212> DNA
<213> Artificial Sequence

<220>
<223> Description of Artificial Sequence: Synthetic probe

<400> 160
gaaggtttta gagctgacta attag 25

<210> 161
<211> 25
<212> DNA
<213> Artificial Sequence

<220>
<223> Description of Artificial Sequence: Synthetic probe

<400> 161
gaaggtttta gagctgtcta attag 25

<210> 162
<211> 25
<212> DNA
<213> Artificial Sequence

<220>
<223> Description of Artificial Sequence: Synthetic probe

<400> 162
gcaacaaata ctatattata tacca 25

<210> 163
<211> 25
<212> DNA
<213> Artificial Sequence

<220>
<223> Description of Artificial Sequence: Synthetic probe

<400> 163
gcaacaaata ccatattata tacca 25

<210> 164
<211> 25
<212> DNA
<213> Artificial Sequence

<220>
<223> Description of Artificial Sequence: Synthetic probe

<400> 164
gtatataata tagtatttgt tgctc 25

<210> 165

<211> 25
<212> DNA
<213> Artificial Sequence

<220>
<223> Description of Artificial Sequence: Synthetic probe

<400> 165
gtatataata tggtatttgt tgctc 25

<210> 166
<211> 25
<212> DNA
<213> Artificial Sequence

<220>
<223> Description of Artificial Sequence: Synthetic probe

<400> 166
tataatatag tatttgttgc tctag 25

<210> 167
<211> 25
<212> DNA
<213> Artificial Sequence

<220>
<223> Description of Artificial Sequence: Synthetic probe

<400> 167
tataatatgg tatttgttgc tctag 25

<210> 168
<211> 25
<212> DNA
<213> Artificial Sequence

<220>
<223> Description of Artificial Sequence: Synthetic probe

<400> 168
agagcaacaa ataccatatt atata 25

<210> 169
<211> 25
<212> DNA
<213> Artificial Sequence

<220>
<223> Description of Artificial Sequence: Synthetic probe

<400> 169
agagcaacaa atactatatt atata 25

<210> 170
<211> 25
<212> DNA
<213> Artificial Sequence

<220>
<223> Description of Artificial Sequence: Synthetic probe

<400> 170
taatggtata taatatagta tttgt 25

<210> 171
<211> 25
<212> DNA
<213> Artificial Sequence

<220>
<223> Description of Artificial Sequence: Synthetic probe

<400> 171
taatggtata taatatggta tttgt 25

<210> 172
<211> 25
<212> DNA
<213> Artificial Sequence

<220>
<223> Description of Artificial Sequence: Synthetic probe

<400> 172
ctagagcaac aaatactata ttata 25

<210> 173
<211> 25
<212> DNA
<213> Artificial Sequence

<220>
<223> Description of Artificial Sequence: Synthetic probe

<400> 173
aacaaatact atattatata ccatt 25

<210> 174
<211> 25
<212> DNA
<213> Artificial Sequence

<220>
<223> Description of Artificial Sequence: Synthetic probe

<400> 174
aacaaatacc atattatata ccatt 25

<210> 175
<211> 25
<212> DNA
<213> Artificial Sequence

<220>
<223> Description of Artificial Sequence: Synthetic probe

<400> 175
ctagagcaac aaataccata ttata 25

<210> 176
<211> 25
<212> DNA
<213> Artificial Sequence

<220>
<223> Description of Artificial Sequence: Synthetic probe

<400> 176
ggtatataat atggtatttg ttgct 25

<210> 177
<211> 25
<212> DNA
<213> Artificial Sequence

<220>
<223> Description of Artificial Sequence: Synthetic probe

<400> 177
ggtatataat atagtatttg ttgct 25

<210> 178
<211> 25
<212> DNA
<213> Artificial Sequence

<220>
<223> Description of Artificial Sequence: Synthetic probe

<400> 178
agcaacaaat accatattat atacc 25

<210> 179
<211> 25
<212> DNA
<213> Artificial Sequence

<220>
<223> Description of Artificial Sequence: Synthetic probe

<400> 179
agcaacaaat actatattat atacc 25

<210> 180
<211> 25
<212> DNA
<213> Artificial Sequence

<220>
<223> Description of Artificial Sequence: Synthetic probe

<400> 180
tggtatataa tatagtattt gttgc 25

<210> 181
<211> 25
<212> DNA
<213> Artificial Sequence

<220>
<223> Description of Artificial Sequence: Synthetic probe

<400> 181
tggtatataa tatggtattt gttgc 25

<210> 182
<211> 25
<212> DNA
<213> Artificial Sequence

<220>
<223> Description of Artificial Sequence: Synthetic probe

<400> 182
tgtggattaa acttgtgcgc ttaaa 25

<210> 183
<211> 25
<212> DNA
<213> Artificial Sequence

<220>
<223> Description of Artificial Sequence: Synthetic probe

<400> 183
tgtggattaa atttgtgcgc ttaaa 25

<210> 184
<211> 25
<212> DNA
<213> Artificial Sequence

<220>
<223> Description of Artificial Sequence: Synthetic probe

<400> 184
atttaagcgc acaaatttaa tccac 25

<210> 185
<211> 25
<212> DNA
<213> Artificial Sequence

<220>
<223> Description of Artificial Sequence: Synthetic probe

<400> 185
atttaagcgc acaagtttaa tccac 25

<210> 186
<211> 25
<212> DNA

<213> Artificial Sequence

<220>
<223> Description of Artificial Sequence: Synthetic probe

<400> 186
ttgtggatta aatttgtgcg cttaa 25

<210> 187
<211> 25
<212> DNA
<213> Artificial Sequence

<220>
<223> Description of Artificial Sequence: Synthetic probe

<400> 187
ttgtggatta aacttgtgcg cttaa 25

<210> 188
<211> 25
<212> DNA
<213> Artificial Sequence

<220>
<223> Description of Artificial Sequence: Synthetic probe

<400> 188
taagcgcaca agtttaatcc acaac 25

<210> 189
<211> 25
<212> DNA
<213> Artificial Sequence

<220>
<223> Description of Artificial Sequence: Synthetic probe

<400> 189
taagcgcaca aatttaatcc acaac 25

<210> 190
<211> 25
<212> DNA
<213> Artificial Sequence

<220>
<223> Description of Artificial Sequence: Synthetic probe

<400> 190
gtgttgtgga ttaaacttgt gcgct 25

<210> 191
<211> 25
<212> DNA
<213> Artificial Sequence

<220>

<223> Description of Artificial Sequence: Synthetic probe

<400> 191
gtgttgtgga ttaaatttgt gcgct 25

<210> 192
<211> 25
<212> DNA
<213> Artificial Sequence

<220>
<223> Description of Artificial Sequence: Synthetic probe

<400> 192
gcgcacaagt ttaatccaca acaca 25

<210> 193
<211> 25
<212> DNA
<213> Artificial Sequence

<220>
<223> Description of Artificial Sequence: Synthetic probe

<400> 193
gcgcacaaat ttaatccaca acaca 25

<210> 194
<211> 25
<212> DNA
<213> Artificial Sequence

<220>
<223> Description of Artificial Sequence: Synthetic probe

<400> 194
ttatttaagc gcacaagttt aatcc 25

<210> 195
<211> 25
<212> DNA
<213> Artificial Sequence

<220>
<223> Description of Artificial Sequence: Synthetic probe

<400> 195
ttatttaagc gcacaaattt aatcc 25

<210> 196
<211> 25
<212> DNA
<213> Artificial Sequence

<220>
<223> Description of Artificial Sequence: Synthetic probe

<400> 196

tttaagcgca caagtttaat ccaca 25

<210> 197
<211> 25
<212> DNA
<213> Artificial Sequence

<220>
<223> Description of Artificial Sequence: Synthetic probe

<400> 197
tttaagcgca caaatttaat ccaca 25

<210> 198
<211> 25
<212> DNA
<213> Artificial Sequence

<220>
<223> Description of Artificial Sequence: Synthetic probe

<400> 198
gtggattaaa tttgtgcgct taaat 25

<210> 199
<211> 25
<212> DNA
<213> Artificial Sequence

<220>
<223> Description of Artificial Sequence: Synthetic probe

<400> 199
gtggattaaa cttgtgcgct taaat 25

<210> 200
<211> 25
<212> DNA
<213> Artificial Sequence

<220>
<223> Description of Artificial Sequence: Synthetic probe

<400> 200
tgtgttgtgg attaaatttg tgcgc 25

<210> 201
<211> 25
<212> DNA
<213> Artificial Sequence

<220>
<223> Description of Artificial Sequence: Synthetic probe

<400> 201
tgtgttgtgg attaaacttg tgcgc 25

<210> 202

<211> 25
<212> DNA
<213> Artificial Sequence

<220>
<223> Description of Artificial Sequence: Synthetic probe

<400> 202
cacaaatgat agcagtaaga taaat 25

<210> 203
<211> 25
<212> DNA
<213> Artificial Sequence

<220>
<223> Description of Artificial Sequence: Synthetic probe

<400> 203
tttatcttac tgctaccatt tgtgt 25

<210> 204
<211> 25
<212> DNA
<213> Artificial Sequence

<220>
<223> Description of Artificial Sequence: Synthetic probe

<400> 204
aaaacacaaa tgatagcagt aagat 25

<210> 205
<211> 25
<212> DNA
<213> Artificial Sequence

<220>
<223> Description of Artificial Sequence: Synthetic probe

<400> 205
aaaacacaaa tggtagcagt aagat 25

<210> 206
<211> 25
<212> DNA
<213> Artificial Sequence

<220>
<223> Description of Artificial Sequence: Synthetic probe

<400> 206
aaacacaaat ggtagcagta agata 25

<210> 207
<211> 25
<212> DNA
<213> Artificial Sequence

<220>
<223> Description of Artificial Sequence: Synthetic probe

<400> 207
aaacacaaat gatagcagta agata 25

<210> 208
<211> 25
<212> DNA
<213> Artificial Sequence

<220>
<223> Description of Artificial Sequence: Synthetic probe

<400> 208
atcttactgc taccatttgt gtttt 25

<210> 209
<211> 25
<212> DNA
<213> Artificial Sequence

<220>
<223> Description of Artificial Sequence: Synthetic probe

<400> 209
atcttactgc tatcatttgt gtttt 25

<210> 210
<211> 25
<212> DNA
<213> Artificial Sequence

<220>
<223> Description of Artificial Sequence: Synthetic probe

<400> 210
attgaaaaca caaatggtag cagta 25

<210> 211
<211> 25
<212> DNA
<213> Artificial Sequence

<220>
<223> Description of Artificial Sequence: Synthetic probe

<400> 211
tttatcttac tgctatcatt tgtgt 25

<210> 212
<211> 25
<212> DNA
<213> Artificial Sequence

<220>
<223> Description of Artificial Sequence: Synthetic probe

<400> 212
gaaaacacaa atggtagcag taaga 25

<210> 213
<211> 25
<212> DNA
<213> Artificial Sequence

<220>
<223> Description of Artificial Sequence: Synthetic probe

<400> 213
gaaaacacaa atgatagcag taaga 25

<210> 214
<211> 25
<212> DNA
<213> Artificial Sequence

<220>
<223> Description of Artificial Sequence: Synthetic probe

<400> 214
cacaaatggt agcagtaaga taaat 25

<210> 215
<211> 25
<212> DNA
<213> Artificial Sequence

<220>
<223> Description of Artificial Sequence: Synthetic probe

<400> 215
atttatctta ctgctatcat ttgtg 25

<210> 216
<211> 25
<212> DNA
<213> Artificial Sequence

<220>
<223> Description of Artificial Sequence: Synthetic probe

<400> 216
tgaaaacaca aatggtagca gtaag 25

<210> 217
<211> 25
<212> DNA
<213> Artificial Sequence

<220>
<223> Description of Artificial Sequence: Synthetic probe

<400> 217
atttatctta ctgctaccat ttgtg 25

<210> 218
<211> 25
<212> DNA
<213> Artificial Sequence

<220>
<223> Description of Artificial Sequence: Synthetic probe

<400> 218
tgaaaacaca aatgatagca gtaag 25

<210> 219
<211> 25
<212> DNA
<213> Artificial Sequence

<220>
<223> Description of Artificial Sequence: Synthetic probe

<400> 219
cttactgcta ccatttgtgt tttca 25

<210> 220
<211> 25
<212> DNA
<213> Artificial Sequence

<220>
<223> Description of Artificial Sequence: Synthetic probe

<400> 220
cttactgcta tcatttgtgt tttca 25

<210> 221
<211> 25
<212> DNA
<213> Artificial Sequence

<220>
<223> Description of Artificial Sequence: Synthetic probe

<400> 221
attgaaaaca caaatgatag cagta 25

<210> 222
<211> 25
<212> DNA
<213> Artificial Sequence

<220>
<223> Description of Artificial Sequence: Synthetic probe

<400> 222
acctgcaaat ccgtaaagtg tacta 25

<210> 223
<211> 25
<212> DNA

<213> Artificial Sequence

<220>
<223> Description of Artificial Sequence: Synthetic probe

<400> 223
acctgcaaat ctgtaaagtg tacta 25

<210> 224
<211> 25
<212> DNA
<213> Artificial Sequence

<220>
<223> Description of Artificial Sequence: Synthetic probe

<400> 224
agtacacttt acagatttgc aggtt 25

<210> 225
<211> 25
<212> DNA
<213> Artificial Sequence

<220>
<223> Description of Artificial Sequence: Synthetic probe

<400> 225
agtacacttt acggatttgc aggtt 25

<210> 226
<211> 25
<212> DNA
<213> Artificial Sequence

<220>
<223> Description of Artificial Sequence: Synthetic probe

<400> 226
acactttacg gatttgcagg ttttg 25

<210> 227
<211> 25
<212> DNA
<213> Artificial Sequence

<220>
<223> Description of Artificial Sequence: Synthetic probe

<400> 227
aacctgcaaa tctgtaaagt gtact 25

<210> 228
<211> 25
<212> DNA
<213> Artificial Sequence

<220>

<223> Description of Artificial Sequence: Synthetic probe

<400> 228
aacctgcaaa tccgtaaagt gtact 25

<210> 229
<211> 25
<212> DNA
<213> Artificial Sequence

<220>
<223> Description of Artificial Sequence: Synthetic probe

<400> 229
tagtacactt tacggatttg caggt 25

<210> 230
<211> 25
<212> DNA
<213> Artificial Sequence

<220>
<223> Description of Artificial Sequence: Synthetic probe

<400> 230
tagtacactt tacagatttg caggt 25

<210> 231
<211> 25
<212> DNA
<213> Artificial Sequence

<220>
<223> Description of Artificial Sequence: Synthetic probe

<400> 231
atatagtaca ctttacggat ttgca 25

<210> 232
<211> 25
<212> DNA
<213> Artificial Sequence

<220>
<223> Description of Artificial Sequence: Synthetic probe

<400> 232
atatagtaca ctttacagat ttgca 25

<210> 233
<211> 25
<212> DNA
<213> Artificial Sequence

<220>
<223> Description of Artificial Sequence: Synthetic probe

<400> 233

atagtacact ttacggattt gcagg 25

<210> 234
<211> 25
<212> DNA
<213> Artificial Sequence

<220>
<223> Description of Artificial Sequence: Synthetic probe

<400> 234
aaacctgcaa atccgtaaag tgtac 25

<210> 235
<211> 25
<212> DNA
<213> Artificial Sequence

<220>
<223> Description of Artificial Sequence: Synthetic probe

<400> 235
aaacctgcaa atctgtaaag tgtac 25

<210> 236
<211> 25
<212> DNA
<213> Artificial Sequence

<220>
<223> Description of Artificial Sequence: Synthetic probe

<400> 236
gcaaaacctg caaatctgta aagtg 25

<210> 237
<211> 25
<212> DNA
<213> Artificial Sequence

<220>
<223> Description of Artificial Sequence: Synthetic probe

<400> 237
gcaaaacctg caaatccgta aagtg 25

<210> 238
<211> 25
<212> DNA
<213> Artificial Sequence

<220>
<223> Description of Artificial Sequence: Synthetic probe

<400> 238
cctgcaaatc cgtaaagtgt actat 25

<210> 239

<211> 25
<212> DNA
<213> Artificial Sequence

<220>
<223> Description of Artificial Sequence: Synthetic probe

<400> 239
acactttaca gatttgcagg ttttg 25

<210> 240
<211> 25
<212> DNA
<213> Artificial Sequence

<220>
<223> Description of Artificial Sequence: Synthetic probe

<400> 240
cctgcaaatc tgtaaagtgt actat 25

<210> 241
<211> 25
<212> DNA
<213> Artificial Sequence

<220>
<223> Description of Artificial Sequence: Synthetic probe

<400> 241
atagtacact ttacagattt gcagg 25

<210> 242
<211> 25
<212> DNA
<213> Artificial Sequence

<220>
<223> Description of Artificial Sequence: Synthetic probe

<400> 242
ttctttaatg ggtacaaaat gtcaa 25

<210> 243
<211> 25
<212> DNA
<213> Artificial Sequence

<220>
<223> Description of Artificial Sequence: Synthetic probe

<400> 243
tttgacatta tgtacccatt aaaga 25

<210> 244
<211> 25
<212> DNA
<213> Artificial Sequence

<220>
<223> Description of Artificial Sequence: Synthetic probe

<400> 244
tttgacattt tgtacccatt aaaga 25

<210> 245
<211> 25
<212> DNA
<213> Artificial Sequence

<220>
<223> Description of Artificial Sequence: Synthetic probe

<400> 245
taatgggtac ataatgtcaa aaata 25

<210> 246
<211> 25
<212> DNA
<213> Artificial Sequence

<220>
<223> Description of Artificial Sequence: Synthetic probe

<400> 246
ttgacatttt gtacccatta aagaa 25

<210> 247
<211> 25
<212> DNA
<213> Artificial Sequence

<220>
<223> Description of Artificial Sequence: Synthetic probe

<400> 247
ttctttaatg ggtacataat gtcaa 25

<210> 248
<211> 25
<212> DNA
<213> Artificial Sequence

<220>
<223> Description of Artificial Sequence: Synthetic probe

<400> 248
tatttttgac attatgtacc catta 25

<210> 249
<211> 25
<212> DNA
<213> Artificial Sequence

<220>
<223> Description of Artificial Sequence: Synthetic probe

<400> 249
atatttttga cattatgtac ccatt 25

<210> 250
<211> 25
<212> DNA
<213> Artificial Sequence

<220>
<223> Description of Artificial Sequence: Synthetic probe

<400> 250
tttaatgggt acaaaatgtc aaaaa 25

<210> 251
<211> 25
<212> DNA
<213> Artificial Sequence

<220>
<223> Description of Artificial Sequence: Synthetic probe

<400> 251
tttaatgggt acataatgtc aaaaa 25

<210> 252
<211> 25
<212> DNA
<213> Artificial Sequence

<220>
<223> Description of Artificial Sequence: Synthetic probe

<400> 252
atatttttga cattttgtac ccatt 25

<210> 253
<211> 25
<212> DNA
<213> Artificial Sequence

<220>
<223> Description of Artificial Sequence: Synthetic probe

<400> 253
ttgacattat gtacccatta aagaa 25

<210> 254
<211> 25
<212> DNA
<213> Artificial Sequence

<220>
<223> Description of Artificial Sequence: Synthetic probe

<400> 254
ttaatgggta cataatgtca aaaat 25

<210> 255
<211> 25
<212> DNA
<213> Artificial Sequence

<220>
<223> Description of Artificial Sequence: Synthetic probe

<400> 255
ttaatgggta caaaatgtca aaaat 25

<210> 256
<211> 25
<212> DNA
<213> Artificial Sequence

<220>
<223> Description of Artificial Sequence: Synthetic probe

<400> 256
tatttttgac attttgtacc catta 25

<210> 257
<211> 25
<212> DNA
<213> Artificial Sequence

<220>
<223> Description of Artificial Sequence: Synthetic probe

<400> 257
tgggtacata atgtcaaaaa tattt 25

<210> 258
<211> 25
<212> DNA
<213> Artificial Sequence

<220>
<223> Description of Artificial Sequence: Synthetic probe

<400> 258
atttttgaca ttatgtaccc attaa 25

<210> 259
<211> 25
<212> DNA
<213> Artificial Sequence

<220>
<223> Description of Artificial Sequence: Synthetic probe

<400> 259
atttttgaca ttttgtaccc attaa 25

<210> 260
<211> 25
<212> DNA

<213> Artificial Sequence

<220>
<223> Description of Artificial Sequence: Synthetic probe

<400> 260
tgggtacaaa atgtcaaaaa tattt 25

<210> 261
<211> 25
<212> DNA
<213> Artificial Sequence

<220>
<223> Description of Artificial Sequence: Synthetic probe

<400> 261
taatgggtac aaaatgtcaa aaata 25

<210> 262
<211> 25
<212> DNA
<213> Artificial Sequence

<220>
<223> Description of Artificial Sequence: Synthetic probe

<400> 262
ttctttaatg cagagtagga cacag 25

<210> 263
<211> 25
<212> DNA
<213> Artificial Sequence

<220>
<223> Description of Artificial Sequence: Synthetic probe

<400> 263
tcctactctg cgttaaagaa gcctg 25

<210> 264
<211> 25
<212> DNA
<213> Artificial Sequence

<220>
<223> Description of Artificial Sequence: Synthetic probe

<400> 264
tgtgtcctac tctgcattaa agaag 25

<210> 265
<211> 25
<212> DNA
<213> Artificial Sequence

<220>

<223> Description of Artificial Sequence: Synthetic probe

<400> 265
caggcttctt taacgcagag tagga 25

<210> 266
<211> 25
<212> DNA
<213> Artificial Sequence

<220>
<223> Description of Artificial Sequence: Synthetic probe

<400> 266
tactctgcat taaagaagcc tgcat 25

<210> 267
<211> 25
<212> DNA
<213> Artificial Sequence

<220>
<223> Description of Artificial Sequence: Synthetic probe

<400> 267
ctgtgtccta ctctgcatta aagaa 25

<210> 268
<211> 25
<212> DNA
<213> Artificial Sequence

<220>
<223> Description of Artificial Sequence: Synthetic probe

<400> 268
aggcttcttt aatgcagagt aggac 25

<210> 269
<211> 25
<212> DNA
<213> Artificial Sequence

<220>
<223> Description of Artificial Sequence: Synthetic probe

<400> 269
aggcttcttt aacgcagagt aggac 25

<210> 270
<211> 25
<212> DNA
<213> Artificial Sequence

<220>
<223> Description of Artificial Sequence: Synthetic probe

<400> 270

ctgtgtccta ctctgcgtta aagaa 25

<210> 271
<211> 25
<212> DNA
<213> Artificial Sequence

<220>
<223> Description of Artificial Sequence: Synthetic probe

<400> 271
atgcaggctt ctttaatgca gagta 25

<210> 272
<211> 25
<212> DNA
<213> Artificial Sequence

<220>
<223> Description of Artificial Sequence: Synthetic probe

<400> 272
tgtgtcctac tctgcgttaa agaag 25

<210> 273
<211> 25
<212> DNA
<213> Artificial Sequence

<220>
<223> Description of Artificial Sequence: Synthetic probe

<400> 273
ggcttcttta acgcagagta ggaca 25

<210> 274
<211> 25
<212> DNA
<213> Artificial Sequence

<220>
<223> Description of Artificial Sequence: Synthetic probe

<400> 274
gcaggcttct ttaacgcaga gtagg 25

<210> 275
<211> 25
<212> DNA
<213> Artificial Sequence

<220>
<223> Description of Artificial Sequence: Synthetic probe

<400> 275
tcctactctg cattaaagaa gcctg 25

<210> 276

<211> 25
<212> DNA
<213> Artificial Sequence

<220>
<223> Description of Artificial Sequence: Synthetic probe

<400> 276
ttctttaacg cagagtagga cacag 25

<210> 277
<211> 25
<212> DNA
<213> Artificial Sequence

<220>
<223> Description of Artificial Sequence: Synthetic probe

<400> 277
ggcttcttta atgcagagta ggaca 25

<210> 278
<211> 25
<212> DNA
<213> Artificial Sequence

<220>
<223> Description of Artificial Sequence: Synthetic probe

<400> 278
tactctgcgt taaagaagcc tgcat 25

<210> 279
<211> 25
<212> DNA
<213> Artificial Sequence

<220>
<223> Description of Artificial Sequence: Synthetic probe

<400> 279
caggcttctt taatgcagag tagga 25

<210> 280
<211> 25
<212> DNA
<213> Artificial Sequence

<220>
<223> Description of Artificial Sequence: Synthetic probe

<400> 280
atgcaggctt ctttaacgca gagta 25

<210> 281
<211> 25
<212> DNA
<213> Artificial Sequence

<220>
<223> Description of Artificial Sequence: Synthetic probe

<400> 281
gcaggcttct ttaatgcaga gtagg 25

<210> 282
<211> 25
<212> DNA
<213> Artificial Sequence

<220>
<223> Description of Artificial Sequence: Synthetic probe

<400> 282
atttttcctg catggcaaga gttta 25

<210> 283
<211> 25
<212> DNA
<213> Artificial Sequence

<220>
<223> Description of Artificial Sequence: Synthetic probe

<400> 283
atttttcctg catagcaaga gttta 25

<210> 284
<211> 25
<212> DNA
<213> Artificial Sequence

<220>
<223> Description of Artificial Sequence: Synthetic probe

<400> 284
cctaaactct tgccatgcag gaaaa 25

<210> 285
<211> 25
<212> DNA
<213> Artificial Sequence

<220>
<223> Description of Artificial Sequence: Synthetic probe

<400> 285
tcctaaactc ttgctatgca ggaaa 25

<210> 286
<211> 25
<212> DNA
<213> Artificial Sequence

<220>
<223> Description of Artificial Sequence: Synthetic probe

<400> 286
tcctaaactc ttgccatgca ggaaa 25

<210> 287
<211> 25
<212> DNA
<213> Artificial Sequence

<220>
<223> Description of Artificial Sequence: Synthetic probe

<400> 287
taatttttcc tgcatggcaa gagtt 25

<210> 288
<211> 25
<212> DNA
<213> Artificial Sequence

<220>
<223> Description of Artificial Sequence: Synthetic probe

<400> 288
tttcctgcat ggcaagagtt tagga 25

<210> 289
<211> 25
<212> DNA
<213> Artificial Sequence

<220>
<223> Description of Artificial Sequence: Synthetic probe

<400> 289
taatttttcc tgcatagcaa gagtt 25

<210> 290
<211> 25
<212> DNA
<213> Artificial Sequence

<220>
<223> Description of Artificial Sequence: Synthetic probe

<400> 290
ttttcctgca tggcaagagt ttagg 25

<210> 291
<211> 25
<212> DNA
<213> Artificial Sequence

<220>
<223> Description of Artificial Sequence: Synthetic probe

<400> 291
tttcctgcat agcaagagtt tagga 25

<210> 292
<211> 25
<212> DNA
<213> Artificial Sequence

<220>
<223> Description of Artificial Sequence: Synthetic probe

<400> 292
cctaaactct tgctatgcag gaaaa 25

<210> 293
<211> 25
<212> DNA
<213> Artificial Sequence

<220>
<223> Description of Artificial Sequence: Synthetic probe

<400> 293
ataatttttc ctgcatggca agagt 25

<210> 294
<211> 25
<212> DNA
<213> Artificial Sequence

<220>
<223> Description of Artificial Sequence: Synthetic probe

<400> 294
ataatttttc ctgcatagca agagt 25

<210> 295
<211> 25
<212> DNA
<213> Artificial Sequence

<220>
<223> Description of Artificial Sequence: Synthetic probe

<400> 295
tcctgcatgg caagagttta ggaga 25

<210> 296
<211> 25
<212> DNA
<213> Artificial Sequence

<220>
<223> Description of Artificial Sequence: Synthetic probe

<400> 296
tcctgcatag caagagttta ggaga 25

<210> 297
<211> 25
<212> DNA

<213> Artificial Sequence

<220>
<223> Description of Artificial Sequence: Synthetic probe

<400> 297
aactcttgcc atgcaggaaa aatta 25

<210> 298
<211> 25
<212> DNA
<213> Artificial Sequence

<220>
<223> Description of Artificial Sequence: Synthetic probe

<400> 298
aactcttgct atgcaggaaa aatta 25

<210> 299
<211> 25
<212> DNA
<213> Artificial Sequence

<220>
<223> Description of Artificial Sequence: Synthetic probe

<400> 299
ttttcctgca tagcaagagt ttagg 25

<210> 300
<211> 25
<212> DNA
<213> Artificial Sequence

<220>
<223> Description of Artificial Sequence: Synthetic probe

<400> 300
ctaaactctt gctatgcagg aaaaa 25

<210> 301
<211> 25
<212> DNA
<213> Artificial Sequence

<220>
<223> Description of Artificial Sequence: Synthetic probe

<400> 301
ctaaactctt gccatgcagg aaaaa 25

<210> 302
<211> 25
<212> DNA
<213> Artificial Sequence

<220>

<223> Description of Artificial Sequence: Synthetic probe

<400> 302
tggaaacatg gttggtccga atgtt 25

<210> 303
<211> 25
<212> DNA
<213> Artificial Sequence

<220>
<223> Description of Artificial Sequence: Synthetic probe

<400> 303
tggaaacatg gttagtccga atgtt 25

<210> 304
<211> 25
<212> DNA
<213> Artificial Sequence

<220>
<223> Description of Artificial Sequence: Synthetic probe

<400> 304
ggaaacatgg ttagtccgaa tgtta 25

<210> 305
<211> 25
<212> DNA
<213> Artificial Sequence

<220>
<223> Description of Artificial Sequence: Synthetic probe

<400> 305
ggaaacatgg ttggtccgaa tgtta 25

<210> 306
<211> 25
<212> DNA
<213> Artificial Sequence

<220>
<223> Description of Artificial Sequence: Synthetic probe

<400> 306
agtggaaaca tggttagtcc gaatg 25

<210> 307
<211> 25
<212> DNA
<213> Artificial Sequence

<220>
<223> Description of Artificial Sequence: Synthetic probe

<400> 307

agtggaaaca tggttggtcc gaatg 25

<210> 308
<211> 25
<212> DNA
<213> Artificial Sequence

<220>
<223> Description of Artificial Sequence: Synthetic probe

<400> 308
attaacattc ggaccaacca tgttt 25

<210> 309
<211> 25
<212> DNA
<213> Artificial Sequence

<220>
<223> Description of Artificial Sequence: Synthetic probe

<400> 309
attaacattc ggactaacca tgttt 25

<210> 310
<211> 25
<212> DNA
<213> Artificial Sequence

<220>
<223> Description of Artificial Sequence: Synthetic probe

<400> 310
gaaacatggt tggtccgaat gttaa 25

<210> 311
<211> 25
<212> DNA
<213> Artificial Sequence

<220>
<223> Description of Artificial Sequence: Synthetic probe

<400> 311
gaaacatggt tagtccgaat gttaa 25

<210> 312
<211> 25
<212> DNA
<213> Artificial Sequence

<220>
<223> Description of Artificial Sequence: Synthetic probe

<400> 312
aacattcgga ccaaccatgt ttcca 25

<210> 313

<211> 25
<212> DNA
<213> Artificial Sequence

<220>
<223> Description of Artificial Sequence: Synthetic probe

<400> 313
aacattcgga ctaaccatgt ttcca 25

<210> 314
<211> 25
<212> DNA
<213> Artificial Sequence

<220>
<223> Description of Artificial Sequence: Synthetic probe

<400> 314
acatggttag tccgaatgtt aatct 25

<210> 315
<211> 25
<212> DNA
<213> Artificial Sequence

<220>
<223> Description of Artificial Sequence: Synthetic probe

<400> 315
acatggttgg tccgaatgtt aatct 25

<210> 316
<211> 25
<212> DNA
<213> Artificial Sequence

<220>
<223> Description of Artificial Sequence: Synthetic probe

<400> 316
tagtggaaac atggttggtc cgaat 25

<210> 317
<211> 25
<212> DNA
<213> Artificial Sequence

<220>
<223> Description of Artificial Sequence: Synthetic probe

<400> 317
tagtggaaac atggttagtc cgaat 25

<210> 318
<211> 25
<212> DNA
<213> Artificial Sequence

<220>
<223> Description of Artificial Sequence: Synthetic probe

<400> 318
cattcggacc aaccatgttt ccact 25

<210> 319
<211> 25
<212> DNA
<213> Artificial Sequence

<220>
<223> Description of Artificial Sequence: Synthetic probe

<400> 319
cattcggact aaccatgttt ccact 25

<210> 320
<211> 25
<212> DNA
<213> Artificial Sequence

<220>
<223> Description of Artificial Sequence: Synthetic probe

<400> 320
taacattcgg accaaccatg tttcc 25

<210> 321
<211> 25
<212> DNA
<213> Artificial Sequence

<220>
<223> Description of Artificial Sequence: Synthetic probe

<400> 321
taacattcgg actaaccatg tttcc 25

## Description

### SEQUENCE LISTING

The instant application contains a Sequence Listing which has been submitted via EFS-Web and is hereby incorporated by reference in its entirety. Said ASCII copy is named 9700.txt and is 79 kilobytes in size.

### TECHNICAL FIELD

The present invention relates to diagnostic assays useful in classification of patients for selection of cancer therapy with one or more Aurora kinase B inhibitors. In particular, the present invention relates to identifying the presence or absence of one or more copy number gains in the ABCB1 gene, identifying patients eligible to receive Aurora kinase inhibitor therapy, either as monotherapy or as part of combination therapy, and monitoring patient response to such therapy.

### BACKGROUND

The Aurora kinase family is a group of highly related serine/threonine kinases that function as key regulators of mitosis. Three Aurora kinases are expressed in mammalian cells. These Aurora kinases are Aurora A, Aurora B, and Aurora C. Each of these Aurora kinases exhibits a different subcellular localization and plays a distinct role (See, Carmena M.E.W., Nat. Rev. Mol. Cell Biol., 4:842-854 (2003) and (Ducat, D.Z.Y., Exp. Cell Res., 301:60-67 (2004)). Specifically, Aurora A localizes to spindle poles and has a crucial role in bipolar spindle formation (See, Marumoto, T.Z.D., et al., Nat. Rev. Cancer, 5:42-50 (2005)). Aurora B, a chromosome passenger protein, localizes to centromeres in early mitosis and then the spindle midzone in anaphase. Aurora B is required for mitotic histone H3 phosphorylation, chromosome biorientation, the spindle assembly checkpoint and cytokinesis (Andrews, P.D., et al., Curr. Opin. Cell Biol., 15:672-683 (2003)). Aurora C is also a chromosomal passenger protein and, in normal cells, its expression is restricted to the testis where it functions primarily in male gametogenesis. As the Aurora kinases serve essential functions in mitosis, considerable attention has been given to targeting this family of kinases for cancer therapy. Several small-molecule inhibitors have been developed including Hesperadin, ZM447439, VX-680/MK0457, AZD1152 and MLN8054 (See, Ditchfield, C, J.V., et al., J. Cell Biol., 161:267-280 (2003), Harrington, E.A., et al., Nat. Med., 10:262-267 (2004), Hauf, S., et al., J. Cell Biol., 161:281-294 (2003), Manfredi, M.G., et al., Proc. Natl. Acad. Sci., USA, 104:4106-4111 (2007)).

AZD1152 is a novel acetanilide-substituted pyrazole-aminoquinazoline prodrug that is rapidly converted to the active drug, AZD1152 HQPA, in human plasma (See, Mortlock, A.A., et al., J. Med. Chem., 50:2213-2224 (2007)). AZD1152 HQPA is a highly potent and selective inhibitor of Aurora B ($K_i$ of 0.36 nM) compared to Aurora A ($K_i$ of 1369 nM) and is inactive against a panel of 50 other kinases. AZD1152 potently inhibits the growth of human colon, lung, and hematologic tumor xenografts in immunodeficient mice. Detailed pharmacodynamic analysis in SW620 colorectal tumor-bearing athymic rats treated intravenously with AZD1152 revealed a temporal sequence of phenotypic events in tumors: transient suppression of histone H3 phosphorylation, accumulation of cells with 4n DNA, followed by an increase in the proportion of polyploid (>4n DNA) cells. Histologic analysis has shown aberrant cell division concurrent with an increase in apoptosis in AZD1152-treated tumors, namely, transient myelosuppression was observed secondary to inhibition of proliferation of the bone marrow, though this effect was fully reversible following cessation of AZD1152 treatment (See, Wilkinson, R.W., et al., Clin. Cancer Res., 13:3683-3688 (2007)).

A major obstacle faced during cancer chemotherapy is the development of cross-resistance of tumors to cytotoxic agents, even to drugs to which the tumor cells were never exposed. This phenotype, known as multidrug resistance (MDR), is frequently observed following treatment with anticancer drugs. While the molecular basis for MDR is often complex, upregulation of members of the ATP-binding cassette (ABC) transporter superfamily has emerged as a core, cell-autonomous mechanism utilized by tumor cells to escape the activity of chemotherapeutic drugs that are pervasive among first- and second-line standards of care. As individuals that have failed previous chemotherapy are those most likely to receive newer, experimental medicines, MDR susceptibility represents a significant hurdle in drug development in oncology. The prototypical ABC transporter, multidrug resistance 1 (MDR1; also known as P-glycoprotein or P-gp; encoded by the gene ABCB1) is composed of two transmembrane domains and two nucleotide binding domains, which, through the hydrolysis of ATP, transports solutes against a concentration gradient into the extracellular space. Other ABC transporters, such as breast cancer resistance protein (BRCP, which is encoded by the gene ABCG2) are expressed as half-transporters and dimerize to yield a mature, functional unit. Although the contribution of BRCP to resistance to chemotherapy is not yet clear, upregulation of MDR1 has been consistently prognostic of failure of chemotherapy and poor survival in individuals with acute myelogenous leukemia (AML) or myelodysplastic syndrome (Pallis, M.R.N., Leukemia, 18:1927-1930 (2004) and van der Holt, B.L.B., et al., Blood, 106:2646-2654 (2005)). Furthermore, MDR1 has been associated with reduced response to chemotherapy in a meta-analysis of 31 breast cancer trials (See, Trock, B.J., et al., J. Natl. Cancer Inst., 89:917-931 1 (1997)). As a result, considerable effort has been invested in the development of substances that inhibit or modulate one or more ABC transporters. In fact, second- and third-generation inhibitors of this type are being evaluated as chemosensitizers in clinical trials (Bates, S.F., et al, Novartis Found Symp., 83-96 (2002)).

Although AZD1152 has shown desirable preclinical efficacy and is being evaluated in Phase I/II clinical trials in AML and solid tumors, the potential for development of resistance to AZD1152 has not been explored. Thus, there is a need in the art to identify the genes that confer tumor cell resistance to subjects being treated with Aurora kinase B inhibitors and to use the information obtained from these genes, such as the up regulation or down regulation of proteins enclosed by these genes, to develop diagnostic methods for determining or classifying whether a patient is eligible for treatment with an Aurora kinase B inhibitor and methods for monitoring patients suffering from cancer and being treated with one or more Aurora kinase B inhibitors for the development of drug resistance.

As further background art, Girdler F. et al, Chemistry & Biology 15, 552-562, June 2008 identified point mutations in the Aurora B kinase gene conferring resistance to the Aurora B kinase inhibitor ZM447439.

## SUMMARY

In a first aspect, the present invention relates to a method of classifying a patient for eligibility for treatment with an Aurora kinase B inhibitor. The method comprises the steps of:

a) providing a test sample from a patient;
b) determining the presence or absence of a copy number gain for the ABCB1 gene at chromosome locus 7q21.1; and
c) classifying the patient as being eligible for receiving treatment with an Aurora kinase B inhibitor based on the presence or absence of a copy number gain for the ABCB1 gene at chromosome locus 7q21.1, wherein the presence of a copy number gain for the ABCB1 gene at chromosome locus 7q21.1 classifies the patient as ineligible for receiving treatment with an Aurora kinase B inhibitor for the treatment of cancer.

In the above aspect, the Aurora kinase B inhibitor can be AZD1152, ZM447439, VX-680/MK0457 or Hersperadin.

In the above aspect, test sample can comprise a tissue sample. Specifically, the tissue sample comprises a peripheral blood sample, a tumor tissue or a suspected tumor tissue, a thin layer cytological sample, a fine needle aspirate sample, a bone marrow sample, a lymph node sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample, a paraffin embedded tissue sample or an extract or processed sample produced from any of a peripheral blood sample, a tumor tissue or a suspected tumor tissue, a thin layer cytological sample, a fine needle aspirate sample, a bone marrow sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample or a paraffin embedded tissue sample.

In the above aspect, the determining step (b) can be performed by *in situ* hybridization. Specifically, the *in situ* hybridization can be performed with a nucleic acid probe that is fluorescently labeled. More specifically, the *in situ* hybridization can be performed with at least two nucleic acid probes. Alternatively, the *in situ* hybridization is performed with a peptide nucleic acid probe.

Alternatively, in the above aspect, the determining step (b) can be performed by polymerase chain reaction.

Still further alternatively, the determining step (b) can be performed by a nucleic acid microarray assay.

In the above aspect, the cancer can be colorectal carcinoma or pancreatic carcinoma.

In the above aspect, the presence of a copy number gain in the ABCB1 gene correlates with an increase in expression of the MDR1 polypeptide.

In the above aspect, the patient is being treated with an anti-sense agent designed to bind to the ABCB1 gene.

In the above aspect, the patient can also optionally be treated with chemotherapy, radiation or combinations thereof.

In a second aspect, the present invention relates to a method of monitoring a patient suffering from cancer and being treated with an Aurora kinase B inhibitor. The method comprises the steps of:

a) providing a test sample from a patient suffering from cancer and currently being treated with at least one Aurora kinase B inhibitor;
b) determining the presence or absence of a copy number gain for the ABCB1 gene at chromosome locus 7q21.1;
c) comparing the copy number of the ABCB1 gene in the test sample against a baseline level or a predetermined level, wherein the baseline level refers to the copy number for the ABCB1 gene in a sample taken from the patient at the initiation of Aurora kinase B inhibitor therapy and wherein the predetermined level refers to an assay cut-off value associated with the progression of cancer; and

d) determining whether the patient should continue to be treated with the Aurora kinase B inhibitor based on the comparison in step c), wherein if the test sample has a copy number gain for the ABCB1 gene at chromosome locus 7q21.1 that is less than the baseline level or the predetermined level or if no copy number is detected, treatment of the patient with the Aurora kinase B inhibitor continues, and wherein if the test sample has a copy number gain for the ABCB1 gene at chromosome locus 7q21.1 that is the same as or higher than the baseline level or the predetermined level, treatment of the patient with the Aurora kinase B inhibitor is discontinued or combined with at least a second therapy as a combination therapy.

In the above second aspect, the Aurora kinase B inhibitor can be AZD1152, ZM447439, VX-680/MK0457 or Hersperadin.

In the above second aspect, test sample can comprise a tissue sample. Specifically, the tissue sample comprises a peripheral blood sample, a tumor tissue or a suspected tumor tissue, a thin layer cytological sample, a fine needle aspirate sample, a bone marrow sample, a lymph node sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample, a paraffin embedded tissue sample or an extract or processed sample produced from any of a peripheral blood sample, a tumor tissue or a suspected tumor tissue, a thin layer cytological sample, a fine needle aspirate sample, a bone marrow sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample or a paraffin embedded tissue sample.

In the above second aspect, the determining step (b) can be performed by *in situ* hybridization. Specifically, the *in situ* hybridization can be performed with a nucleic acid probe that is fluorescently labeled. More specifically, the *in situ* hybridization can be performed with at least two nucleic acid probes. Alternatively, the *in situ* hybridization is performed with a peptide nucleic acid probe.

Alternatively, in the above second aspect, the determining step (b) can be performed by polymerase chain reaction.

Still further alternatively, the determining step (b) can be performed by a nucleic acid microarray assay.

In the above second aspect, the cancer can be colorectal carcinoma or pancreatic carcinoma.

In the second aspect, the presence of a copy number gain in the ABCB1 gene correlates with an increase in expression of the MDR1 polypeptide.

In the above second aspect, the patient is being treated with an anti-sense agent designed to bind to the ABCB1 gene.

In the above second aspect, the patient can also optionally be treated with chemotherapy, radiation or combinations thereof.

In a third aspect, the present invention relates to a method of classifying a patient having a cancer that is resistant to treatment with an Aurora kinase B inhibitor. The method comprises the steps of:

a) providing a test sample from a patient;
b) determining the presence or absence of a copy number gain for the ABCB1 gene at chromosome locus 7q21.1;
c) comparing the presence or absence of the copy number gain for the ABCB 1 gene in the test sample against a baseline level or a predetermined level, wherein the baseline level refers to the copy number for the ABCB1 gene in a sample taken from the patient at the initiation of Aurora kinase B inhibitor therapy and wherein the predetermined level refers to an assay cut-off value associated with resistance to Aurora kinase B inhibitor treatment; and
d) classifying the patient as having a cancer that is resistant to Aurora kinase B inhibitor treatment on (i) the presence of a copy number gain in the ABCB1 gene at chromosome locus 7q21.1; and (ii) if the copy number gain in the test sample is higher than the baseline level or the predetermined level.

In the above third aspect, the Aurora kinase B inhibitor can be AZD1152, ZM447439, VX-680/MK0457 or Hersperadin.

In the above third aspect, test sample can comprise a tissue sample. Specifically, the tissue sample comprises a peripheral blood sample, a tumor tissue or a suspected tumor tissue, a thin layer cytological sample, a fine needle aspirate sample, a bone marrow sample, a lymph node sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample, a paraffin embedded tissue sample or an extract or processed sample produced from any of a peripheral blood sample, a tumor tissue or a suspected tumor tissue, a thin layer cytological sample, a fine needle aspirate sample, a bone marrow sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample or a paraffin embedded tissue sample.

In the above third aspect, the determining step (b) can be performed by *in situ* hybridization. Specifically, the *in situ* hybridization can be performed with a nucleic acid probe that is fluorescently labeled. More specifically, the *in situ* hybridization can be performed with at least two nucleic acid probes. Alternatively, the *in situ* hybridization is performed with a peptide nucleic acid probe.

Alternatively, in the above third aspect, the determining step (b) can be performed by polymerase chain reaction.

Still further alternatively, the determining step (b) can be performed by a nucleic acid microarray assay.

In the above third aspect, the cancer can be colorectal carcinoma or pancreatic carcinoma.

In the third aspect, the presence of a copy number gain in the ABCB1 gene correlates with an increase in expression of the MDR1 polypeptide.

In the above third aspect, the patient is being treated with an anti-sense agent designed to bind to the ABCB1 gene.

In the above third aspect, the patient can also optionally be treated with chemotherapy, radiation or combinations thereof.

## BRIEF DESCRIPTION OF THE DRAWINGS

**Figure 1** shows the identification of ABCB1 and ABCB4 as genes amplified and overexpressed in an SW620 derivative selected for resistance to AZD1152 HQPA as described in the Example. Specifically, **Figure 1A** shows the copy number of ABCB1 and ABCB4 determined by CGH using Affymetrix 100K SNP chips in parental SW620 cells, $SW620^{ABCB1/3}$ cells, and $SW620^{ABCB1/3}$ cells after 3 months in culture in drug-free medium. The vertical line indicates the position of the ABCB1 locus and the horizontal line indicates the normal DNA copy number (two copies). **Figure 1B** shows mRNA expression values for ABCB1 and ABCB4 compared to other solute transporters. The expression levels of ABCB1 (encoding MDR1) and ABCB4 (encoding MDR3) in $SW620^{ABCB1/3}$ are indicated by arrows. **Figure 1C** shows the mRNA expression values for over 14,000 genes plus ESTs (~22,000 probe sets) determined using Affymetrix HG-U133A GeneChips. Data are presented as the fold change in gene expression for $SW620^{ABCB1/3}$ cells compared to the parental SW620 cells compared to all genes whose expression increases 10-fold or greater. **Figure 1D** shows the relative expression of the MDR1 protein was determined by immunoblot analysis. ß-actin was used as a loading control.

**Figure 2** shows that the inhibition of ABCB1 reverses resistance to AZD1152 HQPA in the $SW620^{ABCB1/3}$ derivative. **Figure 2A** shows SW620, $SW620^{ABCB1/3}$, and $SW620^{ABCB1/3}$ cells after 3 months in culture in drug-free medium that were treated with AZD 1152 HQPA in dose response for 90 minutes. Phosphorylation of histone H3 at $Ser^{10}$ was determined by immunoblot analysis. **Figure 2B** shows SW620 or $SW620^{ABCB1/3}$ cells treated with 1 $\mu$M AZD1152 HQPA for 4 hours. Cells were fractionated, and the AZD 1152 HQPA concentration was determined by LC-MS analysis in the respective sample fraction. **Figure 2C** shows $SW620^{ABCB1/3}$ cells treated for 2 hours with either DMSO or 1 $\mu$M PSC-833 prior to AZD1152 HQPA in dose response for 90 minutes. Phosphorylation of histone H3 was then determined by immunoblotting. **Figure 2D** shows the effect of ABCB1 knockdown in the $SW620^{ABCB1/3}$ derivative was assessed by transfecting either Luciferase (siLuciferase) or ABCB1 (siABCB1) siRNAs followed by treatment of the transfected cells with AZD1152 HQPA for 90 minutes. Immunoblot analysis of ABCB1 indicated that protein levels were reduced by about 75% with siABCB1 compared to siLuciferase.

**Figure 3** shows the relationship of pharmacokinetics, pharmacodynamics, and efficacy of AZD1152 HQPA in SW620 vs. $SW620^{ABCB1/3}$ xenografts. **Figure 3A** (top panel) shows the projected threshold intratumor concentration required to inhibit xenograft histone H3 phosphorylation estimated by calculating the product of the intrinsic potency of AZD 1152 HQPA in an assay of histone H3 phosphorylation and the fold reduction in potency of AZD 1152 HQPA when assayed in the presence of 50% ($vv^{-1}$) mouse plasma. The bottom panel shows the intratumor pharmacokinetics of AZD 1152 HQPA determined at 0, 2, 8 and 24 hours post-dose after a single intraperitoneal (i.p.) injection of 100 mg $kg^{-1}$. **Figure 3B** shows mice bearing established SW620 and $SW620^{ABCB1/3}$ tumor xenografts given a single dose of AZD1152 HQPA (100 mg $kg^{-1}$, i.p.), and three tumors per time point were harvested. Tumors were extracted and phospho-histone H3 levels were determined by immunoblotting in SW620 tumor (light blue) and $SW620^{ABCB1/3}$ tumor (dark blue) following treatment with AZD1152. Immunoblots were quantified, and the data expressed as the area under the curve. The mean values from individual time points are present $\pm$s.e.m. **Figure 3C** and **Figure 3D** show SW620 and $SW620^{ABCB1/3}$ cells injected subcutaneously into *scid*-bg mice as described in Example 1. Tumors were size-matched at approximately 500 $mm^3$, and treatment with AZD1152 was initiated on Day 7 post-inoculation. AZD 1152 was administered in a q2d schedule at doses of 50 or 100 mg/kg/day by i.p. injection for 2 weeks. Each point represents the mean $\pm$ s.d. of 10 tumors.

**Figure 4** shows that cell lines which overexpress ABCB1 are resistant to AZD1152 HQPA and VX-680/MK0457 *in*

*vitro.* **Figure 4A** presents immunoblotting of cell lines used in xenograft studies showing the relative expression of ABCB 1. **Figure 4B** shows a panel of cell lines that were evaluated for relative sensitivity to AZD1152 HQPA, VX-680/MK0457, MLN8054, and paclitaxel in 7-day colony formation (adherent lines: SW620, $SW620^{ABCB1/3}$, HCT-15, AsPCl) or viability (non-adherent lines: RS; 411 and DoHH-2). Cells were treated in dose response to determine $IC_{50}$s. **Figure 4C** shows HCT-15 cells that were treated with DMSO or 1 $\mu$M PSC-833 for 1 hour prior to the addition of AZD1152 HQPA at the indicated concentrations for an additional hour. Total and phospho-($Ser^{10}$)-histone H3 was determined by immunoblotting. **Figure 4D** shows AsPCl cells that were treated with DMSO or 10 $\mu$M fumitremorgin C for 1 hour followed by AZD1152 HQPA as described in **Figure 4C.**

## DETAILED DESCRIPTION

The present invention provides methods and compositions for monitoring cancer and tumor cells for resistance to Aurora kinase B inhibitor therapy. The inventors discovered that the presence of a copy number gain for the ABCB1 gene at chromosome locus 7q21.1 is associated with resistance to therapy with an Aurora kinase B inhibitor.

The inventors discovered the copy number gains described above using a microarray-based comparative genomic hybridization technique to detect gene copy number abnormalities (e.g, copy number gain and copy number loss) on a genome-wide scale, thus providing a whole-genome view of chromosomal aberrations accompanied by a change in the DNA copy number. This method is fully disclosed in METHODS FOR ASSEMBLING PANELS OF CANCER CELL LINES FOR USE IN TESTING THE EFFICACY OF ONE OR MORE PHARMACEUTICAL COMPOSITIONS, WO 2010/051320.

The invention provides diagnostic assays for identifying, classifying and monitoring cancer patients which comprises assessing a test sample for the presence or absence of a copy number gain for the ABCB1 gene at chromosome locus 7q21.1. The inventive assays include assay methods for identifying patients eligible to receive Aurora kinase B therapy (as either a monotherapy or as part of a combination therapy (e.g., such as with chemotherapy, radiation or combinations thereof) and for monitoring patient response to such therapy. The invention comprises, for example, determining by fluorescent *in situ* hybridization the presence or absence of a copy number gain for the ABCB1 gene at chromosome locus 7q21.1. Patients classified as having an increase in copy number gain for the ABCB1 gene at chromosome locus 7q21.1 are ineligible to receive Aurora kinase B therapy at least as a monotherapy because they are less likely to respond to this therapy. In addition, patients having this amplification can be resistant to other cancer therapies. Thus, determination of the presence of a copy number gain for the ABCB1 gene at chromosome locus 7q21.1 in cancer and tumor cells is useful as a general therapy stratification marker.

In one embodiment, the invention comprises a method for identifying or classifying a patient as eligible for treatment with an Aurora kinase B inhibitor (as either a monotherapy or part of a combination therapy), the method comprising the steps of:

(a) providing a tissue sample from a patient;
(b) determining the presence or absence of a copy number gain for a ABCB1 gene at chromosome locus 7q21.1; and
(c) classifying the patient as being eligible for treatment with an Aurora kinase B inhibitor based on the absence of a copy number gain for a ABCB1 gene at chromosome locus 7q21.1. In the above method, a patient would be ineligible for treatment with an Aurora kinase B inhibitor (at least as a monotherapy) based on the presence of a copy number gain for a ABCB1 gene at chromosome locus 7q21.1. The patient from whom the test sample is obtained can be a patient suspected of or diagnosed with cancer. Moreover, the inventors found that a copy number gain in the ABCB1 gene correlates with an increase in expression of the MDR1 polypeptide.

In this embodiment, the cancer can be any type of cancer, such as colorectal carcinoma or pancreatic cancer. Moreover, in this embodiment, the gene amplification can be determined by a multi-color fluorescent *in situ* hybridization (FISH) assay, for example, performed on a lung cancer tumor biopsy sample. In other embodiments, the quantitative polymerase chain reaction (Q-PCR) method is used.

In yet another embodiment, the invention comprises a method for identifying or classifying a patient having a cancer that is resistant to therapy with an Aurora kinase B inhibitor, the method comprising the steps of:

(a) providing a test sample (*e.g.*, such as a tissue sample) from a patient;
(b) determining the presence or absence of a copy number gain for a ABCB1 gene at chromosome locus 7q21.1; and
(c) classifying the patient as having a cancer that is resistant to Aurora kinase B inhibitor based on the presence of a copy number gain for a ABCB1 gene at chromosome locus 7q21.1.

In this embodiment, the cancer can be any type of cancer, such as colorectal carcinoma or pancreatic cancer. Moreover, in this embodiment, the gene amplification can be determined by a multi-color fluorescent *in situ* hybridization (FISH) assay, for example, performed on a lung cancer tumor biopsy sample. In other embodiments, the polymerase chain reaction (PCR) is used.

In still yet another embodiment, the invention is directed to methods for monitoring a patient being treated with an Aurora kinase B inhibitor, the method comprising the steps of:

(a) providing a test sample from a cancer patient being treated with at least one Aurora kinase inhibitor (optionally, tumor or cancer cells obtained from a tissue sample can be identified or extracted);
(b) determining in the test sample (for example, in the tumor or cancer cells) the presence or absence of a copy number gain for a ABCB 1 gene at chromosome locus 7q21.1; and
(c) comparing the copy number gain for the ABCB1 gene at chromosome locus 7q21.1 from the test sample (such as in the tumor or cancer cells) against a baseline level or a predetermined level; and
(d) determining whether the patient should continue to be treated with the Aurora kinase B inhibitor based on the comparison in step (c). Specifically, if the test sample (e.g., the tumor or cancer cells) having a copy number gain for the ABCB 1 gene at chromosome locus 7q21.1 is the same as or higher then the baseline level or predetermined level, then treatment with the

Aurora kinase B inhibitor can be discontinued, stopped or terminated (if it is being used solely as a monotherapy). Alternatively, the treating physician may decide to combine the Aurora kinase B inhibitor with at least a second therapy (for example, treatment with a second small molecule) as a combination therapy. However, if the copy number gain for the ABCB1 gene at chromosome locus 7q21.1 obtained from the test sample (*e.g.*, the tumor or cancer cells) is less then the baseline level or the predetermined level or if no copy number gain for the ABCB 1 gene at chromosome locus 7q21.1 is detected, then treatment with the Aurora kinase B inhibitor can be continued. Again, depending on the results obtained with said treatment, the treating physician may decide to combine the Aurora kinase B inhibitor with at least a second therapy (for example, treatment with a second small molecule) as a combination therapy.

Again, FISH and PCR methods can be used to detect the presence or absence of a copy number gain for the ABCB1 gene at chromosome locus 7q21.1 in a test sample obtained from a patient.

Also disclosed herein are kits that package, for example, oligo- or polynucleotides engineered to be used as PCR primers, FISH probes, *etc.*

The invention has significant capability to provide improved stratification of patients for cancer therapy, and in particular for Aurora kinase B inhibitor therapy. The assessment of these biomarkers with the invention also allows tracking of individual patient response to the therapy.

**A. Definitions**

Section headings as used in this section and the entire disclosure herein are not intended to be limiting.

As used herein, the singular forms "a," "an" and "the" include plural referents unless the context clearly dictates otherwise. For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

**a) Aurora Kinase B Inhibitor**

An "Aurora kinase B inhibitor" refers to a therapeutic compound of any type (e.g., non-selective or selective), including small molecule-, antibody-, antisense-, small interfering RNA, or microRNA-based compounds, that binds to at least one of Aurora kinase B or Aurora B, and antagonizes the activity of the Aurora kinase B or Aurora B related nucleic acid or protein. For example, a number of Aurora kinase B inhibitors are known to inhibit at least one of histone H3 phosphorylation or cell division. In addition, a number of Aurora kinase B inhibitors are known to induce apoptosis in at least one cell system (such as an acute myeloid leukemia cell line, a primary acute myeloid leukemia culture, *etc.*) The methods of the present invention are useful with any known or hereafter developed Aurora kinase B inhibitor. Examples of an Aurora kinase B inhibitor are AZD1152, ZM447439, VX-680/MK0457 and Hesperadin.

AZD1152, also known as, 2-[[3-({4-[(5-{2-[(3-Fluorophenyl)amino]-2-oxoethyl}-1*H*-pyrazol-3-yl)amino]-quinazolin-7-yl}oxy)propyl](ethyl)amino]ethyl dihydrogen phosphate, is a prodrug of a pyrazoloquinazoline Aurora kinase inhibitor (AZD1152-hydroxyquinazolien pyrazol anilide (HQPA)) and is converted rapidly to the active AZD1152-HQPA in plasma (See, Mortlock, AA, et al., J. Med. Chem., 50:2213-24 (2007)). AZD1152-HQPA is a highly potent and selective inhibitor of Aurora B.

ZM447439, also known as 4-(4-(*N*-benzoylamino)anilino)-6-methoxy-7-(3-(1-morpholino)propoxy)quinazoline, is a quinazoline derivative, inhibits Aurora A and Aurora B. The chemical structure of ZM447439 is provided in Ditchfield, C., et al., J. Cell Bio., 161(2):267-280 (2003) and Montembault, E., et al., Drugs of the Future, 30(1):1-9 (2005).

VX-680/MK0457 is a cyclopropane carboxylic acid of {4-[4-(4-methyl-piperazin-1-yl)-6-(5-methyl-2H-pyrazol-3-ylamino)-pyrimidin-2-ylsulphanyl]-phenyl}-amide and inhibits Aurora A, Aurora B and Aurora C. The chemical structure of VX-680/MK0457 is provided in Montembault, E., et al., Drugs of the Future, 30(1):1-9 (2005).

Hesperadin, an indolinone, inhibits Aurora B. The chemical structure of Hesperadin is provided in Hauf, S., et al., J. Cell Bio., 161(2):281-294 (2003) and Montembault, E., et al., Drugs of the Future, 30(1):1-9 (2005).

**b) Consisting Essentially of a Polynucleotide Having a % Sequence Identity**

"Consisting essentially of a polynucleotide having a % sequence identity" means that the polynucleotide does not substantially differ in length, but may differ substantially in sequence. Thus, a polynucleotide "A" consisting essentially of a polynucleotide having at least 80% sequence identity to a known sequence "B" of 100 nucleotides means that polynucleotide "A" is about 100 nucleotides (nts) long, but up to 20 nts can vary from the "B" sequence. The polynucleotide sequence in question can be longer or shorter due to modification of the termini, such as, for example, the addition of 1-15 nucleotides to produce specific types of probes, primers and other molecular tools, *etc.,* such as the case of when substantially non-identical sequences are added to create intended secondary structures. Such non-identical nucleotides are not considered in the calculation of sequence identity when the sequence is modified by "consisting essentially of."

**c) Expression, Antisense Inhibition and Co-Suppression**

"Expression" refers to the production of a functional end-product. Expression of a gene involves transcription of the gene and translation of the mRNA into a precursor or mature protein. "Antisense inhibition" refers to the production of antisense RNA transcripts capable of suppressing the expression of the target protein. "Co-suppression" refers to the production of sense RNA transcripts capable of suppressing the expression of identical or substantially similar foreign or endogenous genes (U.S. Patent No. 5,231,020).

**d) Isolated**

As used herein, the term "isolated" in the context of nucleic acid molecules or polynucleotides refers to a nucleic acid molecule or polynucleotide which is separated from other nucleic acid molecules or polynucleotides which are present in the natural source of the nucleic acid molecule or polynucleotide. Moreover, an "isolated" nucleic acid molecule or polynucleotide, such as a cDNA molecule, can be substantially free of other cellular material, or culture medium when produced by recombinant techniques, or substantially free of chemical precursors or other chemicals when chemically synthesized. In one aspect, nucleic acid molecules or polynucleotides are isolated.

**e) Gene**

"Gene" refers to **a** nucleic acid fragment that expresses a specific protein, including regulatory sequences preceding (5' non-coding sequences) and following (3' non-coding sequences) the coding sequence.

**f) Native Gene and Chimeric Construct**

"Native gene" refers to a gene as found in nature with its own regulatory sequences. In contrast, "chimeric construct" refers to a combination of nucleic acid fragments that are not normally found together in nature. Accordingly, a chimeric construct may comprise regulatory sequences and coding sequences that are derived from different sources, or regulatory sequences and coding sequences derived from the same source, but arranged in a manner different than that normally found in nature.

**g) Percent (%) Nucleic Acid Sequence Identity**

"Percent (%) nucleic acid sequence identity" with respect to nucleic acid sequences is defined as the percentage of nucleotides in a candidate sequence that are identical with the nucleotides in the sequence of interest, after aligning the sequences and introducing gaps, if necessary, to achieve the maximum percent sequence identity. Alignment for purposes of determining % nucleic acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

When nucleotide sequences are aligned, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) can be calculated as follows:

$$\% \text{ nucleic acid sequence identity} = W/Z*10($$

where
W is the number of nucleotides scored as identical matches by the sequence alignment program's or algorithm's alignment of C and D
and
Z is the total number of nucleotides in D.

When the length of nucleic acid sequence C is not equal to the length of nucleic acid sequence D, the % nucleic acid sequence identity of C to D will not equal the % nucleic acid sequence identity of D to C.

**h) Polymerase Chain Reaction or PCR**

"Polymerase Chain Reaction" or "PCR" is a technique for the synthesis of large quantities of specific DNA segments, consists of a series of repetitive cycles (Perkin Elmer Cetus Instruments, Norwalk, CT). Typically, the double stranded DNA is heat-denatured, the two primers complementary to the 3' boundaries of the target segment are annealed at low temperature and then extended at an intermediate temperature. One set of these three consecutive steps is referred to as a cycle.

PCR is a powerful technique used to amplify DNA millions of fold, by repeated replication of a template, in a short period of time. ( (Mullis, K., et al., Cold Spring Harb Symp Quant Biol. 51 Pt 1:263-73 (1986)); European Patent Application No. 50,424; European Patent Application No. 84,796; European Patent Application No. 258,017, European Patent Application No. 237,362; European Patent Application No. 201,184, U.S. Patent No. 4,683,202; U.S. Patent No. 4,582,788; and U.S. Patent No. 4,683,194). The process uses sets of specific *in vitro* synthesized oligonucleotides to prime DNA synthesis. The design of the primers is dependent upon the sequences of DNA that are to be analyzed. The technique is carried out through many cycles (usually 20-50) of melting the template at high temperature, allowing the primers to anneal to complementary sequences within the template and then replicating the template with DNA polymerase.

The products of PCR reactions can be analyzed by separation in agarose gels followed by ethidium bromide staining and visualization with UV transillumination. Alternatively, radioactive dNTPs can be added to the PCR in order to incorporate label into the products. In this case the products of PCR are visualized by exposure of the gel to x-ray film. The added advantage of radiolabeling PCR products is that the levels of individual amplification products can be quantitated.

**i) Polynucleotide**

A "polynucleotide" is a nucleic acid polymer of ribonucleic acid (RNA), deoxyribonucleic acid (DNA), modified RNA or DNA, or RNA or DNA mimetics (such as PNAs), and derivatives thereof, and homologues thereof. Thus, polynucleotides include polymers composed of naturally occurring nucleic bases, sugars and covalent inter-nucleoside (backbone) linkages as well as polymers having non-naturally-occurring portions that function similarly. Such modified or substituted nucleic acid polymers are well known in the art and are referred to as "analogues." Oligonucleotides are generally short polynucleotides from about 10 to up to about 160 or 200 nucleotides.

Polynucleotides also comprise primers that specifically hybridize to target sequences, including analogues and/or derivatives of the nucleic acid sequences, and homologues thereof.

Polynucleotides can be prepared by conventional techniques, such as solid-phase synthesis using commercially available equipment, such as that available from Applied Biosystems USA Inc. (Foster City, CA; USA), DuPont, (Wilmington, DE; USA), or Milligen (Bedford, MA; USA). Modified polynucleotides, such as phosphorothioates and alkylated derivatives, can also be readily prepared by similar methods known in the art (See, U.S. Patent Nos. 4,948,882, 5,464,746, and 5,424,414).

**j) Polynucleotide Analogues**

As used herein, the term "polynucleotide analogues" refers to polymers having modified backbones or non-natural inter-nucleoside linkages. Modified backbones include those retaining a phosphorus atom in the backbone, such as phosphorothioates, chiral phosphorothioates, phosphorodithioates, phosphotriesters, aminoalkylphosphotriesters, methyl and other alkyl phosphonates, as well as those no longer having a phosphorus atom, such as backbones formed by short chain alkyl or cycloalkyl inter-nucleoside linkages, mixed heteroatom and alkyl or cycloalkyl inter-nucleoside linkages, or one or more short chain heteroatomic or heterocyclic inter-nucleoside linkages. Modified nucleic acid polymers (analogues) can contain one or more modified sugar moieties.

Analogs that are RNA or DNA mimetics, in which both the sugar and the inter-nucleoside linkage of the nucleotide units are replaced with novel groups, are also useful. In these mimetics, the base units are maintained for hybridization with the target sequence. An example of such a mimetic, which has been shown to have excellent hybridization properties, is a peptide nucleic acid (PNA) (See, Buchardt, O., P. Nielsen, and R. Berg. 1992. Peptide Nucleic Acids).

**k) Predetermined Level**

As used herein, the term "predetermined level" refers generally at an assay cut-off value that is used to assess diagnostic results by comparing the assay results against the predetermined level, and where the predetermined level already that has been linked or associated with various clinical parameters (e.g., assessing risk, severity of disease, progression/non-progression/improvement, determining the age of a test sample, determining whether a test sample (e.g., serum or plasma) has hemolyzed, *etc.).* The present invention provides exemplary predetermined levels, and describes the initial linkage or association of such levels with clinical parameters for exemplary assays as described herein. However, it is well known that cutoff values may vary dependent on the nature of the assay. It further is well within the ordinary skill of one in the art to adapt the invention herein for other assays to obtain assay-specific cut-off values for those other assays based on this description.

**l) Primer or Probe**

A "probe" or "primer" as used herein is a polynucleotide that is at least 8 nucleotides in length and forms a hybrid structure with a target sequence, due to complementarity of at least one sequence in the probe or primer with a sequence in the target region. The polynucleotide regions of the probe can be composed of DNA and/or RNA and/or synthetic nucleotide analogs. Preferably, the probe does not contain a sequence that is complementary to the sequence or sequences used to prime for a target sequence during the polymerase chain reaction.

**m) Recombinant**

"Recombinant" refers to an artificial combination of two otherwise separated segments of sequence, *e.g.*, by chemical synthesis or by the manipulation of isolated segments of nucleic acids by genetic engineering techniques.

**n) Specifically hybridize**

"Specifically hybridize" refers to the ability of a nucleic acid to bind detectably and specifically to a second nucleic acid. Polynucleotides specifically hybridize with target nucleic acid strands under hybridization and wash conditions that minimize appreciable amounts of detectable binding by non-specific nucleic acids.

**o) Stringency or Stringent Conditions**

The specificity of single stranded DNA to hybridize complementary fragments is determined by the stringency of the reaction conditions. Hybridization stringency increases as the propensity to form DNA duplexes decreases. In nucleic acid hybridization reactions, the stringency can be chosen to favor specific hybridizations (high stringency). Less-specific hybridizations (low stringency) can be used to identify related, but not exact, DNA molecules (homologous, but not identical) or segments.

DNA duplexes are stabilized by: (1) the number of complementary base pairs, (2) the type of base pairs, (3) salt concentration (ionic strength) of the reaction mixture, (4) the temperature of the reaction, and (5) the presence of certain organic solvents, such as formamide, which decrease DNA duplex stability. A common approach is to vary the temperature: higher relative temperatures result in more stringent reaction conditions (See, Ausubel, F.M., R. Brent, R.E. Kingston, et al. 1987. Current Protocols in Molecular Biology. John Wiley & Sons, New York) provide an excellent explanation of stringency of hybridization reactions.

Hybridization under "stringent conditions" means hybridization protocols in which nucleotide sequences at least 60% homologous to each other remain hybridized. Polynucleotides can include other appended groups such as peptides (*e.g.*, for targeting host cell receptors *in vivo),* or agents facilitating transport across the cell membrane. In addition, oligonucleotides can be modified with hybridization-triggered cleavage agents (See, van der Krol et al., Biotechniques.

6:958-76 (1988) or intercalculating agents (Zon, G., Pharm Res. 5:539-49 (1988)). The oligonucleotide can be conjugated to another molecule, e.g., a peptide, a hybridization triggered cross-linking agent, a transport agent, a hybridization-triggered cleavage agent, and the like.

**p) Subject(s) or Patient(s)**

As used herein, the terms "subject" and "patient" are used interchangeably irrespective of whether the subject has or is currently undergoing any form of treatment. As used herein, the terms "subject" and "subjects" refer to any vertebrate, including, but not limited to, a mammal (e.g., cow, pig, camel, llama, horse, goat, rabbit, sheep, hamsters, guinea pig, cat, dog, rat, and mouse, a non-human primate (for example, a monkey, such as a cynomolgous monkey, chimpanzee, *etc)* and a human). Preferably, the subject is a human. Subjects or patients can be living or expired.

**q) Target Sequence or Target Nucleic Acid Sequence**

"Target sequence" or "target nucleic acid sequence" means a nucleic acid sequence encompassing, for example, a gene, or complements or fragments thereof, that is amplified, detected, or both using a polynucleotide primer or probe. Additionally, while the term target sequence sometimes refers to a double stranded nucleic acid sequence; a target sequence can also be single-stranded. In cases where the target is double-stranded, polynucleotide primer sequences preferably amplify both strands of the target sequence. A target sequence can be selected that is more or less specific for a particular organism. For example, the target sequence can be specific to an entire genus, to more than one genus, to a species or subspecies, serogroup, auxotype, serotype, strain, isolate or other subset of organisms.

**r) Test sample**

"Test sample" means a sample taken from a subject, or a biological fluid, wherein the sample may contain a target sequence. A test sample can be taken from any source, for example, tissue, blood, saliva, sputa, mucus, sweat, urine, urethral swabs, cervical swabs, urogenital or anal swabs, conjunctival swabs, ocular lens fluid, cerebral spinal fluid, *etc.* A test sample can be used (i) directly as obtained from the source; or (ii) following a pre-treatment to modify the character of the sample. Thus, a test sample can be pre-treated prior to use by, for example, preparing plasma or serum from blood, disrupting cells or viral particles, preparing liquids from solid materials, diluting viscous fluids, filtering liquids, adding reagents, purifying nucleic acids, *etc.*

**s) Treat, Treating or Treatment**

The terms "treat", "treating" or "treatment" as used herein refer to administering one or more active agents or compounds to a subject in an effort to (i) prevent a pathologic condition from occurring (e.g. prophylaxis); (ii) inhibit the pathologic condition or arrest its development; (iii) relieve a pathologic condition and/or prevent or reduce the severity one or more symptoms associated with such a pathologic condition, regardless of whether any of items (i) through (iii) are successful in a subject.

**t) Variant Polynucleotide or Variant Nucleic Acid Sequence**

A "variant polynucleotide" or a "variant nucleic acid sequence" means a polynucleotide having at least about 60% nucleic acid sequence identity, more preferably at least about 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% nucleic acid sequence identity and yet more preferably at least about 99% nucleic acid sequence identity with a given nucleic acid sequence. Variants do not encompass the native nucleotide sequence.

Ordinarily, variant polynucleotides are at least about 8 nucleotides in length, often at least about 9, 10, 11, 12, 13, 14, 15, 16, 17, 18, 19, 20, 21, 22, 23, 24, 25, 26, 27, 28, 29 30, 35, 40, 45, 50, 55, 60 nucleotides in length, or even about 75-200 nucleotides in length, or more.

The realm of nucleotides includes derivatives wherein the nucleic acid molecule has been covalently modified by substitution, chemical, enzymatic, or other appropriate means with a moiety other than a naturally occurring nucleotide.

**B. Polynucleotide Assays**

Nucleic acid assay methods useful in the invention comprise detection of the presence or absence of copy number gains by: (i) *in situ* hybridization assays to intact tissue or cellular samples, (ii) microarray hybridization assays to chromosomal DNA extracted from a tissue sample, and (iii) polymerase chain reaction (PCR) or other amplification assays to chromosomal DNA extracted from a tissue sample. Assays using synthetic analogs of nucleic acids, such as peptide nucleic acids, in any of these formats can also be used.

The assays of the invention are used to identify copy number gains for the ABCB1 gene at chromosome locus 7q21.1 for use in both predicting therapy response and for monitoring patient response to Aurora kinase B inhibitor therapy. Assays for response prediction can be run before start of therapy, and patients that do not show or exhibit showing a copy number gain for the ABCB1 gene at chromosome locus 7q21.1 are eligible to receive Aurora kinase B inhibitor therapy. The copy number gain for the ABCB1 gene at chromosome locus 7q21.1 can also indicate resistance to other cancer therapy, such as chemotherapy or radiation therapy. For monitoring patient response, the assay can be run at the initiation of therapy to establish baseline levels of the biomarker in the tissue sample, for example, the percent of total cells or number of cells showing the copy number gain in the sample. The same tissue is then sampled and assayed and the levels of the biomarker compared to the baseline. Where the levels remain the same or decrease, the therapy is likely being effective and can be continued. Where significant increase over baseline level occurs, the patient may not be responding or may have developed resistance to continued Aurora kinase B inhibitor therapy.

The assays of the invention can be used with targeted cancer therapy, such as targeted therapies to solid tumors (e.g., sarcomas or carcinomas) or hematological malignancies (e.g., cancers that affect blood, bone marrow, and lymph nodes). The assays of the present invention can be used with solid tumors such as colorectal carcinoma, pancreatic carcinoma, thyroid cancer, prostate cancer, bladder cancer, liver cancer, bile duct cancer, oral cancer, non-small-cell lung carcinoma, small-cell lung carcinoma, ovarian cancer or breast cancer. The assays of the present invention can be used with hematological malignancies such as acute lymphocytic leukemia (ALL), acute myeloid leukemia (AML), chronic myelogenous leukemia (CML), diffuse large B-cell lymphoma (DLBCL), Philadelphia chromosome-positive acute lymphoblastic leukemia (Ph+ALL) and chronic lymphocytic leukemia (CLL). The assays can be performed in relation to any cancer type in which amplification or over-expression of Aurora kinase B are involved. The inventive assays are performed on any type of test sample, such as a patient tissue sample of any type or on a derivative thereof, including peripheral blood, tumor or suspected tumor tissues (including fresh frozen and fixed paraffin-embedded tissue), cell isolates such as circulating epithelial cells separated or identified in a blood sample, lymph node tissue, bone marrow and fine-needle aspirates.

The present invention comprises detection of the genomic biomarkers by hybridization assays using detectably labeled nucleic acid-based probes, such as deoxyribonucleic acid (DNA) probes or protein nucleic acid (PNA) probes, or unlabeled primers which are designed/selected to hybridize to a specific chromosomal target. The unlabeled primers are used in amplification assays, such as by polymerase chain reaction (PCR), in which after primer binding, a polymerase amplifies the target nucleic acid sequence for subsequent detection. The detection probes used in PCR or other amplification assays are preferably fluorescent, and still more preferably, detection probes useful in "real-time PCR". Fluorescent labels are also preferred for use *in situ* hybridization but other detectable labels commonly used in hybridization techniques, *e.g.*, enzymatic, chromogenic and isotopic labels, can also be used. Useful probe labeling techniques are described in the literature (Fan, Y.-S. 2002. Molecular cytogenetics: protocols and applications. Humana Press, Totowa, N.J. xiv, p. 411.

In detection of the genomic biomarkers by microarray analysis, these probe labeling techniques are applied to label a chromosomal DNA extracted from a patient sample, which is then hybridized to the microarray.

In the following disclosure, methods of the invention relate to the ABCB1 gene at chromosome locus 7q21.1. Disclosed methods relating to the ABCB4 gene are not part of the present invention.

The polynucleotide sequence for the human ABCB1 gene (SEQ ID NO:1; GenBank Accession No. NM_000927) is shown in Table 1.

TABLE 1

Polynucleotide sequence of human ABCB1 (SEQ ID NO:1; Genbank Accession No. NM 000927)

| | | | | | | |
|---|---|---|---|---|---|---|
| tattcagata | ttctccagat | tcctaaagat | tagagatcat | ttctcattct | cctaggagta | 60 |
| ctcacttcag | gaagcaacca | gataaaagag | aggtgcaacg | gaagccagaa | cattcctcct | 120 |
| ggaaattcaa | cctgtttcgc | agtttctcga | ggaatcagca | ttcagtcaat | ccgggccggg | 180 |
| agcagtcatc | tgtggtgagg | ctgattggct | gggcaggaac | agcgccgggg | cgtgggctga | 240 |
| gcacagccgc | ttcgctctct | ttgccacagg | aagcctgagc | tcattcgagt | agcggctctt | 300 |
| ccaagctcaa | agaagcagag | gccgctgttc | gtttccttta | ggtctttcca | ctaaagtcgg | 360 |
| agtatcttct | tccaaaattt | cacgtcttgg | tggccgttcc | aaggagcgcg | aggtcggaat | 420 |
| ggatcttgaa | ggggaccgca | atggaggagc | aaagaagaag | aacttttta | aactgaacaa | 480 |
| taaaagtgaa | aaagataaga | aggaaaagaa | accaactgtc | agtgtatttt | caatgtttcg | 540 |
| ctattcaaat | tggcttgaca | agttgtatat | ggtggtggga | actttggctg | ccatcatcca | 600 |
| tggggctgga | cttcctctca | tgatgctggt | gtttggagaa | atgacagata | tctttgcaaa | 660 |
| tgcaggaaat | ttagaagatc | tgatgtcaaa | catcactaat | agaagtgata | tcaatgatac | 720 |
| agggttcttc | atgaatctgg | aggaagacat | gaccaggtat | gcctattatt | acagtggaat | 780 |
| tggtgctggg | gtgctggttg | ctgcttacat | tcaggtttca | ttttggtgcc | tggcagctgg | 840 |
| aagacaaata | cacaaaatta | gaaaacagtt | ttttcatgct | ataatgcgac | aggagatagg | 900 |
| ctggtttgat | gtgcacgatg | ttggggagct | taacacccga | cttacagatg | atgtctccaa | 960 |
| gattaatgaa | ggaattggtg | acaaaattgg | aatgttcttt | cagtcaatgg | caacattttt | 1020 |
| cactgggttt | atagtaggat | ttacacgtgg | ttggaagcta | acccttgtga | ttttggccat | 1080 |
| cagtcctgtt | cttggactgt | cagctgctgt | ctgggcaaag | atactatctt | catttactga | 1140 |
| taaagaactc | ttagcgtatg | caaaagctgg | agcagtagct | gaagaggtct | tggcagcaat | 1200 |
| tagaactgtg | attgcatttg | gaggacaaaa | gaaagaactt | gaaaggtaca | acaaaaattt | 1260 |
| agaagaagct | aaaagaattg | ggataaagaa | agctattaca | gccaatattt | ctataggtgc | 1320 |
| tgctttcctg | ctgatctatg | catcttatgc | tctggccttc | tggtatggga | ccaccttggt | 1380 |
| cctctcaggg | gaatattcta | ttggacaagt | actcactgta | ttcttttctg | tattaattgg | 1440 |
| ggcttttagt | gttggacagg | catctccaag | cattgaagca | tttgcaaatg | caagaggagc | 1500 |
| agcttatgaa | atcttcaaga | taattgataa | taagccaagt | attgacagct | attcgaagag | 1560 |
| tgggcacaaa | ccagataata | ttaagggaaa | tttggaattc | agaaatgttc | acttcagtta | 1620 |
| cccatctcga | aaagaagtta | agatcttgaa | gggtctgaac | ctgaaggtgc | agagtgggca | 1680 |
| gacggtggcc | ctggttggaa | acagtggctg | tgggaagagc | acaacagtcc | agctgatgca | 1740 |
| gaggctctat | gaccccacag | aggggatggt | cagtgttgat | ggacaggata | ttaggaccat | 1800 |
| aaatgtaagg | tttctacggg | aaatcattgg | tgtggtgagt | caggaacctg | tattgtttgc | 1860 |
| caccacgata | gctgaaaaca | ttcgctatgg | ccgtgaaaat | gtcaccatgg | atgagattga | 1920 |
| gaaagctgtc | aaggaagcca | atgcctatga | ctttatcatg | aaactgcctc | ataaatttga | 1980 |
| caccctggtt | ggagagagag | gggcccagtt | gagtggtggg | cagaagcaga | ggatcgccat | 2040 |
| tgcacgtgcc | ctggttcgca | accccaagat | cctcctgctg | gatgaggcca | cgtcagcctt | 2100 |
| ggacacagaa | agcgaagcag | tggttcaggt | ggctctggat | aaggccagaa | aaggtcggac | 2160 |
| caccattgtg | atagctcatc | gtttgtctac | agttcgtaat | gctgacgtca | tcgctggttt | 2220 |
| cgatgatgga | gtcattgtgg | agaaaggaaa | tcatgatgaa | ctcatgaaag | agaaaggcat | 2280 |
| ttacttcaaa | cttgtcacaa | tgcagacagc | aggaaatgaa | gttgaattag | aaaatgcagc | 2340 |
| tgatgaatcc | aaaagtgaaa | ttgatgcctt | ggaaatgtct | tcaaatgatt | caagatccag | 2400 |
| tctaataaga | aaaagatcaa | ctcgtaggag | tgtccgtgga | tcacaagccc | aagacagaaa | 2460 |
| gcttagtacc | aaagaggctc | tggatgaaag | tatacctcca | gtttcctttt | ggaggattat | 2520 |
| gaagctaaat | ttaactgaat | ggccttattt | tgttgttggt | gtattttgtg | ccattataaa | 2580 |
| tggaggcctg | caaccagcat | ttgcaataat | attttcaaag | attatagggg | tttttacaag | 2640 |

(continued)

TABLE 1

Polynucleotide sequence of human ABCB1 (SEQ ID NO:1; Genbank Accession No. NM 000927)

| | | | | | | |
|---|---|---|---|---|---|---|
| aattgatgat | cctgaaacaa | aacgacagaa | tagtaacttg | ttttcactat | tgtttctagc | 2700 |
| ccttggaatt | atttctttta | ttacattttt | ccttcagggt | ttcacatttg | gcaaagctgg | 2760 |
| agagatcctc | accaagcggc | tccgatacat | ggttttccga | tccatgctca | gacaggatgt | 2820 |
| gagttggttt | gatgacccta | aaaacaccac | tggagcattg | actaccaggc | tcgccaatga | 2880 |
| tgctgctcaa | gttaaagggg | ctataggttc | caggcttgct | gtaattaccc | agaatatagc | 2940 |
| aaatcttggg | acaggaataa | ttatatcctt | catctatggt | tggcaactaa | cactgttact | 3000 |
| cttagcaatt | gtacccatca | ttgcaatagc | aggagttgtt | gaaatgaaaa | tgttgtctgg | 3060 |
| acaagcactg | aaagataaga | aagaactaga | aggttctggg | aagatcgcta | ctgaagcaat | 3120 |
| agaaaacttc | cgaaccgttg | tttctttgac | tcaggagcag | aagtttgaac | atatgtatgc | 3180 |
| tcagagtttg | caggtaccat | acagaaactc | tttgaggaaa | gcacacatct | ttggaattac | 3240 |
| attttccttc | acccaggcaa | tgatgtattt | ttcctatgct | ggatgtttcc | ggtttggagc | 3300 |
| ctacttggtg | gcacataaac | tcatgagctt | tgaggatgtt | ctgttagtat | tttcagctgt | 3360 |
| tgtctttggt | gccatggccg | tggggcaagt | cagttcattt | gctcctgact | atgccaaagc | 3420 |
| caaaatatca | gcagcccaca | tcatcatgat | cattgaaaaa | acccctttga | ttgacagcta | 3480 |
| cagcacggaa | ggcctaatgc | cgaacacatt | ggaaggaaat | gtcacatttg | gtgaagttgt | 3540 |
| attcaactat | cccacccgac | cggacatccc | agtgcttcag | ggactgagcc | tggaggtgaa | 3600 |
| gaagggccag | acgctggctc | tggtgggcag | cagtggctgt | gggaagagca | cagtggtcca | 3660 |
| gctcctggag | cggttctacg | acccottggc | agggaaagtg | ctgcttgatg | gcaaagaaat | 3720 |
| aaagcgactg | aatgttcagt | ggctccgagc | acacctgggc | atcgtgtccc | aggagcccat | 3780 |
| cctgtttgac | tgcagcattg | ctgagaacat | tgcctatgga | gacaacagcc | gggtggtgtc | 3840 |
| acaggaagag | attgtgaggg | cagcaaagga | ggccaacata | catgccttca | tcgagtcact | 3900 |
| gcctaataaa | tatagcacta | aagtaggaga | caaaggaact | cagctctctg | gtggccagaa | 3960 |
| acaacgcatt | gccatagctc | gtgcccttgt | tagacagcct | catattttgc | ttttggatga | 4020 |
| agccacgtca | gctctggata | cagaaagtga | aaaggttgtc | caagaagccc | tggacaaagc | 4080 |
| cagagaaggc | cgcacctgca | ttgtgattgc | tcaccgcctg | tccaccatcc | agaatgcaga | 4140 |
| cttaatagtg | gtgtttcaga | atggcagagt | caaggagcat | ggcacgcatc | agcagctgct | 4200 |
| ggcacagaaa | ggcatctatt | tttcaatggt | cagtgtccag | gctggaacaa | agcgccagtg | 4260 |
| aactctgact | gtatgagatg | ttaaatactt | tttaatattt | gtttagatat | gacatttatt | 4320 |
| caaagttaaa | agcaaacact | tacagaatta | tgaagaggta | tctgtttaac | atttcctcag | 4380 |
| tcaagttcag | agtcttcaga | gacttcgtaa | ttaaaggaac | agagtgagag | acatcatcaa | 4440 |
| gtggagagaa | atcatagttt | aaactgcatt | ataaatttta | taacagaatt | aaagtagatt | 4500 |
| ttaaaagata | aaatgtgtaa | ttttgtttat | attttcccat | ttggactgta | actgactgcc | 4560 |
| ttgctaaaag | attatagaag | tagcaaaaag | tattgaaatg | tttgcataaa | gtgtctataa | 4620 |
| taaaactaaa | ctttcatgtg | actggagtca | tcttgtccaa | actgcctgtg | aatatatctt | 4680 |
| ctctcaattg | gaatattgta | gataacttct | gctttaaaaa | agttttcttt | aaatatacct | 4740 |
| actcattttt | gtgggaatgg | ttaagcagtt | taaataattc | ctgttgtata | tgtctattca | 4800 |
| cattgggtct | tacagaacca | tctggcttca | ttcttcttgg | acttgatcct | gctgattctt | 4860 |
| gcatttccac | at | | | | | 4872 |

The polynucleotide sequence for the human ABCB4 gene (SEQ ID NO:2; GenBank Accession No. NM_018849) is shown in Table 1.

TABLE 2

Polynucleotide sequence of human ABCB4 (SEQ ID NO:2; Genbank Accession No NM 018849)

| | | | | | | |
|---|---|---|---|---|---|---|
| caaagtccag | gcccctctgc | tgcagcgccc | gcgcgtccag | aggccctgcc | agacacgcgc | 60 |
| gaggttcgag | gctgagatgg | atcttgaggc | ggcaaagaac | ggaacagcct | ggcgccccac | 120 |
| gagcgcggag | ggcgactttg | aactgggcat | cagcagcaaa | caaaaaagga | aaaaaacgaa | 180 |
| gacagtgaaa | atgattggag | tattaacatt | gtttcgatac | tccgattggc | aggataaatt | 240 |
| gtttatgtcg | ctgggtacca | tcatggccat | agctcacgga | tcaggtctcc | ccctcatgat | 300 |
| gatagtattt | ggagagatga | ctgacaaatt | tgttgatact | gcaggaaact | tctcctttcc | 360 |
| agtgaacttt | tccttgtcgc | tgctaaatcc | aggcaaaatt | ctggaagaag | aaatgactag | 420 |
| atatgcatat | tactactcag | gattgggtgc | tggagttctt | gttgctgcct | atatacaagt | 480 |
| ttcattttgg | actttggcag | ctggtcgaca | gatcaggaaa | attaggcaga | agttttttca | 540 |

(continued)

TABLE 2

Polynucleotide sequence of human ABCB4 (SEQ ID NO:2; Genbank Accession No NM 018849)

tgctattcta cgacaggaaa taggatggtt tgacatcaac gacaccactg aactcaatac 600
gcggctaaca gatgacatct ccaaaatcag tgaaggaatt ggtgacaagg ttggaatgtt 660
ctttcaagca gtagccacgt tttttgcagg attcatagtg ggattcatca gaggatggaa 720
gctcaccctt gtgataatgg ccatcagccc tattctagga ctctctgcag ccgtttgggc 780
aaagatactc tcggcattta gtgacaaaga actagctgct tatgcaaaag caggcgccgt 840
ggcagaagag gctctggggg ccatcaggac tgtgatagct ttcgggggcc agaacaaaga 900
gctggaaagg tatcagaaac atttagaaaa tgccaaagag attggaatta aaaaagctat 960
ttcagcaaac atttccatgg gtattgcctt cctgttaata tatgcatcat atgcactggc 1020
cttctggtat ggatccactc tagtcatatc aaaagaatat actattggaa atgcaatgac 1080
agtttttttt tcaatcctaa ttggagcttt cagtgttggc caggctgccc catgtattga 1140
tgcttttgcc aatgcaagag gagcagcata tgtgatcttt gatattattg ataataatcc 1200
taaaattgac agtttttcag agagaggaca caaaccagac agcatcaaag ggaatttgga 1260
gttcaatgat gttcactttt cttacccttc tcgagctaac gtcaagatct tgaagggcct 1320
caacctgaag gtgcagagtg ggcagacggt ggccctggtt ggaagtagtg gctgtgggaa 1380
gagcacaacg gtccagctga tacagaggct ctatgaccct gatgagggca caattaacat 1440
tgatgggcag gatattagga actttaatgt aaactatctg agggaaatca ttggtgtggt 1500
gagtcaggag ccggtgctgt tttccaccac aattgctgaa aatatttgtt atggccgtgg 1560
aaatgtaacc atggatgaga taaagaaagc tgtcaaagag gccaacgcct atgagtttat 1620
catgaaatta ccacagaaat ttgacaccct ggttggagag agagggggccc agctgagtgg 1680
tgggcagaag cagaggatcg ccattgcacg tgccctggtt cgcaacccca agatccttct 1740
gctggatgag gccacgtcag cattggacac agaaagtgaa gctgaggtac aggcagctct 1800
ggataaggcc agagaaggcc ggaccaccat tgtgatagca caccgactgt ctacggtccg 1860
aaatgcagat gtcatcgctg ggtttgagga tggagtaatt gtggagcaag gaagccacag 1920
cgaactgatg aagaaggaag ggtgtactt caaacttgtc aacatgcaga catcaggaag 1980
ccagatccag tcagaagaat ttgaactaaa tgatgaaaag gctgccacta gaatggcccc 2040
aaatggctgg aaatctcgcc tatttaggca ttctactcag aaaaacctta aaaattcaca 2100
aatgtgtcag aagagccttg atgtggaaac cgatggactt gaagcaaatg tgccaccagt 2160
gtcctttctg aaggtcctga aactgaataa aacagaatgg ccctactttg tcgtgggaac 2220
agtatgtgcc attgccaatg gggggcttca gccggcattt tcagtcatat tctcagagat 2280
catagcgatt tttggaccag gcgatgatgc agtgaagcag cagaagtgca acatattctc 2340
tttgattttc ttatttctgg gaattatttc ttttttact ttcttccttc agggtttcac 2400
gtttgggaaa gctggcgaga tcctcaccag aagactgcgg tcaatggctt ttaaagcaat 2460
gctaagacag gacatgagct ggtttgatga ccataaaaac agtactggtg cactttctac 2520

aagacttgcc acagatgctg cccaagtcca aggagccaca ggaaccaggt tggctttaat 2580
tgcacagaat atagctaacc ttggaactgg tattatcata tcatttatct acggttggca 2640
gttaacccta ttgctattag cagttgttcc aattattgct gtgtcaggaa ttgttgaaat 2700
gaaattgttg gctggaaatg ccaaaagaga taaaaaagaa ctggaagctg ctggaaagat 2760
tgcaacagag gcaatagaaa atattaggac agttgtgtct ttgacccagg aaagaaaatt 2820
tgaatcaatg tatgttgaaa aattgtatgg accttacagg aattctgtgc agaaggcaca 2880
catctatgga attactttta gtatctcaca agcatttatg tatttttcct atgccggttg 2940
ttttcgattt ggtgcatatc tcattgtgaa tggacatatg cgcttcagag atgttattct 3000
ggtgttttct gcaattgtat ttggtgcagt ggctctagga catgccagtt catttgctcc 3060
agactatgct aaagctaagc tgtctgcagc ccacttattc atgctgtttg aaagacaacc 3120
tctgattgac agctacagtg aagaggggct gaagcctgat aaatttgaag gaaatataac 3180
atttaatgaa gtcgtgttca actatcccac ccgagcaaac gtgccagtgc ttcaggggct 3240
gagcctggag gtgaagaaag gccagacact agccctggtg ggcagcagtg gctgtgggaa 3300
gagcacggtg gtccagctcc tggagcggtt ctacgacccc ttggcgggga cagtgtttgt 3360
ggactttggt tttcagcttc tcgatggtca agaagcaaag aaactcaatg tccagtggct 3420
cagagctcaa ctcggaatcg tgtctcagga gcctatccta tttgactgca gcattgccga 3480
gaatattgcc tatggagaca acagccgggt tgtatcacag gatgaaattg tgagtgcagc 3540
caaagctgcc aacatacatc ctttcatcga gacgttaccc cacaaatatg aaacaagagt 3600
gggagataag gggactcagc tctcaggagg tcaaaaacag aggattgcta ttgcccgagc 3660
cctcatcaga caacctcaaa tcctcctgtt ggatgaagct acatcagctc tggatactga 3720
aagtgaaaag gttgtccaag aagccctgga caaagccaga gaaggccgca cctgcattgt 3780
gattgctcac cgcctgtcca ccatccagaa tgcagactta atagtggtgt ttcagaatgg 3840
gagagtcaag gagcatggca cgcatcagca gctgctggca cagaaaggca tctattttc 3900
aatggtcagt gtccaggctg ggacacagaa cttatgaact tttgctacag tatattttaa 3960

(continued)

| TABLE 2 | |
|---|---|
| Polynucleotide sequence of human ABCB4 (SEQ ID NO:2; Genbank Accession No NM 018849) | |
| aaataaattc aaattattct accatttt | 3988 |

Preferably, *in situ* hybridization is used to detect the presence of chromosomal copy number increase for (i) the ABCB1 gene; (ii) the ABCB4 gene; or (iii) the ABCB1 gene and the ABCB4 gene. Primer and probes can be made by one of skill in the art using the sequences of SEQ ID NO:1 and SEQ ID NO:2.

Probes for use in the *in situ* hybridization methods of the invention fall into two broad groups: chromosome enumeration probes, *i.e.*, probes that hybridize to a chromosomal region, usually a repeat sequence region, and indicate the presence or absence of an entire chromosome; and locus specific probes, *i.e.*, probes that hybridize to a specific locus on a chromosome and detect the presence or absence of a specific locus. Chromosome arm probes, *i.e.*, probes that hybridize to a chromosomal region and indicate the presence or absence of an arm of a specific chromosome, can also be used. It is preferred to use a locus specific probe that can detect changes of the unique chromosomal DNA sequences at the interrogated locus, such as the ABCB1 and ABCB4 loci. Methods for use of unique sequence probes for *in situ* hybridization are described in U.S. Patent No. 5,447,841.

A chromosome enumeration probe can hybridize to a repetitive sequence, located either near or removed from a centromere, or can hybridize to a unique sequence located at any position on a chromosome. For example, a chromosome enumeration probe can hybridize with repetitive DNA associated with the centromere of a chromosome. Centromeres of primate chromosomes contain a complex family of long tandem repeats of DNA comprised of a monomer repeat length of about 171 base pairs, that are referred to as alpha- satellite DNA. Centromere fluorescent *in situ* hybridization probes to each of chromosomes 14 and 18 are commercially available from Abbott Molecular (Des Plaines, IL).

Exceptionally useful *in situ* hybridization probes are directly labeled fluorescent probes, such as described in U.S. Patent No. 5,491,224, U.S. Patent No. 5,491,224 also describes simultaneous FISH assays using more than one fluorescently labeled probe.

Useful locus specific probes can be produced in any manner and generally contain sequences to hybridize to a chromosomal DNA target sequence of about 10,000 to about 1,000,000 bases long. Preferably the probe hybridizes to a target stretch of chromosomal DNA at the target locus of at least 100,000 bases long to about 500,000 bases long and also includes unlabeled blocking nucleic acid in the probe mix, as disclosed in U.S. Patent No. 5,756,696, to avoid non-specific binding of the probe. It is also possible to use unlabeled, synthesized oligomeric nucleic acid or peptide nucleic acid as the blocking nucleic acid. For targeting the particular gene locus, it is preferred that the probes include nucleic acid sequences that span the gene and thus hybridize to both sides of the entire genomic coding locus of the gene. The probes can be produced starting with human DNA-containing clones such as Bacterial Artificial Chromosomes (BAC's) or the like. BAC libraries for the human genome are available from Invitrogen (Carlsbad, CA) and can be investigated for identification of useful clones. It is preferred to use the University of California Santa Cruz Genome Browser to identify DNA sequences in the target locus. These DNA sequences can then be used to synthesize PCR primers for use to screen BAC libraries to identify useful clones. The clones can then be labeled by conventional nick translation methods and tested as *in situ* hybridization probes.

Examples of fluorophores that can be used in the *in situ* hybridization methods described herein are: 7-amino-4-methylcoumarin-3-acetic acid (AMCA); Texas Red™ (Molecular Probes, Inc., Eugene, OR); 5-(and-6)-carboxy-X-rhodamine; lissamine rhodamine B; 5-(and-6)-carboxyfluorescein; fluorescein-5-isothiocyanate (FITC); 7-diethylaminocoumarin-3-carboxylic acid, tetramethyl-rhodamine-5-(and-6)-isothiocyanate; 5-(and-6)-carboxytetramethylrhodamine; 7-hydroxy-coumarin-3-carboxylic acid; 6-[fluorescein 5-(and-6)-carboxamido]hexanoic acid; N-(4,4-difluoro-5,7-dimethyl-4-bora-3a, 4a diaza-3-indacenepropionic acid; eosin-5-isothiocyanate; erythrosine-5-isothiocyanate; 5-(and-6)-carboxyrhodamine 6G; and Cascade™ blue aectylazide (Molecular Probes; an Invitrogen brand).

Probes can be viewed with a fluorescence microscope and an appropriate filter for each fluorophore, or by using dual or triple band-pass filter sets to observe multiple fluorophores. See, for example, U.S. Patent No. 5,776,688,

Any suitable microscopic imaging method can be used to visualize the hybridized probes, including automated digital imaging systems. Alternatively, techniques such as flow cytometry can be used to examine the hybridization pattern of the chromosomal probes.

Although the cell-by-cell gene amplification analysis resulting from *in situ* hybridization is preferred, the genomic biomarkers can also be detected by quantitative PCR. In this embodiment, chromosomal DNA is extracted from the tissue sample, and is then amplified by PCR using a pair of primers specific to at least one of (i) the ABCB1 gene; (ii) the ABCB4 gene; or (iii) the ABCB1 gene and the ABCB4 gene, or by multiplex PCR, using multiple pairs of primers. Any primer sequence for the biomarkers can be used. Examples of primers that can be used are shown in Table 3. The copy number of the tissue is then determined by comparison to a reference amplification standard.

Table 3

| SEQUENCE | Type of Primer | SEQ ID NO: |
|---|---|---|
| 5'-GGAGAGTAGCAGTGCCTTGGACC-3' | Forward | SEQ ID NO:4 |
| 5'-AGGAGGAGGTAGAAAACAGATAAGGGAAC-3' | Reverse | SEQ ID NO:5 |
| 5'-AGTGCCTTGGACCCCAGCTCTC-3' | Forward | SEQ ID NO:6 |
| 5'-GAAAACAGATAAGGGAACAGTTAGGGATC-3' | Reverse | SEQ ID NO:7 |

Microarray-based copy number analysis can also be used. In this embodiment, the chromosomal DNA after extraction is labeled for hybridization to a microarray comprising a substrate having multiple immobilized unlabeled nucleic acid probes arrayed at probe densities up to several million probes per square centimeter of substrate surface. Multiple microarray formats exist and any of these can be used, in the present invention. Examples of microarrays that can be used are the Affymetrix GeneChip® Mapping 100K Set SNP Array (See Matsuzaki, H., et al., "Genotyping over 100,000 SNPs on a pair of oligonucleotide arrays," Nat Methods. 1:109-11 (2004)); the Affymetrix GeneChip® Mapping 250K Assay Kits (such as the GeneChip® Human Mapping 250K Nsp Array or the GeneChip® Human Mapping 250K Sty Array) or the Affymetrix GeneChip® Mapping 500K Array Set, each of which is commercially available from Affymetrix, Inc., Santa Clara, CA), the Agilent Human Genome aCGH Microarray 44B (available from Agilent Technologies, Inc., Santa Clara, CA), Illumina microarrays (Illumina, Inc., San Diego, CA), Nimblegen aCGH microarrays (Nimblegen, Inc., Madison, WI), *etc.* When using an oligonucleotide microarray to detect amplifications, it is preferred to use a microarray that has probe sequences to more than three separate locations in the targeted region. Examples of probes that can be used in the microarray are shown in below Table 4 and in SEQ ID NOS: 23-321. Flanking sequences for the probes listed below in Table 4 are shown below in Table 5.

Table 4

| SEQ ID NO: | Probe Sequence | Direction |
|---|---|---|
| **23** | AGCTTTAGAACCACCACTTCAGGTC | Forward |
| **24** | AGCTTTAGAACCACCATTTCAGGTC | Forward |
| **25** | GACCTGAAGTGGTGGTTCTAAAGCT | Reverse |
| **26** | TAGAACCACCATTTCAGGTCAATGT | Forward |
| **27** | TAGAACCACCACTTCAGGTCAATGT | Forward |
| **28** | GACCTGAAATGGTGGTTCTAAAGCT | Reverse |
| **29** | TTGACCTGAAGTGGTGGTTCTAAAG | Reverse |
| **30** | TTGACCTGAAATGGTGGTTCTAAAG | Reverse |
| **31** | ACATTGACCTGAAATGGTGGTTCTA | Reverse |
| **32** | ACATTGACCTGAAGTGGTGGTTCTA | Reverse |
| **33** | CATTGACCTGAAGTGGTGGTTCTAA | Reverse |
| **34** | CATTGACCTGAAATGGTGGTTCTAA | Reverse |
| **35** | AACCACCACTTCAGGTCAATGTTTT | Forward |
| **36** | AACCACCATTTCAGGTCAATGTTTT | Forward |
| **37** | AAAACATTGACCTGAAATGGTGGTT | Reverse |
| **38** | AAAACATTGACCTGAAGTGGTGGTT | Reverse |
| **39** | AAACATTGACCTGAAATGGTGGTTC | Reverse |
| **40** | AAACATTGACCTGAAGTGGTGGTTC | Reverse |
| **41** | TTTAGAACCACCACTTCAGGTCAAT | Forward |
| **42** | TTTAGAACCACCATTTCAGGTCAAT | Forward |

(continued)

| SEQ ID NO: | Probe Sequence | Direction |
|---|---|---|
| 43 | TGTTAAAGGTTGTGCTATAATGAAT | Forward |
| 44 | TCATTATAGCACAACCTTTAACACA | Reverse |
| 45 | GTGTGTTAAAGATTGTGCTATAATG | Forward |
| 46 | ATAGCACAATCTTTAACACACCACT | Reverse |
| 47 | TCATTATAGCACAATCTTTAACACA | Reverse |
| 48 | TAGCACAACCTTTAACACACCACTT | Reverse |
| 49 | GTGTGTTAAAGGTTGTGCTATAATG | Forward |
| 50 | ATTATAGCACAACGTTTAACACACC | Reverse |
| 51 | ATTATAGCACAATCTTTAACACACC | Reverse |
| 52 | ATTCATTATAGCACAATCTTTAACA | Reverse |
| 53 | ATAGCACAACCTTTAACACACCACT | Reverse |
| 54 | TTATAGCACAACCTTTAACACACCA | Reverse |
| 55 | TTATAGCACAATCTTTAACACACCA | Reverse |
| 56 | TGTTAAAGATTGTGCTATAATGAAT | Forward |
| 57 | TGGTGTGTTAAAGGTTGTGCTATAA | Forward |
| 58 | TGGTGTGTTAAAGATTGTGCTATAA | Forward |
| 59 | AAGTGGTGTGTTAAAGATTGTGCTA | Forward |
| 60 | ATTCATTATAGCACAACCTTTAACA | Reverse |
| 61 | AAGTGGTGTGTTAAAGGTTGTGCTA | Forward |
| 62 | TAGCACAATCTTTAACACACCACTT | Reverse |
| 63 | ACTGAGATAGTGATAGCAATTTTTT | Reverse |
| 64 | AAAAAATTGCTATCACTATCTCAGT | Forward |
| 65 | AAAAAAAATTGCTGTCACTATCTCA | Forward |
| 66 | AAAAAATTGCTGTCACTATCTCAGT | Forward |
| 67 | GCTACTGAGATAGTGATAGCAATTT | Reverse |
| 68 | GCTACTGAGATAGTGACAGCAATTT | Reverse |
| 69 | ATGAAAAAAAATTGCTATCACTATC | Forward |
| 70 | ATGAAAAAAAATTGCTGTCACTATC | Forward |
| 71 | AAATTGCTGTCACTATCTCAGTAGC | Forward |
| 72 | AAATTGCTATCACTATCTCAGTAGC | Forward |
| 73 | AAAAAAAATTGCTATCACTATCTCA | Forward |
| 74 | AGATAGTGACAGCAATTTTTTTTCA | Reverse |
| 75 | AGATAGTGATAGCAATTTTTTTTCA | Reverse |
| 76 | ACTGAGATAGTGACAGCAATTTTTT | Reverse |
| 77 | TACTGAGATAGTGATAGCAATTTTT | Reverse |
| 78 | TACTGAGATAGTGACAGCAATTTTT | Reverse |
| 79 | CTGAGATAGTGATAGCAATTTTTTT | Reverse |
| 80 | CTGAGATAGTGACAGCAATTTTTTT | Reverse |

(continued)

| SEQ ID NO: | Probe Sequence | Direction |
|---|---|---|
| 81 | AAAAATTGCTATCACTATCTCAGTA | Forward |
| 82 | AAAAATTGCTGTCACTATCTCAGTA | Forward |
| 83 | TTATGCTGTAATACATCCATTAAGC | Reverse |
| 84 | TTATGCTGTAATATATCCATTAAGC | Reverse |
| 85 | AGTTATGCTGTAATATATCCATTAA | Reverse |
| 86 | AGTTATGCTGTAATACATCCATTAA | Reverse |
| 87 | TGCTGTAATATATCCATTAAGCTAT | Reverse |
| 88 | TGCTGTAATACATCCATTAAGCTAT | Reverse |
| 89 | ATGCTGTAATATATCCATTAAGCTA | Reverse |
| 90 | ATGCTGTAATACATCCATTAAGCTA | Reverse |
| 91 | AGCTTAATGGATGTATTACAGCATA | Forward |
| 92 | AGCTTAATGGATATATTACAGCATA | Forward |
| 93 | TAGTTATGCTGTAATACATCCATTA | Reverse |
| 94 | TAGTTATGCTGTAATATATCCATTA | Reverse |
| 95 | CTGTAATATATCCATTAAGCTATTT | Reverse |
| 96 | TTAATGGATGTATTACAGCATAACT | Forward |
| 97 | CTGTAATACATCCATTAAGCTATTT | Reverse |
| 98 | TTAATGGATATATTACAGCATAACT | Forward |
| 99 | TATGCTGTAATATATCCATTAAGCT | Reverse |
| 100 | TATGCTGTAATACATCCATTAAGCT | Reverse |
| 101 | ATAGCTTAATGGATATATTACAGCA | Forward |
| 102 | TATAGTAGCTCAAGTCCCTTAGTCT | Reverse |
| 103 | TATAGTAGCTGAAGTCCCTTAGTCT | Reverse |
| 104 | TAGTAGCTCAAGTCCCTTAGTCTCT | Reverse |
| 105 | TAGTAGCTGAAGTCCCTTAGTCTCT | Reverse |
| 106 | CATAGTTATAGTAGCTGAAGTCCCT | Reverse |
| 107 | CATAGTTATAGTAGCTCAAGTCCCT | Reverse |
| 108 | ACTAAGGGACTTCAGCTACTATAAC | Forward |
| 109 | ACTAAGGGACTTGAGCTACTATAAC | Forward |
| 110 | GTTATAGTAGCTCAAGTCCCTTAGT | Reverse |
| 111 | GTTATAGTAGCTGAAGTCCCTTAGT | Reverse |
| 112 | TTATAGTAGCTCAAGTCCCTTAGTC | Reverse |
| 113 | TTATAGTAGCTGAAGTCCCTTAGTC | Reverse |
| 114 | AGACTAAGGGACTTCAGCTACTATA | Forward |
| 115 | AGACTAAGGGACTTGAGCTACTATA | Forward |
| 116 | GACTAAGGGACTTCAGCTACTATAA | Forward |
| 117 | GACTAAGGGACTTGAGCTACTATAA | Forward |
| 118 | AGTTATAGTAGCTGAAGTCCCTTAG | Reverse |

(continued)

| SEQ ID NO: | Probe Sequence | Direction |
|---|---|---|
| **119** | AGTTATAGTAGCTCAAGTCCCTTAG | Reverse |
| **120** | AAGGGACTTCAGCTACTATAACTAT | Forward |
| **121** | AAGGGACTTGAGCTACTATAACTAT | Forward |
| **122** | AATACTTATGAGATTTATAGAGGAA | Forward |
| **123** | CTTATGAGATTTATAGAGGAAGAAG | Forward |
| **124** | CTTATGAGACTTATAGAGGAAGAAG | Forward |
| **125** | ATACTTATGAGACTTATAGAGGAAG | Forward |
| **126** | ATACTTATGAGATTTATAGAGGAAG | Forward |
| **127** | ACTTCTTCCTCTATAAGTCTCATAA | Reverse |
| **128** | ACTTCTTCCTCTATAAATCTCATAA | Reverse |
| **129** | AATACTTATGAGACTTATAGAGGAA | Forward |
| **130** | TACTTATGAGATTTATAGAGGAAGA | Forward |
| **131** | TACTTATGAGACTTATAGAGGAAGA | Forward |
| **132** | TTCCTCTATAAATCTCATAAGTATT | Reverse |
| **133** | TCTTCCTCTATAAGTCTCATAAGTA | Reverse |
| **134** | TCTTCCTCTATAAATCTCATAAGTA | Reverse |
| **135** | CTCTATAAGTCTCATAAGTATTTGC | Reverse |
| **136** | CTCTATAAATCTCATAAGTATTTGC | Reverse |
| **137** | TTATGAGATTTATAGAGGAAGAAGT | Forward |
| **138** | TTATGAGACTTATAGAGGAAGAAGT | Forward |
| **139** | AAATACTTATGAGATTTATAGAGGA | Forward |
| **140** | AAATACTTATGAGACTTATAGAGGA | Forward |
| **141** | TTCCTCTATAAGTCTCATAAGTATT | Reverse |
| **142** | TTAGAGCTGACTAATTAGATCCTAT | Forward |
| **143** | TTAGAGCTGTCTAATTAGATCCTAT | Forward |
| **144** | ATCTAATTAGACAGCTCTAAAACCT | Reverse |
| **145** | ATCTAATTAGTCAGCTCTAAAACCT | Reverse |
| **146** | AGGATCTAATTAGTCAGCTCTAAAA | Reverse |
| **147** | ATAGGATCTAATTAGTCAGCTCTAA | Reverse |
| **148** | ATAGGATCTAATTAGACAGCTCTAA | Reverse |
| **149** | GGTTTTAGAGCTGACTAATTAGATC | Forward |
| **150** | GGTTTTAGAGCTGTCTAATTAGATC | Forward |
| **151** | TAGAGCTGACTAATTAGATCCTATG | Forward |
| **152** | TAGAGCTGTCTAATTAGATCCTATG | Forward |
| **153** | GGATCTAATTAGACAGCTCTAAAAC | Reverse |
| **154** | GGATCTAATTAGTCAGCTCTAAAAC | Reverse |
| **155** | AGGATCTAATTAGACAGCTCTAAAA | Reverse |
| **156** | TTTTAGAGCTGTCTAATTAGATCCT | Forward |

(continued)

| SEQ ID NO: | Probe Sequence | Direction |
|---|---|---|
| 157 | GATCTAATTAGACAGCTCTAAAACC | Reverse |
| 158 | GATCTAATTAGTCAGCTCTAAAACC | Reverse |
| 159 | TTTTAGAGCTGACTAATTAGATCCT | Forward |
| 160 | GAAGGTTTTAGAGCTGACTAATTAG | Forward |
| 161 | GAAGGTTTTAGAGCTGTCTAATTAG | Forward |
| 162 | GCAACAAATACTATATTATATACCA | Reverse |
| 163 | GCAACAAATACCATATTATATACCA | Reverse |
| 164 | GTATATAATATAGTATTTGTTGCTC | Forward |
| 165 | GTATATAATATGGTATTTGTTGCTC | Forward |
| 166 | TATAATATAGTATTTGTTGCTCTAG | Forward |
| 167 | TATAATATGGTATTTGTTGCTCTAG | Forward |
| 168 | AGAGCAACAAATACCATATTATATA | Reverse |
| 169 | AGAGCAACAAATACTATATTATATA | Reverse |
| 170 | TAATGGTATATAATATAGTATTTGT | Forward |
| 171 | TAATGGTATATAATATGGTATTTGT | Forward |
| 172 | CTAGAGCAACAAATACTATATTATA | Reverse |
| 173 | AACAAATACTATATTATATACCATT | Reverse |
| 174 | AACAAATACCATATTATATACCATT | Reverse |
| 175 | CTAGAGCAACAAATACCATATTATA | Reverse |
| 176 | GGTATATAATATGGTATTTGTTGCT | Forward |
| 177 | GGTATATAATATAGTATTTGTTGCT | Forward |
| 178 | AGCAACAAATACCATATTATATACC | Reverse |
| 179 | AGCAACAAATACTATATTATATACC | Reverse |
| 180 | TGGTATATAATATAGTATTTGTTGC | Forward |
| 181 | TGGTATATAATATGGTATTTGTTGC | Forward |
| 182 | TGTGGATTAAACTTGTGCGCTTAAA | Reverse |
| 183 | TGTGGATTAAATTTGTGCGCTTAAA | Reverse |
| 184 | ATTTAAGCGCACAAATTTAATCCAC | Forward |
| 185 | ATTTAAGCGCACAAGTTTAATCCAC | Forward |
| 186 | TTGTGGATTAAATTTGTGCGCTTAA | Reverse |
| 187 | TTGTGGATTAAACTTGTGCGCTTAA | Reverse |
| 188 | TAAGCGCACAAGTTTAATCCACAAC | Forward |
| 189 | TAAGCGCACAAATTTAATCCACAAC | Forward |
| 190 | GTGTTGTGGATTAAACTTGTGCGCT | Reverse |
| 191 | GTGTTGTGGATTAAATTTGTGCGCT | Reverse |
| 192 | GCGCACAAGTTTAATCCACAACACA | Forward |
| 193 | GCGCACAAATTTAATCCACAACACA | Forward |
| 194 | TTATTTAAGCGCACAAGTTTAATCC | Forward |

(continued)

| SEQ ID NO: | Probe Sequence | Direction |
|---|---|---|
| 195 | TTATTTAAGCGCACAAATTTAATCC | Forward |
| 196 | TTTAAGCGCACAAGTTTAATCCACA | Forward |
| 197 | TTTAAGCGCACAAATTTAATCCACA | Forward |
| 198 | GTGGATTAAATTTGTGCGCTTAAAT | Reverse |
| 199 | GTGGATTAAACTTGTGCGCTTAAAT | Reverse |
| 200 | TGTGTTGTGGATTAAATTTGTGCGC | Reverse |
| 201 | TGTGTTGTGGATTAAACTTGTGCGC | Reverse |
| 202 | CACAAATGATAGCAGTAAGATAAAT | Forward |
| 203 | TTTATCTTACTGCTACCATTTGTGT | Reverse |
| 204 | AAAACACAAATGATAGCAGTAAGAT | Forward |
| 205 | AAAACACAAATGGTAGCAGTAAGAT | Forward |
| 206 | AAACACAAATGGTAGCAGTAAGATA | Forward |
| 207 | AAACACAAATGATAGCAGTAAGATA | Forward |
| 208 | ATCTTACTGCTACCATTTGTGTTTT | Reverse |
| 209 | ATCTTACTGCTATCATTTGTGTTTT | Reverse |
| 210 | ATTGAAAACACAAATGGTAGCAGTA | Forward |
| 211 | TTTATCTTACTGCTATCATTTGTGT | Reverse |
| 212 | GAAAACACAAATGGTAGCAGTAAGA | Forward |
| 213 | GAAAACACAAATGATAGCAGTAAGA | Forward |
| 214 | CACAAATGGTAGCAGTAAGATAAAT | Forward |
| 215 | ATTTATCTTACTGCTATCATTTGTG | Reverse |
| 216 | TGAAAACACAAATGGTAGCAGTAAG | Forward |
| 217 | ATTTATCTTACTGCTACCATTTGTG | Reverse |
| 218 | TGAAAACACAAATGATAGCAGTAAG | Forward |
| 219 | CTTACTGCTACCATTTGTGTTTTCA | Reverse |
| 220 | CTTACTGCTATCATTTGTGTTTTCA | Reverse |
| 221 | ATTGAAAACACAAATGATAGCAGTA | Forward |
| 222 | ACCTGCAAATCCGTAAAGTGTACTA | Forward |
| 223 | ACCTGCAAATCTGTAAAGTGTACTA | Forward |
| 224 | AGTACACTTTACAGATTTGCAGGTT | Reverse |
| 225 | AGTACACTTTACGGATTTGCAGGTT | Reverse |
| 226 | ACACTTTACGGATTTGCAGGTTTTG | Reverse |
| 227 | AACCTGCAAATCTGTAAAGTGTACT | Forward |
| 228 | AACCTGCAAATCCGTAAAGTGTACT | Forward |
| 229 | TAGTACACTTTACGGATTTGCAGGT | Reverse |
| 230 | TAGTACACTTTACAGATTTGCAGGT | Reverse |
| 231 | ATATAGTACACTTTACGGATTTGCA | Reverse |
| 232 | ATATAGTACACTTTACAGATTTGCA | Reverse |

(continued)

| SEQ ID NO: | Probe Sequence | Direction |
|---|---|---|
| 233 | ATAGTACACTTTACGGATTTGGAGG | Reverse |
| 234 | AAACCTGCAAATCCGTAAAGTGTAC | Forward |
| 235 | AAACCTGCAAATCTGTAAAGTGTAC | Forward |
| 236 | GCAAAACCTGCAAATCTGTAAAGTG | Forward |
| 237 | GCAAAACCTGCAAATCCGTAAAGTG | Forward |
| 238 | CCTGCAAATCCGTAAAGTGTACTAT | Forward |
| 239 | ACACTTTACAGATTTGCAGGTTTTG | Reverse |
| 240 | CCTGCAAATCTGTAAAGTGTACTAT | Forward |
| 241 | ATAGTACACTTTACAGATTTGCAGG | Reverse |
| 242 | TTCTTTAATGGGTACAAAATGTCAA | Reverse |
| 243 | TTTGACATTATGTACCCATTAAAGA | Forward |
| 244 | TTTGACATTTTGTACCCATTAAAGA | Forward |
| 245 | TAATGGGTACATAATGTCAAAAATA | Reverse |
| 246 | TTGACATTTTGTACCCATTAAAGAA | Forward |
| 247 | TTCTTTAATGGGTACATAATGTCAA | Reverse |
| 248 | TATTTTTGACATTATGTACCCATTA | Forward |
| 249 | ATATTTTTGACATTATGTACCCATT | Forward |
| 250 | TTTAATGGGTACAAAATGTCAAAAA | Reverse |
| 251 | TTTAATGGGTACATAATGTCAAAAA | Reverse |
| 252 | ATATTTTTGACATTTTGTACCCATT | Forward |
| 253 | TTGACATTATGTACCCATTAAAGAA | Forward |
| 254 | TTAATGGGTACATAATGTCAAAAAT | Reverse |
| 255 | TTAATGGGTACAAAATGTCAAAAAT | Reverse |
| 256 | TATTTTTGACATTTTGTACCCATTA | Forward |
| 257 | TGGGTACATAATGTCAAAAATATTT | Reverse |
| 258 | ATTTTTGACATTATGTACCCATTAA | Forward |
| 259 | ATTTTTGACATTTTGTACCCATTAA | Forward |
| 260 | TGGGTACAAAATGTCAAAAATATTT | Reverse |
| 261 | TAATGGGTACAAAATGTCAAAAATA | Reverse |
| 262 | TTCTTTAATGCAGAGTAGGACACAG | Reverse |
| 263 | TCCTACTCTGCGTTAAAGAAGCCTG | Forward |
| 264 | TGTGTCCTACTCTGCATTAAAGAAG | Forward |
| 265 | CAGGCTTCTTTAACGCAGAGTAGGA | Reverse |
| 266 | TACTCTGCATTAAAGAAGCCTGCAT | Forward |
| 267 | CTGTGTCCTACTCTGCATTAAAGAA | Forward |
| 268 | AGGCTTCTTTAATGCAGAGTAGGAC | Reverse |
| 269 | AGGCTTCTTTAACGCAGAGTAGGAC | Reverse |
| 270 | CTGTGTCCTACTCTGCGTTAAAGAA | Forward |

(continued)

| SEQ ID NO: | Probe Sequence | Direction |
|---|---|---|
| 271 | ATGCAGGCTTCTTTAATGCAGAGTA | Reverse |
| 272 | TGTGTCCTACTCTGCGTTAAAGAAG | Forward |
| 273 | GGCTTCTTTAACGCAGAGTAGGACA | Reverse |
| 274 | GCAGGCTTCTTTAACGCAGAGTAGG | Reverse |
| 275 | TCCTACTCTGCATTAAAGAAGCCTG | Forward |
| 276 | TTCTTTAACGCAGAGTAGGACACAG | Reverse |
| 277 | GGCTTCTTTAATGCAGAGTAGGACA | Reverse |
| 278 | TACTCTGCGTTAAAGAAGCCTGCAT | Forward |
| 279 | CAGGCTTCTTTAATGCAGAGTAGGA | Reverse |
| 280 | ATGCAGGCTTCTTTAACGCAGAGTA | Reverse |
| 281 | GCAGGCTTCTTTAATGCAGAGTAGG | Reverse |
| 282 | ATTTTTCCTGCATGGCAAGAGTTTA | Forward |
| 283 | ATTTTTCCTGCATAGCAAGAGTTTA | Forward |
| 284 | CCTAAACTCTTGCCATGCAGGAAAA | Reverse |
| 285 | TCCTAAACTCTTGCTATGCAGGAAA | Reverse |
| 286 | TCCTAAACTCTTGCCATGCAGGAAA | Reverse |
| 287 | TAATTTTTCCTGCATGGCAAGAGTT | Forward |
| 288 | TTTCCTGCATGGCAAGAGTTTAGGA | Forward |
| 289 | TAATTTTTCCTGCATAGCAAGAGTT | Forward |
| 290 | TTTTCCTGCATGGCAAGAGTTTAGG | Forward |
| 291 | TTTCCTGCATAGCAAGAGTTTAGGA | Forward |
| 292 | CCTAAACTCTTGCTATGCAGGAAAA | Reverse |
| 293 | ATAATTTTTCCTGCATGGCAAGAGT | Forward |
| 294 | ATAATTTTTCCTGCATAGCAAGAGT | Forward |
| 295 | TCCTGCATGGCAAGAGTTTAGGAGA | Forward |
| 296 | TCCTGCATAGCAAGAGTTTAGGAGA | Forward |
| 297 | AACTCTTGCCATGCAGGAAAAATTA | Reverse |
| 298 | AACTCTTGCTATGCAGGAAAAATTA | Reverse |
| 299 | TTTTCCTGCATAGCAAGAGTTTAGG | Forward |
| 300 | CTAAACTCTTGCTATGCAGGAAAAA | Reverse |
| 301 | CTAAACTCTTGCCATGCAGGAAAAA | Reverse |
| 302 | TGGAAACATGGTTGGTCCGAATGTT | Forward |
| 303 | TGGAAACATGGTTAGTCCGAATGTT | Forward |
| 304 | GGAAACATGGTTAGTCCGAATGTTA | Forward |
| 305 | GGAAACATGGTTGGTCCGAATGTTA | Forward |
| 306 | AGTGGAAACATGGTTAGTCCGAATG | Forward |
| 307 | AGTGGAAACATGGTIGGTCCGAATG | Forward |
| 308 | ATTAACATTCGGACCAACCATGTTT | Reverse |

(continued)

| SEQ ID NO: | Probe Sequence | Direction |
|---|---|---|
| 309 | ATTAACATTCGGACTAACCATGTTT | Reverse |
| 310 | GAAACATGGTTGGTCCGAATGTTAA | Forward |
| 311 | GAAACATGGTTAGTCCGAATGTTAA | Forward |
| 312 | AACATTCGGACCAACCATGTTTCCA | Reverse |
| 313 | AACATTCGGACTAACCATGTTTCCA | Reverse |
| 314 | ACATGGTTAGTCCGAATGTTAATCT | Forward |
| 315 | ACATGGTTGGTCCGAATGTTAATCT | Forward |
| 316 | TAGTGGAAACATGGTTGGTCCGAAT | Forward |
| 317 | TAGTGGAAACATGGTTAGTCCGAAT | Forward |
| 318 | CATTCGGACCAACCATGTTTCCACT | Reverse |
| 319 | CATTCGGACTAACCATGTTTCCACT | Reverse |
| 320 | TAACATTCGGACCAACCATGTTTCC | Reverse |
| 321 | TAACATTCGGACTAACCATGTTTCC | Reverse |

Table 5

| SEQ ID NO: | FLANKING SEQUENCE | Corresponding Probes (SEQ ID NOS) |
|---|---|---|
| 8 | ccatgttgaaaaacattgacctgaa[A/G]tggtggttctaaagcttcggtgaat | 23-42 |
| 9 | tgccctacaattcattatagcacaa[C/T]ctttaacacaccacttaataactgt | 43-62 |
| 10 | aaccatcaggctactgagatagtga[C/T]agcaattttttttcatacttcttct | 63-82 |
| 11 | ccaaaatattagttatgctgtaata[C/T]atccattaagctatttaagaaaaca | 83-101 |
| 12 | ctttccctacatagttatagtagct[C/G]aagtcccttagtctctccacattcc | 102-121 |
| 13 | agtcctgtaacttcttcctctataa[A/G]tctcataagtatttgctttcttttc | 122-141 |
| 14 | tacaataaacataggatctaattag[A/T]cagctctaaaaccttcttcagtaag | 142-161 |
| 15 | tcttagaaactagagcaacaaatac[C/T]atattatataccattaaatactttt | 162-181 |
| 16 | taaatattatgtgttgtggattaaa[C/T]ttgtgcgcttaaataaatttcagtt | 182-201 |
| 17 | aaaattacaatttatcttactgcta[C/T]catttgtgttttcaatcttcatctt | 202-221 |
| 18 | taaggaaaaatatagtacactttac[A/G]gatttgcaggttttgctatttataa | 222-241 |
| 19 | caagaccctttctttaatgggtaca[A/T]aatgtcaaaaatattttatataat | 242-261 |
| 20 | gacaccttgatgcaggcttctttaa[C/T] gcagagtaggacacagatggctgga | 262-281 |
| 21 | atttcagtatctcctaaactcttgc[C/T]atgcaggaaaaattattttagtga | 282-301 |
| 22 | taagagggaagattaacattcggac[C/T] aaccatgtttccactaaaccaatta | 302-321 |

**C. Detecting Expression: mRNA**

The level of gene expression for (i) the ABCB1 gene; (ii) the ABCB4 gene; or (iii) the ABCB1 gene and the ABCB4 gene can be determined by assessing the amount of one or more mRNAs in the test sample. Methods of measuring mRNA in samples are known in the art. To measure mRNA levels, the cells in a test sample can be lysed, and the levels of mRNA in the lysates or in RNA purified or semi-purified from lysates can be measured by any variety of methods familiar to those in the art. Such methods include hybridization assays using detectably labeled DNA or RNA probes (*i.e.*, Northern blotting) or quantitative or semi-quantitative RT-PCR methodologies using appropriate oligonucleotide primers. Alternatively, quantitative or semi-quantitative *in situ* hybridization assays can be carried out using, for example, tissue sections, or unlysed cell suspensions, and detectably labeled (*e.g.*, fluorescent, or enzyme-labeled) DNA or RNA probes. Additional methods for quantifying mRNA include RNA protection assay (RPA), cDNA and oligonucleotide microarrays, representation difference analysis (RDA), differential display, EST sequence analysis, and serial analysis of gene expression (SAGE).

In suitable embodiments, PCR amplification is used to detect for (i) the ABCB1 gene; (ii) the ABCB4 gene; or (iii) the ABCB1 gene and the ABCB4 gene in the test sample. Briefly, in PCR, two primer sequences are prepared that are complementary to regions on opposite complementary strands of the marker sequence, for example containing the sequences for (i) the ABCB1 gene; (ii) the ABCB4 gene; or (iii) the ABCB1 gene and the ABCB4 gene. An excess of deoxynucleotide triphosphates are added to a reaction mixture along with a DNA polymerase, e.g., Taq polymerase. If the target sequence is present in a sample, the primers will bind to the sequence and the polymerase will cause the primers to be extended along the marker sequence by adding on nucleotides. By raising and lowering the temperature of the reaction mixture, the extended primers will dissociate from the marker to form reaction products, excess primers will bind to the marker and to the reaction products and the process is repeated, thereby generating amplification products. A reverse transcriptase PCR amplification procedure can be performed in order to quantify the amount of mRNA amplified.

Any suitable fragment of (i) the ABCB1 gene; (ii) the ABCB4 gene; or (iii) the ABCB1 gene and the ABCB4 gene can be amplified and detected. Designing efficient primers for PCR is within the ordinary skill in the art. Examples of primers that can be used are shown in Table 3. Typically, amplified fragments for detection are approximately 50 to 300 nucleotides in length.

Amplification products can be detected in several ways. Amplification products can be visualized by electrophoresis of the sample in a gel and then staining with a DNA binding dye, e.g., ethidium bromide. Alternatively, the amplification products can be integrally labeled with a radio- or fluorescence nucleotide and then visualized using x-ray film or under the appropriate stimulating spectra.

Amplification can be also monitored using "real-time" methods. Real-time PCR allows for the detection and quantitation of a nucleic acid target. Typically, this approach to quantitative PCR utilizes a fluorescent dye, which can be a double-strand specific dye, such as SYBR GREEN®. Alternatively, other fluorescent dyes (e.g., FAM or HEX) can be conjugated to an oligonucleotide probe or a primer. Various instruments capable of performing real time PCR are known in the art and include, for example, the ABI PRISM® 7900 (Applied Biosystems) and LIGHTCYCLER® systems (Roche). The fluorescent signal generated at each cycle of PCR is proportional to the amount of PCR product. A plot of fluorescence versus cycle number is used to describe the kinetics of amplification and a fluorescence threshold level is used to define a fractional cycle number related to initial template concentration. When amplification is performed and detected on an instrument capable of reading fluorescence during thermal cycling, the intended PCR product from non-specific PCR products can be differentiated using melting analysis. By measuring the change in fluorescence while gradually increasing the temperature of the reaction subsequent to amplification and signal generation it can be possible to determine the Tm of the intended product(s) as well as that of the nonspecific product.

The methods can include amplifying multiple nucleic acids in sample, also known as "multiplex detection" or "multiplexing." Multiplex PCR" refers to PCR that involves adding more than one set of PCR primers to the reaction in order to detect and quantify multiple nucleic acids, including nucleic acids from one or more target gene markers. Furthermore, multiplexing with an internal control (e.g., 18S rRNA, GADPH, or actin) provides a control for the PCR without reaction.

### D. Sample Processing and Assay Performance

As discussed previously herein, the test sample of the present invention can be a tissue sample. The tissue sample to be assayed by the methods of the present invention can comprise any type, including a peripheral blood sample, a tumor tissue or a suspected tumor tissue, a thin layer cytological sample, a fine-needle aspirate sample, a bone marrow sample, a lymph node sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample, a paraffin embedded tissue sample or an extract or processed sample produced from any of a peripheral blood sample, a tumor tissue or a suspected tumor tissue, a thin layer cytological sample, a fine needle aspirate sample, a bone marrow sample, a lymph node sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample or a paraffin embedded tissue sample. For example, a patient peripheral blood sample can be initially processed to extract an epithelial cell population, and this extract can then be assayed. A microdissection of the tissue sample to obtain a cellular sample enriched with suspected tumor cells can also be used. The preferred tissue samples for use herein are peripheral blood, tumor tissue or suspected tumor tissue, including fine needle aspirates, fresh frozen tissue and paraffin embedded tissue, and bone marrow.

The tissue sample can be processed by any desirable method for performing *in situ* hybridization or other nucleic acid assays. For the preferred *in situ* hybridization assays, a paraffin embedded tumor tissue sample or bone marrow sample is fixed on a glass microscope slide and deparaffinized with a solvent, typically xylene. Useful protocols for tissue deparaffinization and *in situ* hybridization are available from Abbott Molecular Inc. (Des Plaines, Illinois). Any suitable instrumentation or automation can be used in the performance of the inventive assays. PCR based assays can be performed on the m2000 instrument system (Abbott Molecular, Des Plaines, IL). Automated imaging can be used for the preferred fluorescent *in situ* hybridization assays.

In one embodiment, the sample comprises a peripheral blood sample from a patient which is processed to produce an extract of circulating tumor or cancer cells to be examined for the presence or absence of a copy number gain for (i) a ABCB1 gene; (ii) a ABCB4 gene; or (iii) a ABCB1 gene and a ABCB4 gene. The circulating tumor cells can be separated by immunomagnetic separation technology such as that available from Immunicon (Huntingdon Valley, Pennsylvania). The copy number determined for the circulating tumor cells is then compared to the baseline level or predetermined level of circulating tumor cells having a copy number determined at a previous point in time, such as at the start of therapy. Increases in the copy number compared to the baseline level or the predetermined level can indicate therapy failure.

Test samples can comprise any number of cells that is sufficient for a clinical diagnosis, and typically contain at least about 100 cells. In a typical FISH assay, the hybridization pattern is assessed in about 25-1,000 cells. Test samples are typically considered "test positive" when found to contain the gene amplification in a sufficient proportion of the sample. The number of cells identified with chromosomal copy number and used to classify a particular sample as positive, in general, varies with the number of cells in the sample. The number of cells used for a positive classification is also known as the cut-off value. Examples of cut-off values that can be used in the determinations include about 5, 25, 50, 100 and 250 cells, or 5%, 10%, 15%, 20%, 25%, 30%, 35%, 40%, 50% and 60% of cells in the sample population. As low as one cell can be sufficient to classify a sample as positive. In a typical paraffin embedded tissue sample, it is preferred to identify at least 30 cells as positive and more preferred to identify at least 20 cells as positive for having the chromosomal copy number gain. For example, detection in a typical paraffin embedded colorectal carcinoma of 30 cells would be sufficient to classify the tissue as positive and eligible for treatment.

## E. Kits

Disclosed herein are kits for detecting the presence or absence of a copy number gain for (i) the ABCB1 gene; (ii) the ABCB4 gene; or (iii) the ABCB1 gene and the ABCB4 gene in a test sample. Such kits can comprise one or more reagents for determining the presence or absence of the above described copy number gain. For example, said kit can contain one or more nucleic acid probes. Alternatively, or in addition to the probes, the kit can contain one or more nucleic acid primers.

Thus, the present disclosure further provides for diagnostic and quality control kits comprising one or more nucleic acid primers, nucleic acid probes or nucleic acid primers and probes described herein. Optionally the assays, kits and kit components can be optimized for use on commercial platforms (e.g., immunoassays on the Prism®, AxSYM®, ARCHITECT® and EIA (Bead) platforms of Abbott Laboratories, Abbott Park, IL, as well as other commercial and/or in vitro diagnostic assays). Additionally, the assays, kits and kit components can be employed in other formats, for example, on electrochemical or other hand-held or point-of-care assay systems. The present disclosure is, for example, applicable to the commercial Abbott Point of Care (i-STAT®, Abbott Laboratories, Abbott Park, IL) electrochemical immunoassay system that performs sandwich immunoassays for several cardiac markers, including TnI, CKMB and BNP. Immunosensors and methods of operating them in single-use test devices are described, for example, in U.S. Patent Application Publication Nos. 2003/0170881, 2004/0018577, 2005/0054078 and 2006/0160164,

Additional background on the manufacture of electrochemical and other types of immunosensors is found in U.S. Patent No. 5,063,081 for its teachings regarding same.

Optionally the kits include quality control reagents (e.g., sensitivity panels, calibrators, and positive controls). Preparation of quality control reagents is well known in the art, and is described, *e.g.*, on a variety of immunodiagnostic product insert sheets.

The kit can incorporate a detectable label, such as a fluorophore, radioactive moiety, enzyme, biotin/avidin label, chromophore, chemiluminescent label, or the like, or the kit may include reagents for labeling the nucleic acid primers, the nucleic acid probes or the nucleic acid primers and nucleic acid probes for detecting the presence or absence of a copy number gain as described herein. The primers and/or probes, calibrators and/or controls can be provided in separate containers or pre-dispensed into an appropriate assay format, for example, into microtiter plates.

The kits can optionally include other reagents required to conduct a diagnostic assay or facilitate quality control evaluations, such as buffers, salts, enzymes, enzyme co-factors, substrates, detection reagents, and the like. Other components, such as buffers and solutions for the isolation and/or treatment of a test sample (*e.g.*, pretreatment reagents), may also be included in the kit. The kit may additionally include one or more other controls. One or more of the components of the kit may be lyophilized and the kit may further comprise reagents suitable for the reconstitution of the lyophilized components.

The various components of the kit optionally are provided in suitable containers. As indicated above, one or more of the containers may be a microtiter plate. The kit further can include containers for holding or storing a sample (*e.g.*, a container or cartridge for a blood or urine sample). Where appropriate, the kit may also optionally contain reaction vessels, mixing vessels and other components that facilitate the preparation of reagents or the test sample. The kit may also include one or more instruments for assisting with obtaining a test sample, such as a syringe, pipette, forceps, measured spoon, or the like.

The kit further can optionally include instructions for use, which may be provided in paper form or in computer-readable form, such as a disc, CD, DVD or the like.

**F. Adaptation of Kits**

The kit (or components thereof), as well as the method of determining the presence or absence of a copy number gain for (i) the ABCB1 gene; (ii) the ABCB4 gene; or (iii) the ABCB1 I gene and the ABCB4 gene using the components and methods described herein, can be adapted for use in a variety of automated and semi-automated systems (including those wherein the solid phase comprises a microparticle), as described, e.g., in U.S. Patent Nos. 5,089,424 and 5,006,309, and as commercially marketed, *e.g.*, by Abbott Laboratories (Abbott Park, IL) as ARCHITECT®.

Some of the differences between an automated or semi-automated system as compared to a non-automated system (*e.g.*, ELISA) include the substrate to which the first specific binding partner (*e.g.*, capture antibody) is attached (which can impact sandwich formation and analyte reactivity), and the length and timing of the capture, detection and/or any optional wash steps. Whereas a non-automated format such as an ELISA may require a relatively longer incubation time with sample and capture reagent (*e.g.*, about 2 hours) an automated or semi-automated format (*e.g.*, ARCHITECT®, Abbott Laboratories) may have a relatively shorter incubation time (*e.g.*, approximately 18 minutes for ARCHITECT®). Similarly, whereas a non-automated format such as an ELISA may incubate a detection antibody such as the conjugate reagent for a relatively longer incubation time (*e.g.*, about 2 hours), an automated or semi-automated format (*e.g.*, ARCHITECT®) may have a relatively shorter incubation time (*e.g.*, approximately 4 minutes for the ARCHITECT®).

Other platforms available from Abbott Laboratories include, but are not limited to, AxSYM®, IMx® (see, *e.g.*, U.S. Patent No. 5,294,404 ), PRISM®, EIA (bead), and Quantium™ II, as well as other platforms. Additionally, the assays, kits and kit components can be employed in other formats, for example, on electrochemical or other hand-held or point-of-care assay systems. The present disclosure is, for example, applicable to the commercial Abbott Point of Care (i-STAT®, Abbott Laboratories) electrochemical immunoassay system that performs sandwich immunoassays. Immunosensors and their methods of manufacture and operation in single-use test devices are described, for example in, U.S. Patent No. 5,063,081, U.S. Patent. Application Publication Nos. 2003/0170881, 2004/0018577, 2005/0054078, and 2006/0160164.

for their teachings regarding same.

It further goes without saying that the methods and kits as described herein necessarily encompass other reagents and methods for carrying out the assays. For instance, encompassed are various buffers such as are known in the art and/or which can be readily prepared or optimized to be employed.

By way of example and not of limitation, an example of the present disclosure shall now be given.

**EXAMPLE**

*Reagents*

Antibodies were purchased from the indicated suppliers as follows: MDR1/P-glycoprotein (Catalog No. 517310) were from Calbiochem (San Diego, CA), anti-BRCP antibody (Catalog No. sc-58222) was from Santa Cruz Biotechnology, Inc. (Santa Cruz, CA), anti-phospho-histone H3 (Ser10) (Catalog No. 9701), and anti-histone H3 (Catalog No. 9715) were from Cell Signaling Technology (Danvers, MA), phycoerythrin (PE)-conjugated goat anti-mouse IgG (Catalog No. 550589) and PE-conjugated anti-human CD44 (Catalog No. 555479) were from BD Biosciences (Franklin Lakes, NJ), ß-actin was from Sigma Aldrich (St. Louis, MO), Alexa Fluor 680-conjugated goat anti-rabbit IgG (Catalog No. A21109) was from Invitrogen (Carlsbad, CA), and IRDye 800-conjugated donkey anti-mouse (Catalog No. 610-732-124) was from Rockland Immunochemicals, Inc. (Gilbertsville, PA). Paclitaxel was purchased from Sigma Aldrich (St. Louis, MO), PSC-833 was purchased from Wenger Chemtech (Riehen, Switzerland), and Fumetrimorgin C was purchased from Alexis Biochemicals Corporation (San Diego, CA).

The chemical structures of MLN8054 (4-{[9-choloro-7-(2,6-difluorophenyl)-5*H*-pyrimido[5,4-D][2]benzazepin-2-yl]amino}-benzoic acid) (See, Manfriedi, MG, et al., Proc. Natl. Acad. Sci. USA 104:4106-4111 (2007)); MLN8075 inhibits Aurora A), AZD1152 (2-[[3-({4-[(5-{2-[(3-Fluorophenyl)amino]-2-oxoethyl}-1*H*-pyrazol-3-yl)amino]-quinazolin-7-yl}oxy) propyl](ethyl)amino]ethyl Dihydrogen Phosphate), and VX-680/MK-0457 (cyclopropane carboxylic acid {4-[4-(4-methyl-piperazin-1-yl)-6-(5-methyl-2H-pyrazol-3-ylamino)-pyrimidin-2-ylsulphanyl]-phenyl}-amide) have been disclosed and are known to those skilled in the art.

*Cell Culture and Generation of AZD1152 HPQA-Resistant Cell Lines*

SW620, HCT-15 and AsPC1 cell lines were obtained from the American Type Culture Collection (ATCC; Manassas, VA) and propagated according to ATCC recommendations.

Polyclonal SW620$^{ABCB1/3}$ cells were selected by culture in the presence of 1μM AZD1152 HQPA (changing the medium two times weekly) over a 3-month period. After 12 weeks, sensitivity to AZD1152 HQPA was assessed. The doubling time of each parent/drug-resistant pair was not significantly different after drug selection. All cells were maintained at 37°C in 5% $CO_2$.

*Flow Cytometry*

Determination of cell surface expression of BCRP or human CD44 was performed by flow cytometry using a Cytofix/Cytoperm kit (Catalog No. 554714, BD Biosciences (Franklin Lakes, NJ). Samples were run on a BD LSR II flow cytometer and analyzed using BD FACSDiva software (BD Biosciences, Franklin Lakes, NJ).

*Colony Formation Assay*

SW620$^{ABCB1/3}$ and the respective parental cell lines were washed and 500 cells/well were seeded into six-well plates in drug-free medium. Then, 24 hours later, compounds were diluted in DMEM or RPMI, added to the cells, which were cultured at 37°C for 7-10 days. Cells were then fixed and stained with 0.2% crystal violet to visualize and count colonies.

*Microarray Analysis*

Total RNA was isolated, and 5 μg was used for microarray analysis using the standard protocol provided by Affymetrix, Inc. (Santa Clara, CA). Fragmented, labeled cRNA was synthesized using an IVT labeling kit and hybridized to a high-density Affymetrix microarray (Affymetrix human genome U133A version 2.0) at 45°C overnight. The scanned image and intensity files were imported into Rosetta Resolver gene expression analysis software version 6.0 (Rosetta Inpharmatics, Kirkland, WA). Resolver's Affymetrix error model was applied, and replicates were combined. Expression profiles were derived from mRNA from three independent samples for each cell line.

*Immunoblot Analysis*

SW620 and SW620$^{ABCB1/3}$ cells were washed and allowed to grow in drug-free medium overnight. To monitor phosphorylation of histone H3, cells were treated for 90 minutes with AZD 1152 HQPA, then extracted immediately in cell extraction buffer (Catalog No. FNN0011) from Biosource (Camarillo, CA) supplemented with phosphatase inhibitor cocktails 1 and 2 and protease inhibitor cocktail (Sigma Aldrich (St. Louis, MO)). The lysates were then probe-sonicated for 10 seconds then clarified by centrifugation at 15,000g for 15 minutes at 4°C. After treatment with SDS-sample buffer, protein extracts were resolved on NuPAGE Bis-Tris 4-12% gels (Invitrogen (Carlsbad, CA)). Samples were electrotransferred to PVDF membranes (Invitrogen (Carlsbad, CA)), incubated with primary antibody overnight, and developed using Pierce Dura-Signal chemiluminescence reagents (Pierce, Rockford, IL), or Odyssey infrared imaging system from LI-COR Biosciences (Lincoln, NE).

*Measurement of Intracellular and Extracellular Drug Concentrations*

SW620 and SW620$^{ABCB1/3}$ cells were washed and allowed to grow in drug-free medium overnight. The cells were then treated with 1 μM AZD1152 HQPA for 4 hours. Cytosolic drug accumulation was determined by LC-MS analysis. Briefly, cells were rinsed once with PBS and extracted in cell lysis buffer. The medium, PBS wash, and cell lysate were treated with 2 volumes of acidified MeOH. Crude whole-cell lysates were then clarified by centrifugation at 15000g for 15 minutes at 4°C yielding an insoluble cell pellet and cytosol. The insoluble cell pellet was diluted 1:10 with 50% acetonitrile and centrifuged at 11,000g for 5 minutes. The concentration of AZD 1152 HQPA in each fraction was determined relative to a standard curve generated from using pure compound.

*siRNA-Mediated Silencing of ABCB1 and ABCB4*

Deconvoluted ON-TARGETplus SMARTpool of four individual siRNAs (ABCB1, Catalog No. LQ-003868, ABCB4; Catalog No. LQ-007302-00) and a Luciferase siRNA negative control (5'-AACGUACGCGGAAUACUUCGA-3' (SEQ ID NO:3) were purchased from Dharmacon, Inc. (Lafayette, CO). SW620 and SW620$^{ABCB1/3}$ cells were washed and seeded at 30,000 cells per well in a 24-well plate and allowed to adhere overnight. The following day, cells were transfected with siRNA oligos at a final concentration of 25 nM per oligo using Lipofectamine2000 (Invitrogen, Carlsbad, CA) according to the manufacturer's instructions. Cells were harvested 48 hour post-transfection.

*RNA isolation and RT-PCR for DNA sequence analysis*

Total cellular RNA was isolated from cell lines using the RNeasy Mini Kit (Qiagen, Valencia, CA), and quantified by UV absorbance spectroscopy. The reverse transcription polymerase chain reaction (RT-PCR) was performed using (OneStep RT-PCR kit) from Qiagen. Aurora B primers (forward primer: 5'-GGAGAGTAGCAGTGCCTTGGACC-3' (SEQ ID NO:4), and reverse primer: 5'-AGGAGGAGGTAGAAAACAGATAAGGGAAC-3' (SEQ ID NO:5)) were used for PCR amplification. The Aurora B nested primers (forward primer: 5'-AGTGCCTTGGACCCCAGCTCTC-3' (SEQ ID NO:6), and reverse primer: 5'-GAAAACAGATAAGGGAACAGTTAGGGATC-3' (SEQ ID NO:7)) were used for direct sequencing using BigDye Terminator v3.1 cycle sequencing kit (Applied Biosystems, Foster City, CA). DNA sequencing was carried out by the DNA sequencing laboratory at Abbott Laboratories. Briefly, direct sequencing of the RT-PCR product was carried out using Applied Biosystems Big Dye™ Terminator version 3.1 cycle sequencing reagents following the manufacturer's recommended protocol (P/N 4337035). Sequencing primers were nested internally to the 5 prime and 3' prime amplification primers by ten and eleven bases respectively. Electrophoresis of the fluorescent labeled sequencing products was carried out on a 3130x1 Genetic Analyzer using a 50 cm array and POP-7™ polymer. Base calling was performed via Sequence Analysis version 5.2 with the KB basecaller.

*Comparative genomic hybridization*

Genomic DNA was isolated using a DNAeasy kit (Qiagen, Valencia, CA) and run on 100K SNP genotyping array sets (Affymetrix, Santa Clara, CA). The arrays were run according to the manufacturer's protocol. The raw microarray data files have been loaded into Gene Expression Omnibus (Accession No. GSE7068) (Gene Expression Omnibus is a gene expression/molecular abundance repository supporting MIAME compliant data submissions, and a curated, online resource for gene expression data browsing, query and retrieval) and Array Express (Accession No. E-MEXP-1008) (ArrayExpress is a public repository for transcriptomics data, which is aimed at storing MIAME- and MINSEQE-compliant data in accordance with MGED recommendations. The ArrayExpress Warehouse stores gene-indexed expression profiles from a curated subset of experiments in the repository). The data were processed using the GTYPE software (Affymetrix, Santa Clara, CA) to create copy number (*.cnt*) files containing information on the inferred copy number for each probe set (SNP). The *.cnt* files contained combined information from both arrays in the set. The files were analyzed using GeneWalker, an internally developed UNIX-based software package (See, Olejniczak et al., Mol. Can. Res.,.5(4):331-339 (2007)).

*In Vivo Studies*

C.B.-17 .*scid*-bg (*scid*-bg) or C.B.-17 *scid* (*scid*) mice were obtained from Charles River Laboratories (Wilmington, MA, USA) at 5-6 weeks of age and used for studies when greater than 8 weeks of age and/or about 20 grams in size. All animal studies were conducted in a specific pathogen-free environment in accordance with the Internal Institutional Animal Care and Use Committee (IACUC), accredited by the American Association of Laboratory Animal Care under conditions that meet or exceed the standards set by the United States Department of Agriculture Animal Welfare Act, Public Health Service policy on humane care and use of animals and the NIH guide on laboratory animal welfare. Overt signs of dehydration, lack of grooming, lethargy, greater than 15% weight loss as well as tumor volume greater then 20% body weight were used to determine tumor end point.

SW620 cell lines were obtained from the ATCC (Manassas, VA) and cultured according to their recommendations without antibiotics and routinely tested for *Mycoplasma* and confirmed to be microbe-free by infectious microbe PCR amplification test (IMPACT; Missouri Research Animal Diagnostic Laboratory, Columbia, MO) prior to *in vivo* inoculation. SW620 cells were grown in Dulbecco's minimal essential medium (DMEM) supplemented with 1mM L-glutamine and 10% fetal bovine serum (FBS), maintained at 37°C in a humidified atmosphere equilibrated with 5% $CO_2$ 95% air and used between passages 3-7 when in log phase for tumor cell inoculation. Cells (1-2 x $10^6$) were mixed 1: 1 with matrigel (BD Biosciences, Franklin Lakes, NJ) and injected subcutaneously (0.2 ml) into the shaved flank of female mice. Tumors were size matched (408-605 $mm^3$) and allocated into treatment groups before dosing was initiated. Two bisecting diameters were measured with calipers and tumor volumes were estimated from the formula: (length x width$^2$) /2. Treatment effect on tumor growth rate was assessed by determining 96T/$C_{day}$ calculated by: [(mean tumor volume of treated group on day X/ mean tumor volume of control vehicle group on day X) x 100]. %TGI was calculated by 100-%T/$C_{day}$ calculated. VX-680 was administered intraperitoneally (i.p., 50 mg/kg/day, twice a day (b.i.d.) to end; 17-21 days depending on when the end point was reached and the study was terminated) in a vehicle containing 10% Solutol (BASF, Florham Park, NJ) and 90% tartaric acid (Sigma Aldrich, St. Louis, MO). AZD1152 was administered intraperitoneally (100 mg/kg/day, b.i.d. x 3, 4 days on, 3 days off, 1-2 cycles) in a vehicle containing 2% ethanol, 5% Tween 20, 20% PEG-400 and 73% HPMC (Sigma Aldrich, St. Louis, MO).

*Results*

To uncover potential mechanisms that cancer cells may cell-autonomously utilize to subvert the activity of inhibitors of Aurora kinases, attempts were made to generate cell lines that were intrinsically resistant to the active alcohol of AZD1152, AZD1152 HQPA. SW620 colon carcinoma cells were propagated in the presence of 1 μM AZD1152 HQPA (~50-fold the $IC_{50}$) for a period of three months. Less than 0.01% of cells survived this treatment after 5 passages (data not shown). Cells that survived the initial selection phase were either maintained in the presence of 1 μM AZD1152 HQPA for an additional three months or were propagated in the absence of drug for the same period. Next, genome-wide microarray analysis of the parental and drug-resistant cells was carried out to identify gene expression changes that could be correlated with resistance to AZD1152 HQPA. In the drug-resistant SW620 derivative (hereafter referred to as $SW620^{ABCB1/3}$), ABCB1, which encodes MDR1, was the most highly overexpressed gene on the array, and was identified by two distinct probe sets (Fig. 1A, inset). It was also observed that a second gene, ABCB4, which encodes MDR3, was also upregulated in $SW620^{ABCB1/3}$, although not to the extent of ABCB1. Among the gene set that encodes known small molecule transporters, ABCB1 and ABCB4 are the only two that show highly differential expression (Fig. 1A). The apparent co-upregulation of ABCB1 and ABCB4 was curious given that these genes lie juxtaposed within a common genomic locus on the long arm of chromosome 7 (7q21.1). This suggested that the genomic region comprising ABCB1 and ABCB4 may have been amplified during selection in AZD1152 HQPA, resulting in the tandem overexpression of these transporter genes. An increase in DNA copy number was observed for both ABCB1 (5 copies) and ABCB4 (3 copies) in $SW620^{ABCB1/3}$ relative to parental SW620 by comparative genomic hybridization (CGH) analysis (Fig. 1B). In $SW620^{ABCB1/3}$, the copy number alterations for both genes were maintained three months after withdrawal of AZD1152 HQPA from the culture medium (Fig. 1B), indicating that the resistance phenotype involved a sustained genetic event consistent with gene amplification. MDR1 was highly upregulated at the protein level in cells propagated in the presence of AZD 1152 HQPA and persisted even after the selection pressure was removed (Fig. 1C).

The $SW620^{ABCB1/3}$ derivative required approximately 100-fold more AZD1152 HQPA to inhibit phosphorylation of the Aurora B substrate, histone H3, than the parental line (Fig. 2A). As MDR1 is an ATP-dependent xenobiotic transporter, it was rationalized that AZD1152 HQPA may be eliminated by efflux from the intracellular compartment in $SW620^{ABCB1/3}$, thus sparing histone H3 phosphorylation. Significantly less drug was measured in the cytosol of the resistant line compared to parental SW620 cells by LC-MS analysis (Fig. 2B). PSC-833, a small-molecule inhibitor of MDR1 (See, Girdler, F., et al., Chem. Biol., 15:552-562 (2008), Twentyman PR, Eur. J. Cancer, 27:1639-1642 (1991)) and MDR3 (See, Boesch, D., Cancer Res., 51:4226-4233 (1991)), was effective in reversing the resistance of $SW620^{ABCB1/3}$ cells to AZD1152 HQPA (Fig. 2C). Partial knockdown of ABCB1 (~75%) with siRNA partially restored inhibition of histone H3 phosphorylation by AZD1152 HQPA in $SW620^{ABCB1/3}$ (Fig. 2D), suggesting that ABCB1 is required for full resistance to AZD1152 HQPA in this model.

The minimum intratumor concentration of AZD 1152 HQPA necessary for inhibition of Aurora B was estimated by calculating the product of the intrinisic potency of AZD1152 HQPA in SW620 or $SW620^{ABCB1/3}$ (0.02 or 2 μM, respectively) and the fold loss in potency of AZD1152 HQPA when assayed in the presence of 50% (v/v) mouse plasma (the loss in potency is presumably due to plasma protein binding; data not shown). Based on this prediction, a minimum threshold concentration of AZD1152 HQPA of 0.1 or 10 μM must be achieved in SW620 or $SW620^{ABCB1/3}$ xenografts, respectively, to produce inhibition of histone H3 phosphorylation (Fig. 3A, top panel). Tumor pharmacokinetics were assessed after a single IP administration of AZD1152 HQPA over a 24-hour period post-dose. This analysis demonstrated a reduction in the overall tumor AUC in $SW620^{ABCB1/3}$ xenografts compared to the parental cohort. Based on the aforementioned prediction, AZD1152 HQPA concentrations in the $SW620^{ABCB1/3}$ tumors exceeded the minimum threshold concentration for only a brief period (-6 hours), whereas in SW620 tumors, concentrations above threshold were achieved for at least 24 hours (Fig. 3A, bottom panel). Correspondingly, only a transient inhibition of histone H3 phosphorylation was observed in $SW620^{ABCB1/3}$ compared to parental tumors. As polyploidization is a manifestation of inhibiting Aurora B during mitosis, one would predict that AZD1152 must to be present at the minimum threshold concentration long enough to allow proliferating cells in the tumor to attempt a single mitosis or, a period roughly equivalent to one cell cycle (15-20 hours). Sustained inhibition of Aurora B in parental SW620 xenografts is a concomitant of the highly efficacious activity observed in this model, whereas the transient inhibition observed in $SW620^{ABCB1/3}$ tumors yields little or no antitumor effect at either dose (cf. Fig. 3B versus Fig. 3C-D).

Given that, in these models, upregulation of the genes ABCB1 and ABCB4 conferred resistance to the anticancer properties of AZD1152 both *in vitro* and *in vivo,* the next step was to ascertain whether or not the presence of these putative Aurora inhibitor resistance genes was indeed predictive of intrinsic tumor resistance. The internal gene expression data from a panel of human xenografts (data not shown) was queried to identify tumor models that displayed upregulation of ABCB1 (MDR1). Among those models, HCT-15 and AsPC1 were confirmed to express elevated levels of MDR1 at the protein level, respectively (Fig. 4A). Three representative Aurora kinase inhibitors, as well as paclitaxel, a known substrate of MDR1 (See, Smith AJ., J. Biol. Chem., 275:23530-23539 (2000)) were evaluated in colony formation and cell proliferation assays in a cell line panel that incorporated HCT-15 and AsPC1. In general, most cell lines were quite sensitive to AZD1152 HQPA displaying IC50s within the low nanomolar range (4-15 nM; Fig. 4B). In contrast, $SW620^{ABCB1/3}$, HCT-15, and AsPC1 were significantly resistant to this compound (IC50s of 2, 1.4, and 2.2, .63 μM, respectively). Curiously, the pan-Aurora kinase inhibitor, VX-680, exhibited a similar activity profile in the cell line panel, though the degree of resistance for $SW620^{ABCB1/3}$, HCT-15, and AsPC1 was lower. As anticipated, $SW620^{ABCB1/3}$ and HCT-15, but not AsPC1 were relatively insensitive to the natural product, paclitaxel. No apparent loss in potency was observed for the Aurora A-selective compound, MLN8054. Importantly, the SW620 cell lines that were relatively sensitive to AZD1152 HQPA expressed no detectable ABCB4 (MDR3) by immunoblot analysis (Fig. 4A and data not shown). It was confirmed that BRCP was required for resistance to AZD1152 HQPA in HCT-15 and AsPC1, respectively, using PSC-833 and fumitremorgin C (Fig. 4C-D).

Growth of HCT-15 colon carcinoma xenografts was unabated by treatment with either AZD1152 or VX-680 (Fig. 4E), whereas both therapies induced significant tumor growth inhibition in HCT116, an alternative colon carcinoma model (Fig. 4D), as well as in DoHH-2 B-cell lymphoma xenografts (Fig. 4C).

One skilled in the art would readily appreciate that the present disclosure is well adapted to carry out the objects and obtain the ends and advantages mentioned, as well as those inherent therein.

## Claims

1. A method of classifying a patient for eligibility for treatment with an Aurora kinase B inhibitor, the method comprising the steps of:

   a) providing a test sample from a patient;
   b) determining the presence or absence of a copy number gain for the ABCB1 gene at chromosome locus 7q21.1; and
   c) classifying the patient as being eligible for receiving treatment with an Aurora kinase B inhibitor based on the presence or absence of a copy number gain for the ABCB1 gene at chromosome locus 7q21.1, wherein the presence of a copy number gain for the ABCB1 gene at chromosome locus 7q21.1 classifies the patient as ineligible for receiving treatment with an Aurora kinase B inhibitor for the treatment of cancer.

2. A method of monitoring a patient suffering from cancer and being treated with an Aurora kinase B inhibitor, the method comprising the steps of:

   a) providing a test sample from a patient suffering from cancer and currently being treated with at least one Aurora kinase B inhibitor;

b) determining the presence or absence of a copy number gain for the ABCB1 gene at chromosome locus 7q21.1;
c) comparing the copy number of the ABCB1 gene in the test sample against a baseline level or a predetermined level, wherein the baseline level refers to the copy number for the ABCB 1 gene in a sample taken from the patient at the initiation of Aurora kinase B inhibitor therapy and wherein the predetermined level refers to an assay cut-off value associated with the progression of cancer; and
d) determining whether the patient should continue to be treated with the Aurora kinase B inhibitor based on the comparison in step c), wherein if the test sample has a copy number gain for the ABCB 1 gene at chromosome locus 7q21.1 that is less than the baseline level or the predetermined level or if no copy number is detected, treatment of the patient with the Aurora kinase B inhibitor continues, and wherein if the test sample has a copy number gain for the ABCB1 gene at chromosome locus 7q21.1 that is the same as or higher than the baseline level or the predetermined level, treatment of the patient with the Aurora kinase B inhibitor is discontinued or combined with at least a second therapy as a combination therapy.

**3.** A method of classifying a patient having a cancer that is resistant to treatment with an Aurora kinase B inhibitor, the method comprising the steps of:

a) providing a test sample from a patient;
b) determining the presence or absence of a copy number gain for the ABCB1 gene at chromosome locus 7q21.1;
c) comparing the presence or absence of the copy number gain for the ABCB1 gene in the test sample against a baseline level or a predetermined level, wherein the baseline level refers to the copy number for the ABCB1 gene in a sample taken from the patient at the initiation of Aurora kinase B inhibitor therapy and wherein the predetermined level refers to an assay cut-off value associated with resistance to Aurora kinase B inhibitor treatment; and
d) classifying the patient as having a cancer that is resistant to Aurora kinase B inhibitor treatment on (i) the presence of a copy number gain in the ABCB 1 gene at chromosome locus 7q21.1; and (ii) if the copy number gain in the test sample is higher than the baseline level or the predetermined level.

**4.** The method of claims 1, 2 or 3, wherein the Aurora kinase B inhibitor is AZD1152, ZM447439, VX-680/MK0457 or Hersperadin.

**5.** The method of claims 1, 2 or 3, wherein the test sample comprises a tissue sample.

**6.** The method of claim 5, wherein the tissue sample comprises a peripheral blood sample, a tumor tissue or a suspected tumor tissue, a thin layer cytological sample, a fine needle aspirate sample, a bone marrow sample, a lymph node sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample, a paraffin embedded tissue sample or an extract or processed sample produced from any of a peripheral blood sample, a tumor tissue or a suspected tumor tissue, a thin layer cytological sample, a fine needle aspirate sample, a bone marrow sample, a urine sample, an ascites sample, a lavage sample, an esophageal brushing sample, a bladder or lung wash sample, a spinal fluid sample, a brain fluid sample, a ductal aspirate sample, a nipple discharge sample, a pleural effusion sample, a fresh frozen tissue sample or a paraffin embedded tissue sample.

**7.** The method of claims 1, 2 or 3, wherein the determining step (b) is performed by *in situ* hybridization.

**8.** The method of claim 7, wherein *the in situ* hybridization is performed with (a) a nucleic acid probe that is fluorescently labeled; (b) at least two nucleic acid probes; or (c) a peptide nucleic acid probe.

**9.** The method of claims 1, 2 or 3, wherein the determining step (b) is performed by polymerase chain reaction or a nucleic acid microarray assay.

**10.** The method of claims 1, 2 or 3, wherein the cancer is colorectal carcinoma or pancreatic carcinoma.

**11.** The method of claims 1 , 2 or 3 , wherein the presence of a copy number gain in the ABCB1 gene correlates with an increase in expression of the MDR1 polypeptide.

**12.** The method of claims 1, 2 or 3, wherein the patient is also being treated with chemotherapy, radiation or combinations thereof.

**Patentansprüche**

1. Ein Verfahren zur Einstufung eines Patienten nach Eignung zur Behandlung mit einem Aurora-Kinase B-Inhibitor, wobei das Verfahren folgende Schritte umfasst:

   a) Bereitstellung einer Untersuchungsprobe von einem Patienten,
   b) Bestimmung der Anwesenheit oder Abwesenheit einer Zunahme der Kopienzahl für das ABCB1-Gen am Chromosomenlokus 7q21.1, und
   c) Einstufung des Patienten als geeignet, eine Behandlung mit einem Aurora-Kinase B-Inhibitor zu erhalten, basierend auf der Anwesenheit oder Abwesenheit einer Zunahme der Kopienzahl für das ABCB1-Gen am Chromosomenlokus 7q21.1, worin die Anwesenheit einer Zunahme der Kopienzahl für das ABCB1-Gen am Chromosomenlokus 7q21.1 den Patienten als nicht geeignet, eine Behandlung mit einem Aurora-Kinase B-Inhibitor zur Behandlung von Krebs zu erhalten, einstuft.

2. Ein Verfahren zur Überwachung eines Patienten, der an Krebs leidet und mit einem Aurora-Kinase B-Inhibitor behandelt wird, wobei das Verfahren folgende Schritte umfasst:

   a) Bereitstellung einer Untersuchungsprobe von einem Patienten, der an Krebs leidet und zurzeit mit mindestens einem Aurora-Kinase B-Inhibitor behandelt wird,
   b) Bestimmung der Anwesenheit oder Abwesenheit einer Zunahme der Kopienzahl für das ABCB1-Gen am Chromosomenlokus 7q21.1,
   c) Vergleichen der Kopienzahl des ABCB1-Gens in der Untersuchungsprobe mit einem Basiswert oder einem vordefinierten Wert, worin der Basiswert sich auf die Kopienzahl für das ABCB1-Gen in einer von dem Patienten zu Beginn der Aurora-Kinase B-Inhibitor-Therapie genommenen Probe bezieht und worin sich der vordefinierte Wert auf einen Test-Anhaltewert im Zusammenhang mit dem Fortschreiten von Krebs bezieht, und
   d) Bestimmung, ob der Patient weiterhin mit dem Aurora-Kinase B-Inhibitor behandelt werden sollte, basierend auf dem Vergleich in Schritt c), worin, wenn die Untersuchungsprobe eine Zunahme der Kopienzahl für das ABCB1-Gen am Chromosomenlokus 7q21.1 ergibt, die geringer ist als der Basiswert oder der vordefinierte Wert, oder wenn keine Kopienzahl bestimmt wird, die Behandlung des Patienten mit dem Aurora-Kinase B-Inhibitor fortgesetzt wird, und worin, wenn die Untersuchungsprobe eine Zunahme der Kopienzahl für das ABCB1-Gen am Chromosomenlokus 7q21.1 ergibt, die identisch mit dem oder höher als der Basiswert oder der vordefinierte Wert ist, die Behandlung des Patienten mit dem Aurora-Kinase B-Inhibitor nicht fortgesetzt oder mit mindestens einer zweiten Therapie als Kombinationstherapie kombiniert wird.

3. Ein Verfahren zur Einstufung eines Patienten mit einem Krebs, der resistent gegenüber Behandlung mit einem Aurora-Kinase B-Inhibitor ist, wobei das Verfahren folgende Schritte umfasst:

   a) Bereitstellung einer Untersuchungsprobe von einem Patienten,
   b) Bestimmung der Anwesenheit oder Abwesenheit einer Zunahme der Kopienzahl für das ABCB1-Gen am Chromosomenlokus 7q21.1,
   c) Vergleich der Anwesenheit oder Abwesenheit der Zunahme der Kopienzahl für das ABCB1-Gen in der Untersuchungsprobe mit einem Basiswert oder einem vordefinierten Wert, worin der Basiswert sich auf die Kopienzahl für das ABCB1-Gen in einer von dem Patienten zu Beginn der Aurora-Kinase B-Inhibitor-Therapie genommenen Probe bezieht und worin sich der vordefinierte Wert auf einen Test-Anhaltewert im Zusammenhang mit Resistenz gegenüber Aurora-Kinase B-Inhibitor-Behandlung bezieht, und
   d) Einstufung des Patienten als einen Krebs habend, der resistent gegenüber Aurora-Kinase B-Inhibitor-Behandlung ist, (i) bei dem Vorhandensein einer Zunahme der Kopienzahl für das ABCB1-Gen am Chromosomenlokus 7q21.1, und (ii) wenn die Zunahme der Kopienzahl in der Untersuchungsprobe größer ist als der Basiswert oder der vordefinierte Wert.

4. Das Verfahren gemäß Anspruch 1, 2 oder 3, worin der Aurora-Kinase B-Inhibitor AZD1152, ZM447439, VX-680/MK0457 oder Hersperadin ist.

5. Das Verfahren gemäß Anspruch 1, 2 oder 3, worin die Untersuchungsprobe eine Gewebeprobe umfasst.

6. Das Verfahren gemäß Anspruch 5, worin die Gewebeprobe Folgendes umfasst: eine periphere Blutprobe, ein Tumorgewebe oder ein mutmaßliches Tumorgewebe, eine zytologische Dünnschichtprobe, eine Dünnnadel-Aspiratprobe, eine Knochenmark-Probe, eine Lymphknotenprobe, eine Urinprobe, eine Aszitesprobe, eine Spülungsprobe, eine ösophageale Abstrichprobe, eine Blasen-oder Lungen-Waschprobe, eine Rückenmarksflüssigkeitsprobe, eine Gehirnflüssigkeitsprobe, eine Gang-Aspiratprobe, eine Brustwarzen-Absonderungsprobe, eine Pleuraergussprobe, eine frisch gefrorene Gewebeprobe, eine in Paraffin eingebettete Gewebeprobe oder ein Extrakt oder eine verarbeitete Probe, produziert aus einem beliebigen von Folgendem: einer peripheren Blutprobe, einem Tumorgewebe oder einem mutmaßlichen Tumorgewebe, einer zytologischen Dünnschichtprobe, einer Dünnnadel-Aspiratprobe, einer Knochenmarksprobe, einer Urinprobe, einer Aszitesprobe, einer Spülungsprobe, einer ösophagealen Abstrichprobe, einer Blasen- oder Lungen-Waschprobe, einer Rückenmark-Flüssigkeitsprobe, einer Gehirnflüssigkeitsprobe, einer Gang-Aspiratprobe, einer Brustwarzen-Absonderungsprobe, einer Pleuraergussprobe, einer frisch gefrorenen Gewebeprobe oder einer in Paraffin eingebetteten Gewebeprobe.

**7.** Das Verfahren gemäß Anspruch 1, 2 oder 3, worin der Bestimmungsschritt (b) durch *In situ*-Hybridisierung durchgeführt wird.

**8.** Das Verfahren gemäß Anspruch 7, worin die *In situ*-Hybridisierung durchgeführt wird mit (a) einer Nukleinsäuresonde, die fluoreszent markiert ist, (b) mindestens zwei Nukleinsäuresonden oder (c) einer Peptid-Nukleinsäuresonde.

**9.** Das Verfahren gemäß Anspruch 1, 2 oder 3, worin der Bestimmungsschritt (b) durch Polymerasekettenreaktion oder einen Nukleinsäure-Mikroarray-Test durchgeführt wird.

**10.** Das Verfahren gemäß Anspruch 1, 2 oder 3, worin der Krebs kolorektaler Krebs oder pankreatischer Krebs ist.

**11.** Das Verfahren gemäß Anspruch 1, 2 oder 3, worin die Anwesenheit einer Zunahme der Kopienzahl für das ABCB1-Gen mit einer Zunahme der Expression des MDR 1-Polypeptids korreliert.

**12.** Das Verfahren gemäß Anspruch 1, 2 oder 3, worin der Patient auch mit Chemotherapie, Strahlung oder Kombinationen davon behandelt wird.

## Revendications

**1.** Procédé de classement d'un patient pour l'éligibilité pour un traitement avec un inhibiteur de kinase Aurora B, le procédé comprenant les étapes de :

a) fourniture d'un échantillon d'essai provenant d'un patient ;
b) détermination de la présence ou de l'absence d'un gain du nombre de copies pour le gène ABCB1 au locus chromosomique 7q21.1 ; et
c) classement du patient comme étant éligible pour recevoir un traitement avec un inhibiteur de kinase Aurora B sur la base de la présence ou de l'absence d'un gain du nombre de copies pour le gène ABCB1 au locus chromosomique 7q21.1, où la présence d'un gain du nombre de copies pour le gène ABCB1 au locus chromosomique 7q21.1 classe le patient comme inéligible pour recevoir un traitement avec un inhibiteur de kinase Aurora B pour le traitement d'un cancer.

**2.** Procédé de suivi d'un patient souffrant d'un cancer et étant traité avec un inhibiteur de kinase Aurora B, le procédé comprenant les étapes de :

a) fourniture d'un échantillon d'essai provenant d'un patient souffrant d'un cancer et étant actuellement traité avec au moins un inhibiteur de kinase Aurora B ;
b) détermination de la présence ou de l'absence d'un gain du nombre de copies pour le gène ABCB1 au locus chromosomique 7q21.1 ;
c) comparaison du nombre de copies du gène ABCB1 dans l'échantillon d'essai avec un niveau initial de base ou un niveau prédéterminé, où le niveau initial de base désigne le nombre de copies pour le gène ABCB1 dans un échantillon prélevé sur le patient au début d'une thérapie avec un inhibiteur de kinase Aurora B et où le niveau prédéterminé désigne une valeur limite de test associée avec l'évolution du cancer ; et
d) détermination de ce que le patient devrait continuer à être traité avec l'inhibiteur de kinase Aurora B sur la base de la comparaison dans l'étape c), où, si l'échantillon d'essai a un gain du nombre de copies pour le gène ABCB1 au locus chromosomique 7q21.1 qui est inférieur au niveau initial de base ou au niveau prédéterminé ou si aucun nombre de copies n'est détecté, le traitement du patient avec l'inhibiteur de kinase Aurora B continue, et où si l'échantillon d'essai a un gain du nombre de copies pour le gène ABCB1 au locus chromosomique 7q21.1 qui est le même que ou supérieur au niveau initial de base ou au niveau prédéterminé, le traitement du patient avec l'inhibiteur de kinase Aurora B est interrompu ou combiné avec au moins une seconde thérapie sous forme d'une thérapie de combinaison.

**3.** Procédé de classement d'un patient ayant un cancer qui est résistant à un traitement avec un inhibiteur de kinase Aurora B, le procédé comprenant les étapes de :

a) fourniture d'un échantillon d'essai provenant d'un patient ;
b) détermination de la présence ou de l'absence d'un gain du nombre de copies pour le gène ABCB1 au locus chromosomique 7q21.1 ;
c) comparaison de la présence ou de l'absence du gain du nombre de copies pour le gène ABCB1 dans l'échantillon d'essai avec un niveau initial de base ou un niveau prédéterminé, où le niveau initial de base désigne le nombre de copies pour le gène ABCB1 dans un échantillon prélevé sur le patient au début d'une thérapie avec un inhibiteur de kinase Aurora B et où le niveau prédéterminé désigne une valeur limite de test associée avec une résistance à un traitement avec un inhibiteur de kinase Aurora B ; et
d) classement du patient comme ayant un cancer qui est résistant à un traitement avec un inhibiteur de kinase Aurora B sur (i) la présence d'un gain du nombre de copies dans le gène ABCB1 au locus chromosomique 7q21.1 ; et (ii) si le gain du nombre de copies dans l'échantillon d'essai est supérieur au niveau initial de base ou au niveau prédéterminé.

**4.** Procédé selon la revendication 1, 2 ou 3, où l'inhibiteur de kinase Aurora B est AZD1152, ZM447439, VX-680/MK0457 ou l'Herspéradine.

**5.** procédé selon la revendication 1, 2 ou 3, où l'échantillon d'essai comprend un échantillon tissulaire.

**6.** Procédé selon la revendication 5, où l'échantillon tissulaire comprend un échantillon de sang périphérique, un tissu tumoral ou un tissu tumoral suspecté, un échantillon cytologique en couche mince, un échantillon de produit d'aspiration avec une aiguille fine, un échantillon de moelle osseuse, un échantillon de ganglion lymphatique, un échantillon d'urine, un échantillon d'ascite, un échantillon de lavage, un échantillon de brossage oesophagien, un échantillon de lavage de la vessie ou du poumon, un échantillon de liquide médullaire, un échantillon de liquide cérébral, un échantillon de produit d'aspiration canalaire, un échantillon d'écoulement mammaire, un échantillon d'épanchement pleural, un échantillon tissulaire congelé frais, un échantillon tissulaire inclus dans la paraffine ou un extrait ou échantillon traité produit à partir de l'un quelconque d'un échantillon de sang périphérique, d'un échantillon tumoral ou d'un échantillon tumoral suspecté, d'un échantillon cytologique en couche mince, d'un échantillon de produit d'aspiration avec une aiguille fine, d'un échantillon de moelle osseuse, d'un échantillon d'urine, d'un échantillon d'ascite, d'un échantillon de lavage, d'un échantillon de brossage oesophagien, d'un échantillon de lavage de la vessie ou du poumon, d'un échantillon de liquide médullaire, d'un échantillon de liquide cérébral, d'un échantillon de produit d'aspiration canalaire, d'un échantillon d'écoulement mammaire, d'un échantillon d'épanchement pleural, d'un échantillon tissulaire congelé frais ou d'un échantillon tissulaire inclus dans la paraffine.

**7.** Procédé selon la revendication 1, 2 ou 3, où l'étape de détermination (b) est accomplie par hybridation *in situ.*

**8.** Procédé selon la revendication 7, où l'hybridation *in situ* est accomplie avec (a) une sonde d'acide nucléique qui est marquée par fluorescence ; (b) au moins deux sondes d'acide nucléique ; ou (c) une sonde d'acide nucléique peptidique.

**9.** Procédé selon la revendication 1, 2 ou 3, où l'étape de détermination (b) est accomplie par une réaction en chaîne par polymérase ou un test avec une micromatrice d'acide nucléique.

**10.** Procédé selon la revendication 1, 2 ou 3, où le cancer est le cancer colorectal ou le cancer du pancréas.

**11.** Procédé selon la revendication 1, 2 ou 3, où la présence d'un gain du nombre de copies dans le gène ABCB1 est corrélée avec une augmentation de l'expression du polypeptide MDR1.

**12.** Procédé selon la revendication 1, 2 ou 3, où le patient est traité aussi avec une chimiothérapie, un rayonnement ou des combinaisons de ceux-ci.

# Fig. 1A

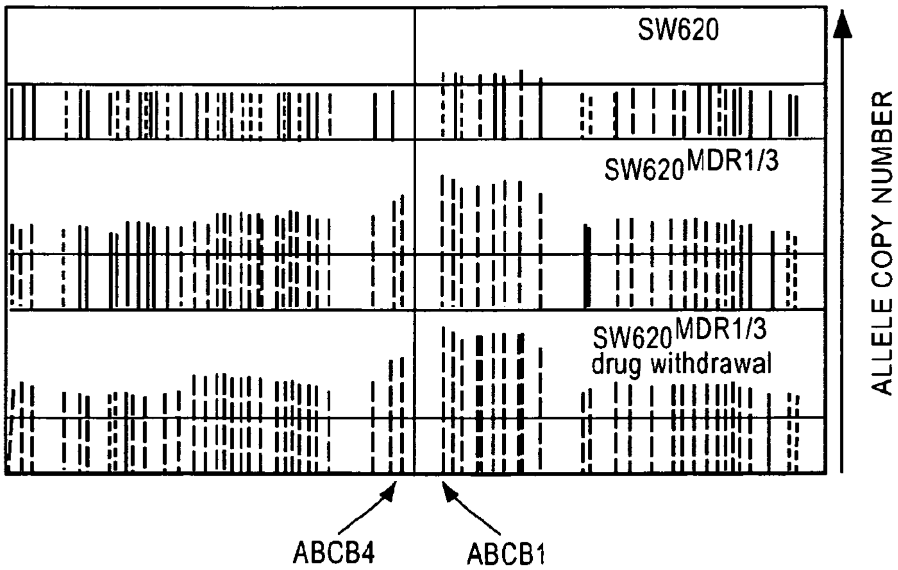
SW620
SW620MDR1/3
SW620MDR1/3
drug withdrawal
ALLELE COPY NUMBER
ABCB4
ABCB1

Fig. 1B

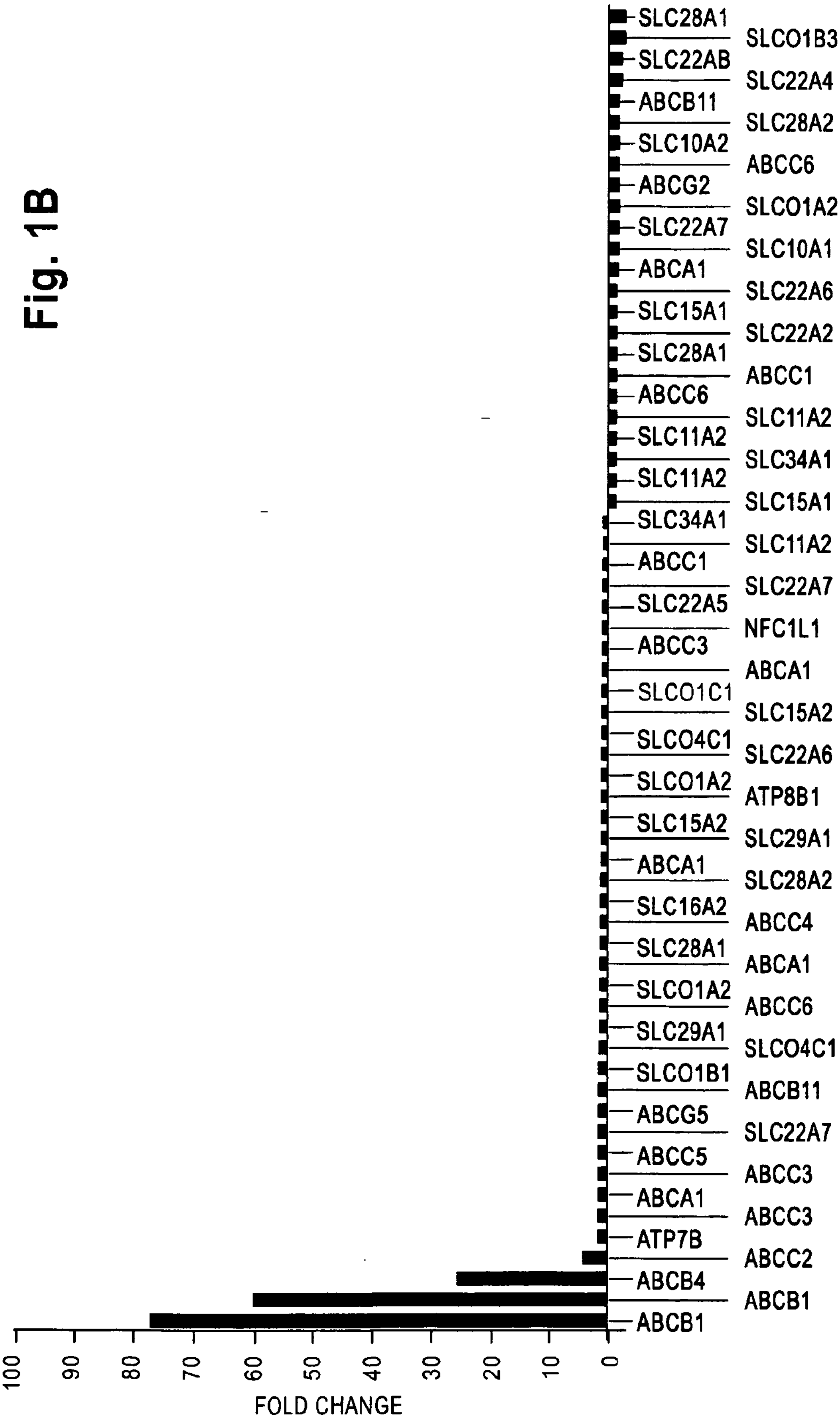
SLC28A1
SLCO1B3
SLC22AB
SLC22A4
ABCB11
SLC28A2
SLC10A2
ABCC6
ABCG2
SLCO1A2
SLC22A7
SLC10A1
ABCA1
SLC22A6
SLC15A1
SLC22A2
SLC28A1
ABCC1
ABCC6
SLC11A2
SLC11A2
SLC34A1
SLC11A2
SLC15A1
SLC34A1
SLC11A2
ABCC1
SLC22A7
SLC22A5
NFC1L1
ABCC3
ABCA1
SLCO1C1
SLC15A2
SLCO4C1
SLC22A6
SLCO1A2
ATP8B1
SLC15A2
SLC29A1
ABCA1
SLC28A2
SLC16A2
ABCC4
SLC28A1
ABCA1
SLCO1A2
ABCC6
SLC29A1
SLCO4C1
SLCO1B1
ABCB11
ABCG5
SLC22A7
ABCC5
ABCC3
ABCA1
ABCC3
ATP7B
ABCC2
ABCB4
ABCB1
ABCB1
100
90
80
70
60
50
40
30
20
10
0
FOLD CHANGE

Fig. 1C

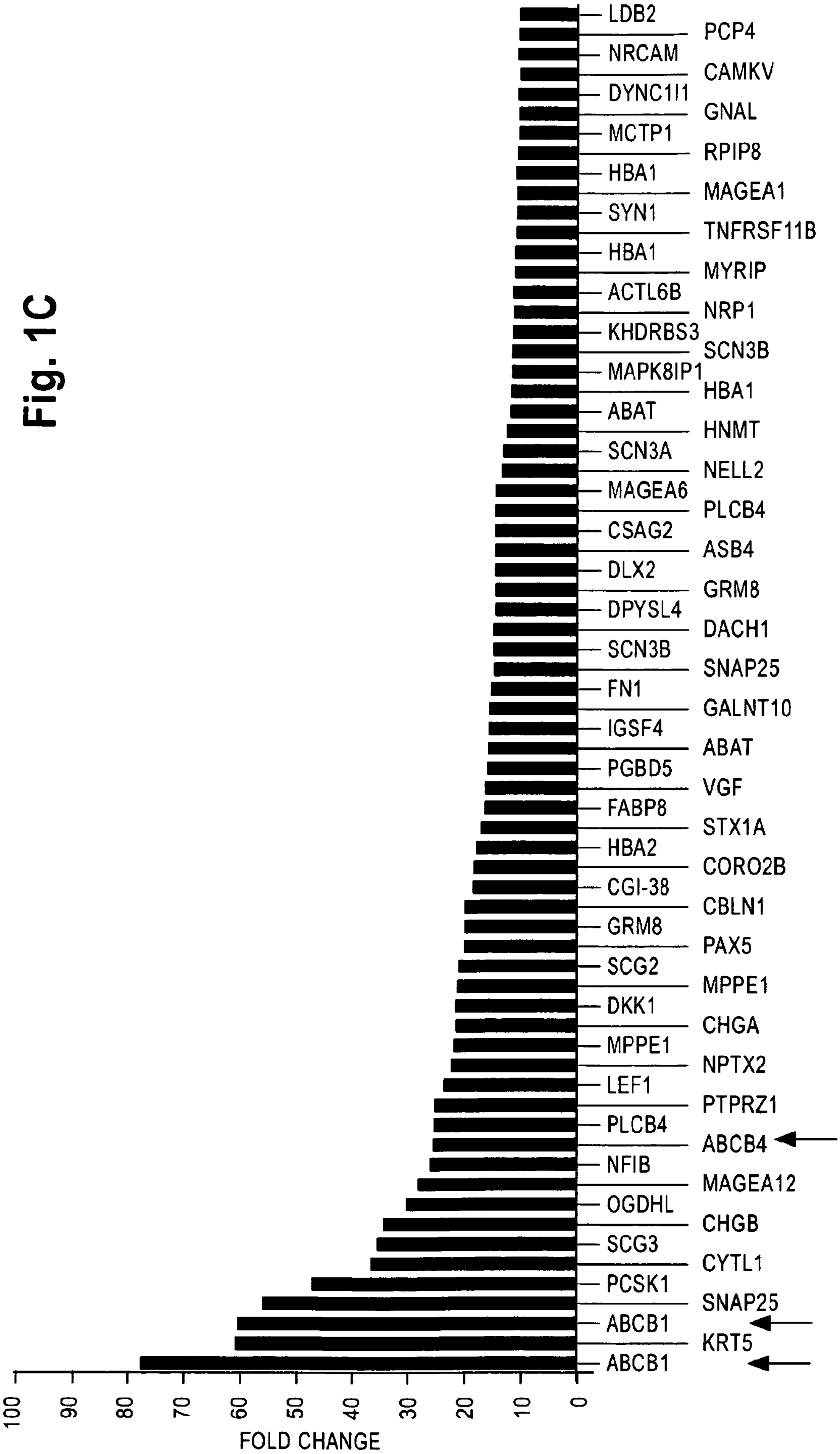
LDB2
PCP4
NRCAM
CAMKV
DYNC1I1
GNAL
MCTP1
RPIP8
HBA1
MAGEA1
SYN1
TNFRSF11B
HBA1
MYRIP
ACTL6B
NRP1
KHDRBS3
SCN3B
MAPK8IP1
HBA1
ABAT
HNMT
SCN3A
NELL2
MAGEA6
PLCB4
CSAG2
ASB4
DLX2
GRM8
DPYSL4
DACH1
SCN3B
SNAP25
FN1
GALNT10
IGSF4
ABAT
PGBD5
VGF
FABP8
STX1A
HBA2
CORO2B
CGI-38
CBLN1
GRM8
PAX5
SCG2
MPPE1
DKK1
CHGA
MPPE1
NPTX2
LEF1
PTPRZ1
PLCB4
ABCB4
NFIB
MAGEA12
OGDHL
CHGB
SCG3
CYTL1
PCSK1
SNAP25
ABCB1
KRT5
ABCB1
100
90
80
70
60
50
40
30
20
10
0
FOLD CHANGE

Fig. 1D

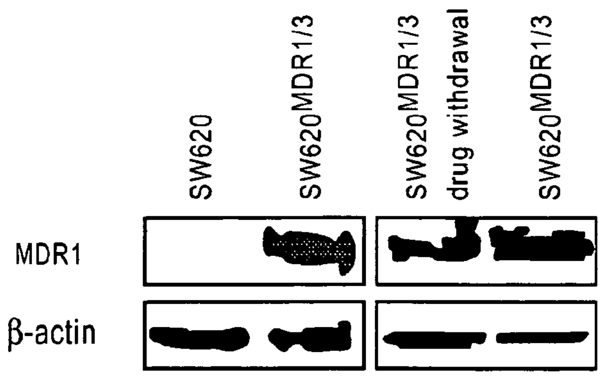
SW620
SW620MDR1/3
SW620MDR1/3
drug withdrawal
SW620MDR1/3
MDR1
β-actin

# Fig. 2A

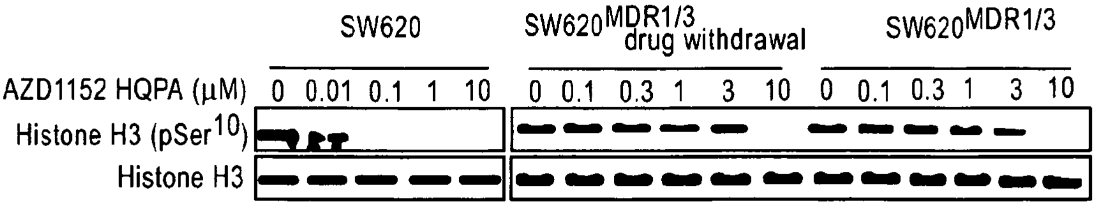


# Fig. 2B

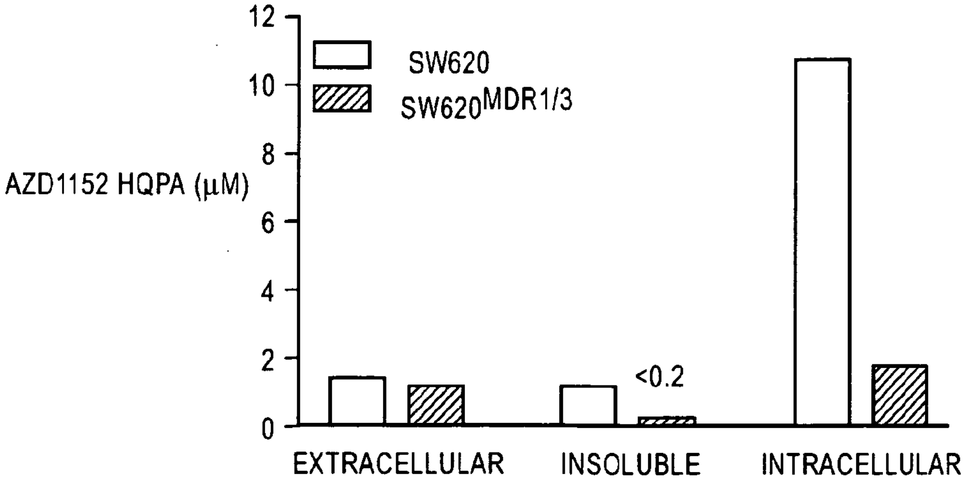


# Fig. 2C

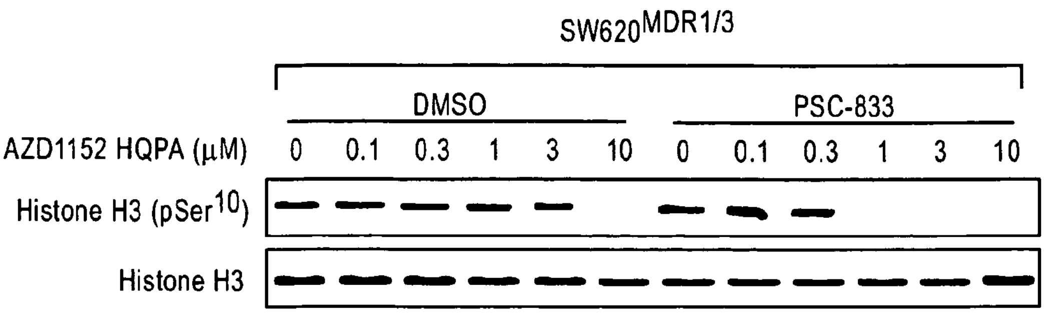

Fig. 2D

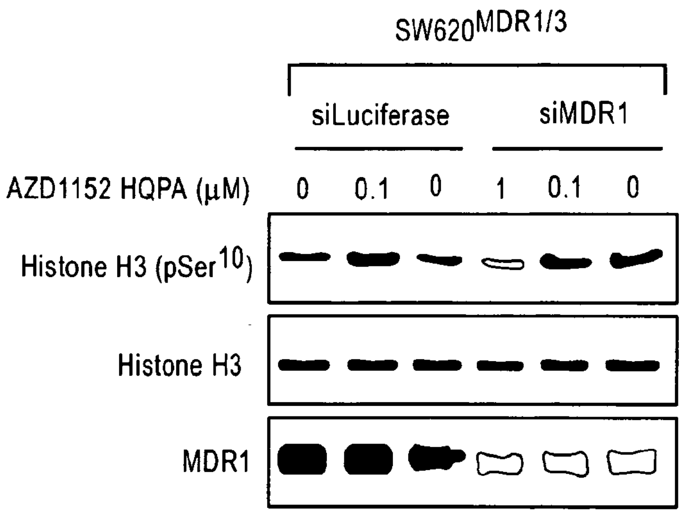
SW620MDR1/3
siLuciferase
siMDR1
AZD1152 HQPA (μM)
0 0.1 0 1 0.1 0
Histone H3 (pSer10)
Histone H3
MDR1

# Fig. 3A

| | histone H3-(pSer[10]) (IC50, μM) x | $IC_{50}$ fold-shift (mouse plasma) = | projected threshold $[AZD1152]_{Tumor}$, μM |
|---|---|---|---|
| SW620 | 0.02 | 5 | 0.1 |
| $SW620^{MDR1/3}$ | 2 | 5 | 10 |

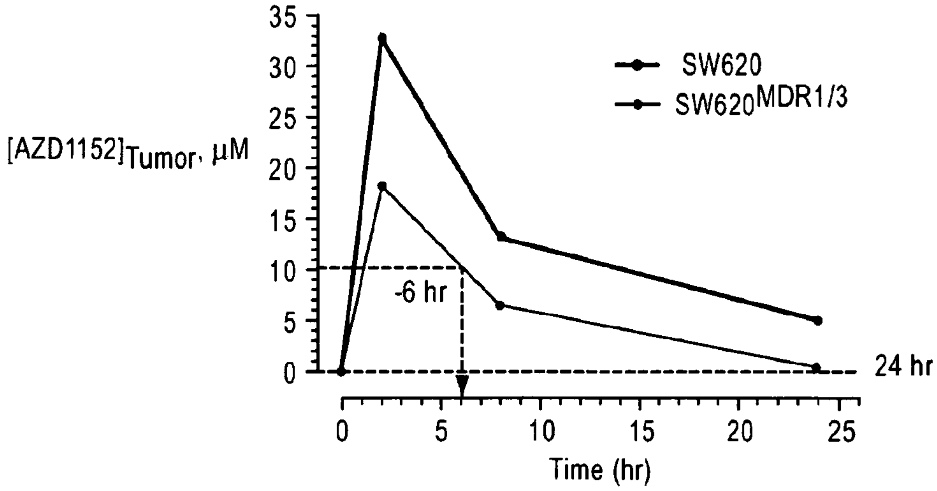


# Fig. 3B

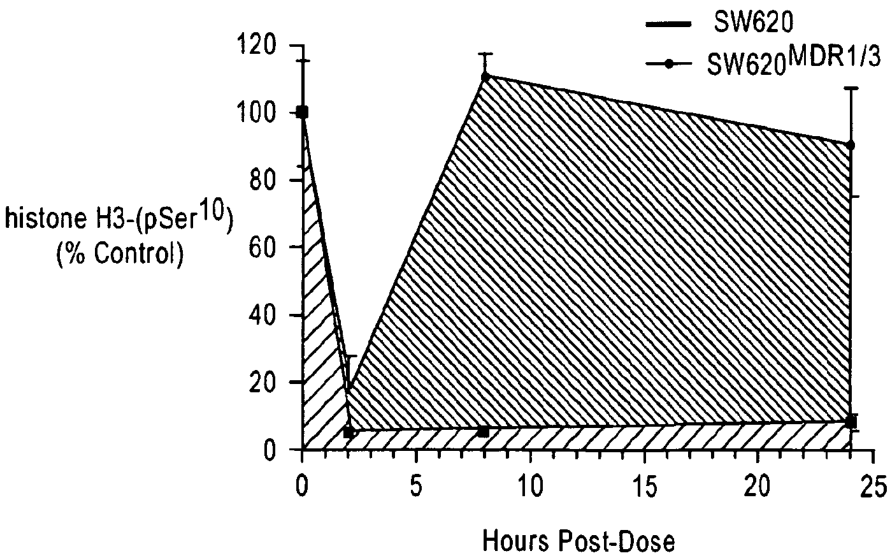

# Fig. 3C

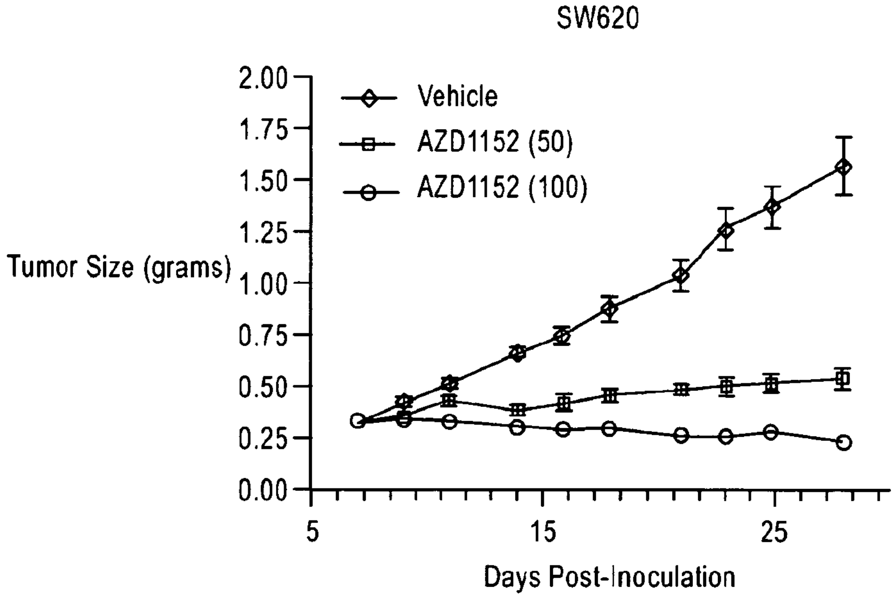
SW620
Vehicle
AZD1152 (50)
AZD1152 (100)
Tumor Size (grams)
2.00
1.75
1.50
1.25
1.00
0.75
0.50
0.25
0.00
5
15
25
Days Post-Inoculation

# Fig. 3D

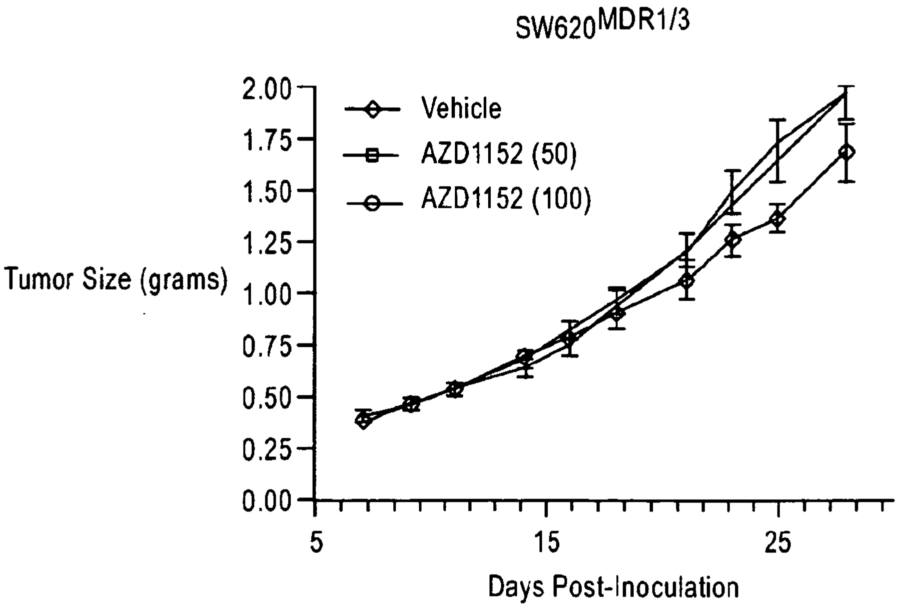
SW620MDR1/3
Vehicle
AZD1152 (50)
AZD1152 (100)
Tumor Size (grams)
2.00
1.75
1.50
1.25
1.00
0.75
0.50
0.25
0.00
5
15
25
Days Post-Inoculation

Fig. 4A

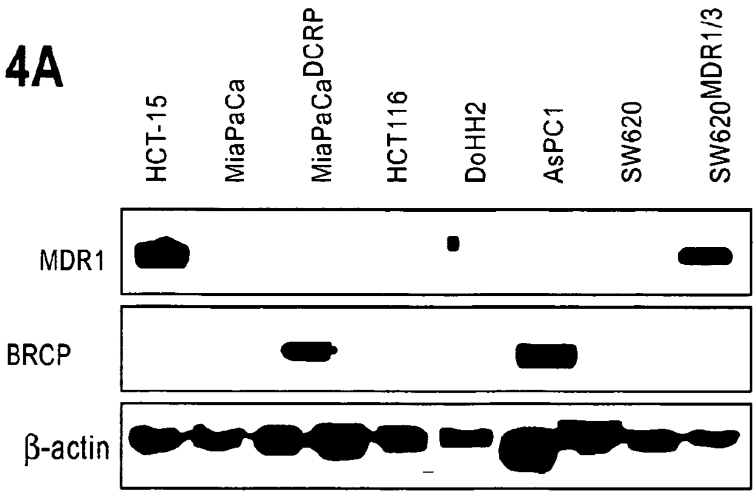


Fig. 4B

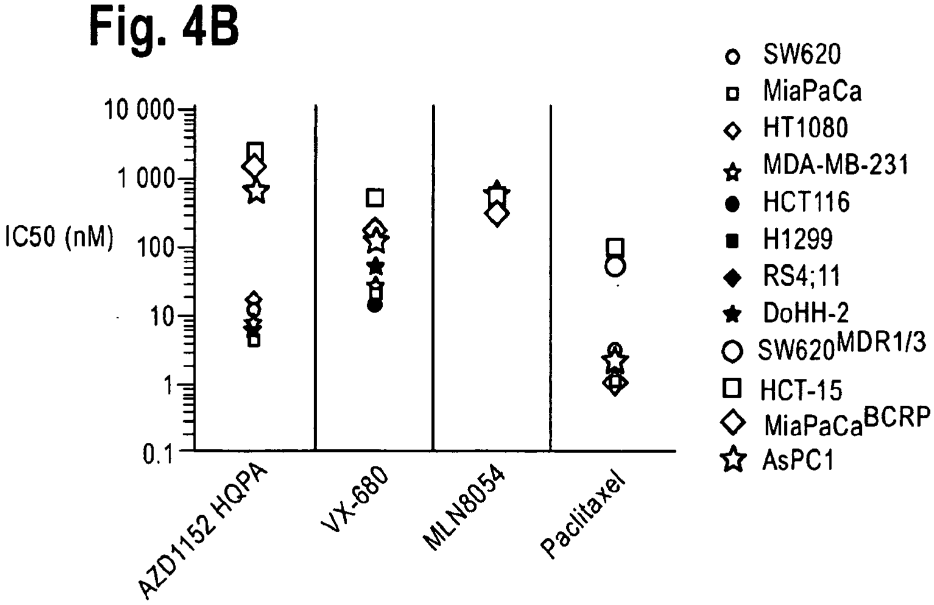


Fig. 4C

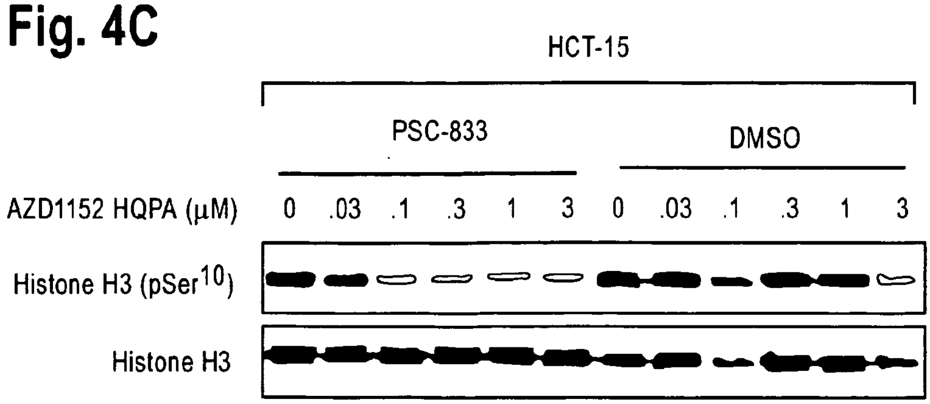

Fig. 4D

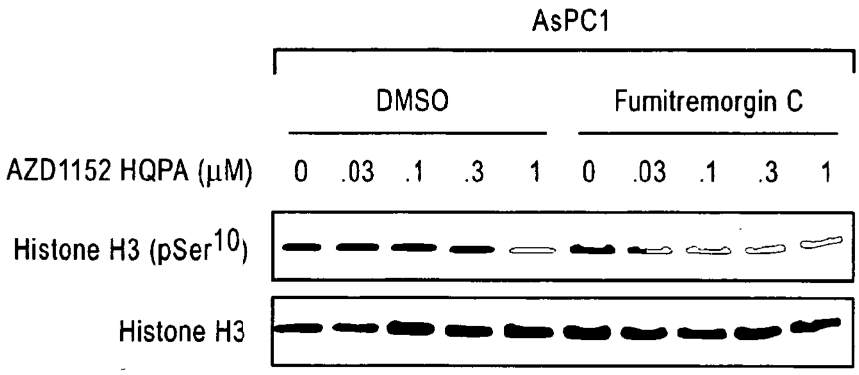
AsPC1
DMSO
Fumitremorgin C
AZD1152 HQPA (μM)
0 .03 .1 .3 1 0 .03 .1 .3 1
Histone H3 (pSer10)
Histone H3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010051320 A
- US 5231020 A
- EP 50424 A
- EP 84796 A
- EP 258017 A
- EP 237362 A
- EP 201184 A
- US 4683202 A
- US 4582788 A
- US 4683194 A
- US 4948882 A
- US 5464746 A
- US 5424414 A
- US 5447841 A
- US 5491224 A
- US 5756696 A
- US 5776688 A
- US 20030170881 A
- US 20040018577 A
- US 20050054078 A
- US 20060160164 A
- US 5063081 A
- US 5089424 A
- US 5006309 A
- US 5294404 A


### Non-patent literature cited in the description


- **CARMENA M.E.W.** *Nat. Rev. Mol. Cell Biol.,* 2003, vol. 4, 842-854
- **DUCAT, D.Z.Y.** *Exp. Cell Res.,* 2004, vol. 301, 60-67
- **MARUMOTO, T.Z.D. et al.** *Nat. Rev. Cancer,* 2005, vol. 5, 42-50
- **ANDREWS, P.D. et al.** *Curr. Opin. Cell Biol.,* 2003, vol. 15, 672-683
- **DITCHFIELD, C, J.V. et al.** *J. Cell Biol.,* 2003, vol. 161, 267-280
- **HARRINGTON, E.A. et al.** *Nat. Med.,* 2004, vol. 10, 262-267
- **HAUF, S. et al.** *J. Cell Biol.,* 2003, vol. 161, 281-294
- **MANFREDI, M.G. et al.** *Proc. Natl. Acad. Sci., USA,* 2007, vol. 104, 4106-4111
- **MORTLOCK, A.A. et al.** *J. Med. Chem.,* 2007, vol. 50, 2213-2224
- **WILKINSON, R.W. et al.** *Clin. Cancer Res.,* 2007, vol. 13, 3683-3688
- **PALLIS, M.R.N.** *Leukemia,* 2004, vol. 18, 1927-1930
- **VAN DER HOLT, B.L.B. et al.** *Blood,* 2005, vol. 106, 2646-2654
- **TROCK, B.J. et al.** *J. Natl. Cancer Inst.,* 1997, vol. 89, 917-931 1
- **BATES, S.F. et al.** *Novartis Found Symp.,* 2002, 83-96
- **GIRDLER F. et al.** *Chemistry & Biology,* June 2008, vol. 15, 552-562
- **MORTLOCK, AA et al.** *J. Med. Chem.,* 2007, vol. 50, 2213-24
- **DITCHFIELD, C. et al.** *J. Cell Bio.,* 2003, vol. 161 (2), 267-280
- **MONTEMBAULT, E. et al.** *Drugs of the Future,* 2005, vol. 30 (1), 1-9
- **HAUF, S. et al.** *J. Cell Bio.,* 2003, vol. 161 (2), 281-294
- **MULLIS, K. et al.** *Cold Spring Harb Symp Quant Biol.,* 1986, vol. 51, 263-73
- **BUCHARDT, O. ; P. NIELSEN ; R. BERG.** *Peptide Nucleic Acids,* 1992
- **AUSUBEL, F.M. ; R. BRENT ; R.E. KINGSTON et al.** Current Protocols in Molecular Biology. John Wiley & Sons, 1987
- **VAN DER KROL et al.** *Biotechniques,* 1988, vol. 6, 958-76
- **ZON, G.** *Pharm Res.,* 1988, vol. 5, 539-49
- **FAN, Y.-S.** Molecular cytogenetics: protocols and applications. Humana Press, 2002, 411
- **MATSUZAKI, H. et al.** Genotyping over 100,000 SNPs on a pair of oligonucleotide arrays. *Nat Methods,* 2004, vol. 1, 109-11
- **MANFRIEDI, MG et al.** *Proc. Natl. Acad. Sci. USA,* 2007, vol. 104, 4106-4111
- **OLEJNICZAK et al.** *Mol. Can. Res.,* 2007, vol. 5 (4), 331-339
- **GIRDLER, F. et al.** *Chem. Biol.,* 2008, vol. 15, 552-562
- **TWENTYMAN PR.** *Eur. J. Cance,* 1991, vol. 27, 1639-1642
- **BOESCH, D.** *Cancer Res.,* 1991, vol. 51, 4226-4233

- **SMITH AJ.** *J. Biol. Chem.,* 2000, vol. 275, 23530-23539